# EUROPEAN PATENT SPECIFICATION

(11) **EP 3 810 091 B1**
(45) Date of publication and mention of the grant of the patent: **20.05.2026**
(21) Application number: 19807397.5
(22) Date of filing: 21.05.2019
(51) Int. Cl.: A61K 9/14, A61K 9/51, A61K 47/42, A61K 31/436, A61P 9/12

(54) **COMPOSITIONS FOR TREATING PULMONARY HYPERTENSION**
ZUSAMMENSETZUNGEN ZUR BEHANDLUNG PULMONALER HYPERTONIE
COMPOSITIONS POUR LE TRAITEMENT DE L'HYPERTENSION PULMONAIRE

(30) Priority: 22.05.2018 US 201862675110 P; 25.02.2019 US 201962810290 P; 19.03.2019 US 201962820838 P; 19.03.2019 US 201962820842 P
(43) Date of publication of application: 28.04.2021
(73) Proprietor: Abraxis BioScience, LLC, Summit, New Jersey 07901 (US)
(72) Inventor: DESAI, Neil P., Pacific Palisades, California 90272 (US); HOU, Shihe, Millington, New Jersey 07946 (US)
(74) Representative: Jones Day
(86) International application number: PCT/US2019/033372
(87) International publication number: WO 2019/226685

(56) References cited:
- WO-A1-2016/057712
- WO-A1-2017/004249
- WO-A1-2017/004264
- WO-A1-2017/004266
- WO-A1-2017/004267
- WO-A1-2018/064405
- US-A1- 2018 133 157
- XIE, J. ET AL.: "mTOR inhibitors in cancer therapy", F1000RESEARCH, vol. 5, 2016, pages 1 - 11, XP055657735

## Description

### TECHNICAL FIELD

The present disclosure pertains to methods and compositions for treating, stabilizing, preventing, and/or delaying pulmonary hypertension using nanoparticles that comprise mTOR inhibitor (e.g., rapamycin or a derivative thereof) and a carrier protein.

### BACKGROUND OF THE INVENTION

Pulmonary hypertension (PH) is a syndrome characterized by increased pulmonary artery pressure. PH is defined hemodynamically as a systolic pulmonary artery pressure greater than 30 mm Hg or evaluation of mean pulmonary artery pressure greater than 25 mm Hg. *See* Zaiman et al., Am. J. Respir. Cell Mol. Biol. 33:425-31 (2005). Further, PH, as a result of the increased pressure, damages both the large and small pulmonary arteries. The walls of the smallest blood vessels thicken and are no longer able to transfer oxygen and carbon dioxide normally between the blood and the lungs. In time, pulmonary hypertension leads to thickening of the pulmonary arteries and narrowing of the passageways through which blood flows. Once pulmonary hypertension develops, the right side of the heart works harder to compensate; however, the increased effort causes it to become enlarged and thickened. Proliferation of smooth muscle and endothelial cells which normally exist in a quiescent state leads to remodeling of the vessels with obliteration of the lumen of the pulmonary vasculature. This causes a progressive rise in pulmonary pressures as blood is pumped through decreased lumen area. The enlarged right ventricle places a person at risk for pulmonary embolism because blood tends to pool in the ventricle and in the legs. If clots form in the pooled blood, they may eventually travel and lodge in the lungs with disastrous consequences. The progressive rise in pressure also places an additional workload on the right ventricle which eventually fails and leads to premature death in these patients.

Various pathologic changes occur in pulmonary arteries as a result of PH. Persistent vasoconstriction and structural remodeling of the pulmonary vessels are cardinal features of PH. Pulmonary vascular smooth muscle cells undergo a phenotypic switch from contractile normal phenotype to a synthetic phenotype leading to cell growth and matrix deposition. Histological examination of tissue samples from patients with pulmonary hypertension shows intimal thickening, as well as smooth muscle cell hypertrophy, especially for those vessels <100 µm diameter. Further, abnormal smooth muscle cells often overexpress endothelin and serotonin transporters, which likely play a role in the development of PH.

The most common symptom of pulmonary hypertension initially is shortness of breath upon exertion. Some people feel light-headed or fatigued upon exertion, and an angina-like chest pain is common. Because body tissues are not receiving enough oxygen, general weakness is another problem. Other symptoms, such as coughing and wheezing, may be caused by an underlying lung disease. Edema, particularly of the legs, may occur because fluid may leak out of the veins and into the tissues, signaling that cor pulmonale has developed. Some people with pulmonary hypertension have connective tissue disorders, especially scleroderma. When people have both conditions, pulmonary hypertension and connective tissue disorders, Raynaud's phenomenon often develops before symptoms of pulmonary hypertension appear, sometimes as long as years earlier.

Treatment of some types of pulmonary hypertension is often directed at the underlying lung disease. Currently, the treatment options available for those suffering from PH target cellular dysfunction that leads to constriction of the vasculature. Therapies such as prostanoids, phosphodiesterase-5 inhibitors and endothelin receptor antagonists primarily work by causing dilation of the pulmonary vessels. Vasodilators, such as calcium channel blockers, nitric oxide, and prostacyclin, are often helpful for pulmonary hypertension associated with scleroderma, chronic liver disease, and HIV infection. In contrast, these drugs have not been proven effective for people with pulmonary hypertension due to an underlying lung disease. For most people with pulmonary hypertension due to an unknown cause, vasodilators, such as prostacyclin, drastically reduce blood pressure in the pulmonary arteries. Prostacyclin given intravenously through a catheter surgically implanted in the skin improves the quality of life, increases survival, and reduces the urgency of lung transplantation. Unfortunately, many patients respond poorly to these therapies or stop responding to them over time. The only remaining option at that point in time is a single or double lung transplantation to treat PH. Although there is some evidence that available therapies have secondary effects on vascular remodeling, there are currently no therapies that target abnormal cell proliferation in PAH.

Many anti-proliferative agents are dissolved in a solvent/surfactant which produces hypersensitivity reactions. Great efforts have been invested on the development of water soluble prodrugs and derivatives of anti-proliferative agents with higher hydrophilic groups to enhance water solubility and thus obviate the need for potentially toxic solvents/surfactants. Another approach to address the problem associated with the poor water solubility of anti-proliferative agents is the development of various formulations such as nanoparticles, oil-in-water emulsions, and liposomes. Nanoparticle compositions of substantially poorly water soluble drugs and uses thereof have been disclosed, for example, in PCT Application Pub. No. WO07/027941 and WO 2008/109163. US 2018/133157 A1 relates to nanoparticles comprising a taxane and/or nanoparticles comprising a rapamycin for treating pulmonary hypertension.

### BRIEF SUMMARY OF THE INVENTION

Disclosed herein are methods of treating pulmonary hypertension in an individual, comprising administering to the individual a composition comprising nanoparticles comprising an mTOR inhibitor and a carrier protein, wherein the dose of the mTOR inhibitor in the composition is no more than about 10 mg/m². In some examples, the dose of the mTOR inhibitor in the composition is no less than about 0.1 mg/m². In some examples, the dose of the mTOR inhibitor in the composition is no less than about 5 mg/m².

In some examples according to any one of the methods described herein, the dose of the mTOR inhibitor in the composition is no more than about 5 mg/m². In some examples, the dose of the mTOR inhibitor in the composition is about 5 mg/m².

In some examples according to any one of the methods described herein, the concentration of the mTOR inhibitor in the blood is at least about 2 ng/ml five days after administration of the nanoparticle composition.

In some examples according to any one of the methods described herein, the concentration of the mTOR inhibitor in the blood is no more than about 20 ng/ml seven days after administration of the nanoparticle composition.

In some examples according to any one of the methods described herein, the nanoparticle composition is administered at least once a week.

In some examples according to any one of the methods described herein, the nanoparticle composition is administered no more than once a week. In some examples, the nanoparticle composition is administered once a week. In some examples, the nanoparticle composition is administered once every two weeks, two out of three weeks, or three out of four weeks.

In some examples according to any one of the methods described herein, the nanoparticle composition is administered for at least about four weeks.

In some examples according to any one of the methods described herein, the pulmonary hypertension is pulmonary arterial hypertension.

In some examples according to any one of the methods described herein, the pulmonary hypertension is selected from the group consisting of idiopathic pulmonary arterial hypertension (IPAH), heritable pulmonary arterial hypertension (HPAH), drug and toxin induced PAH, PAH associated with connective tissue disease, and PAH associated with congenital heart defects.

In some examples according to any one of the methods described herein, the individual has a WHO functional class III or IV pulmonary arterial hypertension.

In some examples according to any one of the methods described herein, the mTOR inhibitor is the only pharmaceutically active agent useful for treating pulmonary hypertension that is administered to the individual.

In some examples according to any one of the methods described herein, the composition comprises more than about 50% of the mTOR inhibitor in nanoparticle form.

In some examples according to any one of the methods described herein, the nanoparticle composition is administered parenterally. In some examples, the nanoparticle composition is administered intravenously. In some examples, the nanoparticle composition is administered subcutaneously.

In some examples according to any one of the methods described herein, the mTOR inhibitor is rapamycin.

In some examples according to any one of the methods described herein, the individual has had at least one prior therapy for pulmonary hypertension. In some examples, the individual has had at least two prior therapies for pulmonary hypertension. In some examples, the prior therapy comprises administering an agent selected from the group consisting of a prostacyclin analogue, an endothelin-1 receptor antagonist, a phosphodiesterase 5 (PDE-5) inhibitor and a soluble guanylate cyclase (sGC) stimulator. In some examples, the individual has progressed on the prior therapy.

In some examples according to any one of the methods described herein, the carrier protein is albumin. In some examples, the albumin is human serum albumin.

In some examples according to any one of the methods described herein, the average diameter of the nanoparticles in the composition is no greater than about 200 nm.

In some examples according to any one of the methods described herein, the weight ratio of the carrier protein to the mTOR inhibitor in the nanoparticles is less than about 18:1.

In some examples according to any one of the methods described herein, the individual is human.

Further disclosed herein are unit dosage forms for treatment of pulmonary hypertension comprising (a) nanoparticles that comprise an mTOR inhibitor and a carrier protein, wherein the dose of the mTOR inhibitor in the composition is no more than about 10 mg/m², and (b) a pharmaceutical acceptable carrier.

Further disclosed herein are kits comprising (a) nanoparticles that comprise an mTOR inhibitor and a carrier protein, wherein the dose of the mTOR inhibitor in the kit is no more than about 10 mg/m2, and (b) instructions for using the kit in treating pulmonary hypertension.

The present invention provides a composition comprising nanoparticles comprising rapamycin and a carrier protein for use in a method of treating pulmonary hypertension in an individual, wherein the method comprises subcutaneously administering to the individual a dose of from about 1 mg/m² to about 10 mg/m² of rapamycin in the composition.

In some embodiments, the dose of rapamycin in the composition is from about 2.5 mg/m² to about 5 mg/m². In a preferred embodiment, the dose of rapamycin in the composition is about 5 mg/m².

In some embodiments, the concentration of rapamycin in the blood of the individual is at least about 2 ng/ml five days after administration of the nanoparticle composition. In some embodiments, the concentration of rapamycin in the blood of the individual is no more than about 20 ng/ml seven days after administration of the nanoparticle composition.

In some embodiments, the nanoparticle composition is administered at least once a week, no more than once a week, once a week, once every two weeks, two out of three weeks, or three out of four weeks. In some embodiments, the nanoparticle composition is administered for at least about four weeks.

In some embodiments, the pulmonary hypertension is pulmonary arterial hypertension (PAH). In some preferred embodiments, the PAH is selected from the group consisting of idiopathic pulmonary arterial hypertension (IPAH), heritable pulmonary arterial hypertension (HPAH), drug and toxin induced PAH, PAH associated with connective tissue disease, and PAH associated with congenital heart defects. In some preferred embodiments, the individual has a WHO functional class III or IV PAH.

In some embodiments, the composition comprises more than about 50% of rapamycin in nanoparticle form. In some embodiments, the carrier protein is albumin. In some preferred embodiments, the carrier protein is a human serum albumin.

In some embodiments, the average diameter of the nanoparticles in the composition is no greater than about 200 nm.

In some embodiments, the weight ratio of the carrier protein to rapamycin in the nanoparticles is less than about 18:1.

In some embodiments, the individual has had at least one prior therapy for pulmonary hypertension. In some preferred embodiments, the individual has had at least two prior therapies for pulmonary hypertension.

In some embodiments, the prior therapy comprises administering an agent selected from the group consisting of a prostacyclin analogue, an endothelin-1 receptor antagonist, a phosphodiesterase 5 (PDE-5) inhibitor and a soluble guanylate cyclase (sGC) stimulator.

In some embodiments, the individual has progressed on the prior therapy.

In some embodiments, the method further comprises administering to the individual a second therapy. In some preferred embodiments, the second therapy is selected from the group consisting of a vasodilator, a prostacyclin analogue, an endothelin-1 receptor antagonist, a PDE-5 inhibitor, an sGC stimulator, epoprostenol, iloprost, treprostiml, bosentan, macitentan, ambrisentan, sildenafil, tadalafil, riociguat and a tyrosine kinase inhibitor. In some preferred embodiments, the second therapy is imatinib. In some preferred embodiments, the composition is administered simultaneously, concurrently or sequentially with the second therapy.

In some embodiments, the individual is a human.

In some embodiments, the composition is in form of a unit dosage form, wherein the unit dosage form comprises: (a) said composition; and (b) a pharmaceutical acceptable carrier.

The invention further provides a kit for use in a method of treating pulmonary hypertension in an individual, wherein the kit comprises: (a) a composition comprising nanoparticles comprising rapamycin and a carrier protein; and (b) instructions for said use of said composition, wherein the method comprises subcutaneously administering to the individual a dose of from about 1 mg/m² to about 10 mg/m² of rapamycin in said composition.

The composition for use of the present invention comprises nanoparticles comprising rapamycin and a carrier protein. In the context of the present invention, the mTOR inhibitor is rapamycin; the route of administration is subcutaneous; the dose of rapamycin in the composition is from about 1 mg/m² to about 10 mg/m². Furthermore, reference to methods in the context of the present invention should be understood as reference to the composition of the invention for use in such methods. Methods of treatment of an individual by therapy or surgery is not part of the claimed subject matter.

### BRIEF DESCRIPTION OF FIGURES

FIGS. 1A and 1B provide trough concentrations of rapamycin (ng/ml) in the whole blood measured weekly during a 16-week period of ABI-009 administration (FIG. 1A) and dosages of rapamycin in ABI-009 administered at each week for each subject (FIG. 1B).
FIG. 2 provides the levels of pulmonary vascular resistance (PVR, dyn.sec/cm⁵), Cardiac Output (CO, L/min) and Cardiac Index (CI, L/min/m²) after a period of 16-week administration of ABI-009 as compared to those at the baseline.
FIG. 3 provides a summary of PAH patients' improvements in the functional and hemodynamic parameters after treatments with ABI-009.
FIG. 4 provides study scheme of a phase 1 clinical trial.
FIG. 5 provides results of efficacy parameters including 6-minute walking distance (6MWD), pulmonary vascular resistance (PVR), cardiac output, and NT proBNP post 16-week treatment. The whiskers represent min and max, the boxes span the interquartile range.
FIG. 6 provides rapamycin levels (ng/g) in blood, lung and liver of rats at 24 hours for *nab*-rapamycin (*nab*-R) and oral rapamycin (oral-R). Actual values were indicated (N=5 each group).
FIG. 7 shows rapamycin concentrations in whole blood samples taken from rats after subcutaneous (SC) or intravenous (IV) administration of a single dose of *nab*-rapamycin (ABI-009) between 0 and 24 hours after administration.
FIG. 8 shows rapamycin concentrations in whole blood samples taken from rats after subcutaneous (SC) or intravenous (IV) administration of a single dose of *nab*-rapamycin (ABI-009) between 0 and 168 hours after administration.
FIG. 9 shows rapamycin concentrations in whole blood samples taken from rats after subcutaneous (SC) or intravenous (IV) administration of a single dose of *nab*-rapamycin (ABI-009) between 0 and 24 hours after administration.
FIG. 10 shows the bioavailability of *nab*-rapamycin (ABI-009) after subcutaneous (subQ) or intravenous (IV) administration of a single dose in rats as indicated by the calculated area under the curve (AUC).
FIG. 11 shows the concentration of rapamycin in rat bone marrow (top) or brain (bottom) 24 or 168 hours after subcutaneous (subQ) or intravenous (IV) administration of a single dose of *nab*-rapamycin (ABI-009).
FIG. 12 shows the concentration of rapamycin in rat heart (top) or liver (bottom) 24 or 168 hours after subcutaneous (subQ) or intravenous (IV) administration of a single dose of *nab-*rapamycin (ABI-009).
FIG. 13 shows the concentration of rapamycin in rat lung (top) or pancreas (bottom) 24 or 168 hours after subcutaneous (subQ) or intravenous (IV) administration of a single dose of *nab*-rapamycin (ABI-009).
FIG. 14 shows a comparison of histopathology scores assessed on skins from rats among different treatment groups.
FIG. 15 is a representative histogram image of skin from rat in Group 1 (0.9% saline). Histologic lesions are limited to an aggregate of mixed inflammatory cells (black arrow) within the subcutaneous tissues (SC). The dermis (D) and epidermis (E) are indicated.
FIG. 16 is a representative histogram image of skin from rat in Group 2 (HSA in 0.9% saline). Multifocal mixed inflammatory cell aggregates (black arrows) are visible within the subcutis (SC). The epidermis (E) and dermis (D) are unremarkable.
FIG. 17 is a representative histogram image of skin from rat in Group 3 (ABI-009, 1.7 mg/kg). Minimal mixed inflammatory cell infiltration (black arrow) is visible in the subcutaneous tissues (SC). The epidermis (E) and dermis (D) are indicated.
FIG. 18 is a representative histogram image of skin from rat in Group 4 (ABI-009, 5 mg/kg). Scattered mixed inflammatory cell infiltration (black arrow) and a site of minimal necrosis (blue arrow) are present in the subcutis (SC). The epidermis (E) and dermis (D) are unremarkable.
FIG. 19 is a representative histogram image of skin from rat in Group 4 (ABI-009, 10 mg/kg). Subcutaneous (SC) mixed inflammatory cell infiltration (black arrow) and a region of necrosis (blue arrow) are captured. The epidermis (E) and dermis (D) are unremarkable.
FIG. 20 shows the mean trough sirolimus blood levels in rats administered with ABI-009 at 1.7 mg/kg, 5 mg/kg or 10 mg/kg.
FIG. 21 shows the concentration of rapamycin in rat brain (A), heart (B), liver (C), lung (D), pancreas (E) and blood (F) 2, 8, 24, 72, or 120 hours after intravenous (IV) administration of a single dose of *nab*-rapamycin (ABI-009).

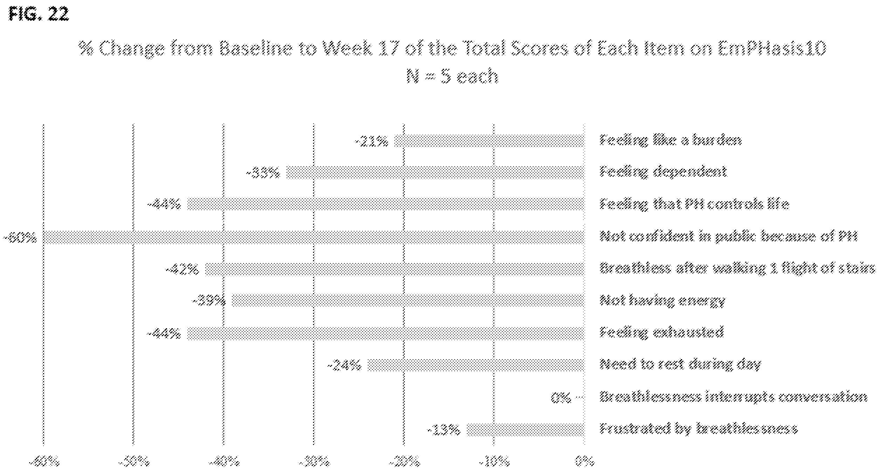

FIG. 22 shows the changes (%) of the total score of each item on EmPHasis 10 (patient number n=5 for each item) from baseline to week 17.

### DETAILED DESCRIPTION OF THE INVENTION

Disclosed herein are methods of treating pulmonary hypertension (*e.g*., a severe form of pulmonary arterial hypertension, *e.g*., WHO Function Class III or IV pulmonary arterial hypertension) in an individual, comprising administering to the individual an effective amount of a composition comprising an mTOR inhibitor (*e.g.,* a limus drug, *e.g.,* rapamycin or a derivative thereof) and a carrier protein (*e.g.,* an albumin). The current approved pulmonary arterial hypertension (PAH) therapeutics mainly function as vasodilators and do not address the endothelial and smooth muscle cell hyperproliferation aspect of the disease. Imatinib, a tyrosine kinase inhibitor is the only antiproliferative drug that has been tested in treating PAH in latestage clinical trials but caused significant safety issues. This invention is based in part upon applicants' surprising finding that administering a composition comprising an mTOR inhibitor (e*.g.,* a nanoparticle composition comprising rapamycin and albumin) into an individual having a severe form of pulmonary hypertension (*e.g*., WHO Function Class III PAH) not only reduces pulmonary vascular resistance (PVR), but also remarkably ameliorates circulatory inadequacy, for example, remarkably improving cardiac output, and/or improves six-minute walking distance performance. Such advantageous effect was achieved with a dose of no more than one tenth or one twentieth of the maximum tolerated dose (MTD). For example, a nanoparticle composition comprising rapamycin and albumin produced such effect at a dose of no more than about 1, 5 or 10mg/m² (*e.g.,* a weekly dose of 1-10 mg/m²) while the MTD of the nanoparticle composition is about 100 mg/m². Such doses of rapamycin composition also achieve a favorable safety profile.

Accordingly, in some aspects, the present invention provides a composition for use in methods of treating pulmonary hypertension comprising subcutaneously administering to the individual an effective amount of a composition comprising rapamycin and a carrier protein *(e.g.,* an albumin), wherein the dose of rapamycin is such that it strikes a balance of producing a favorable safe profile while providing advantageous effect of treating pulmonary hypertension, i.e., from about 1 mg/m² to about 10 mg/m² of rapamycin in the composition. In some aspects, the methods of the invention ameliorate circulatory inadequacy (*e.g.*, cardiac output) in an individual having pulmonary hypertension. In some aspects, the methods of the invention reduce pulmonary vascular resistance (PVR) in an individual having pulmonary hypertension. In some aspects, the methods of the invention improve six-minute walking distance (6MWD) performance in an individual having pulmonary hypertension. According to the invention, the dose of rapamycin in the composition is from about 1 mg/m² to about 10 mg/m². In some embodiments, the nanoparticle composition is administered for at least about four weeks (e.g., at least about eight, twelve, sixteen, twenty-four, thirty-two, forty, or forty-eight weeks).

### Definitions

Unless specifically indicated otherwise, all technical and scientific terms used herein have the same meaning as commonly understood by those of ordinary skill in the art to which this invention belongs. In addition, any method or material similar or equivalent to a method or material described herein can be used in the practice of the present invention. For purposes of the present invention, the following terms are defined.

It is understood that embodiments provided herein include "consisting" and/or "consisting essentially of" embodiments.

As used herein, "the composition" or "compositions" includes and is applicable to compositions of the invention. The patent also provides pharmaceutical compositions of the composition for use of the invention.

Reference to "rapamycin" herein applies to rapamycin or its derivatives. In this patent, "rapamycin" and "sirolimus" are used interchangeably. Rapamycin is sometimes referred to elsewhere as rapamycin, rapammune, or rapamune. Reference to "rapamycin" is to simplify the description and is exemplary. Derivatives of rapamycin include, but are not limited to, compounds that are structurally similar to rapamycin, or are in the same general chemical class as rapamycin, analogs of rapamycin, or pharmaceutically acceptable salts of rapamycin or its derivatives or analogs. In some embodiments, rapamycin increases basal AKT activity, increases AKT phosphorylation, increases PI3-kinase activity, increases the length of activation of AKT (e.g., activation induced by exogenous IGF-1), inhibits serine phosphorylation of IRS-1, inhibits IRS-1 degradation, inhibits or alters CXCR4 subcellular localization, inhibits VEGF secretion, decreases expression of cyclin D2, decreases expression of survivin, inhibits IL-6-induced multiple myeloma cell growth, inhibits pulmonary hypertension cell proliferation, increases apoptosis, increases cell cycle arrest, increases cleavage of poly(ADPribose) polymerase, increases cleavage of caspase-8/caspase-9, alters or inhibits signaling in the phosphatidylinositol 3-kinase/AKT/mTOR and/or cyclin D1/retinoblastoma pathways, inhibits angiogenesis, and/or inhibits osteoclast formation. As disclosed, the derivative of rapamycin retains one or more similar biological, pharmacological, chemical and/or physical properties (including, for example, functionality) as rapamycin. An exemplary rapamycin derivative includes benzoyl rapamycin, such as that disclosed in paragraph [0022] of WO 2006/089207. Other exemplary rapamycin derivatives include WY-090217, AY-22989, NSC-226080, SiiA-9268A, oxaazacyclohentriacontine, temrapamycin (CCI 779 (Wyeth)), everolimus (RAD 001 (Novartis)), pimecrolimus (ASM981), SDZ-RAD, SAR943, ABT-578, AP23573, and Biolimus A9.

Unless clearly indicated otherwise, "an individual" as used herein intends a mammal, including but not limited to a primate, human, bovine, horse, feline, canine, or rodent.

As used herein, "treatment" or "treating" is an approach for obtaining beneficial or desired results including clinical results. For purposes of this invention, beneficial or desired clinical results include, but are not limited to, one or more of the following: decreasing one more symptoms resulting from the disease, diminishing the extent of the disease, stabilizing the disease (*e.g.*, preventing or delaying the worsening of the disease), preventing or delaying the occurrence of the disease, delay or slowing the progression of the disease, ameliorating the disease state, decreasing the dose of one or more other medications required to treat the disease, increasing the quality of life, and/or prolonging survival. In some embodiments, the composition reduces the severity of one or more symptoms associated with pulmonary hypertension by at least about any of 10%, 20%, 30%, 40%, 50%, 60%, 70%, 80%, 90%, 95%, or 100% compared to the corresponding symptom in the same subject prior to treatment or compared to the corresponding symptom in other subjects not receiving the composition. Also encompassed by "treatment" is a reduction of pathological consequence of pulmonary hypertension. The methods herein contemplate any one or more of these aspects of treatment.

As used herein, "delaying" the development of pulmonary hypertension means to defer, hinder, slow, retard, stabilize, and/or postpone development of the disease. This delay can be of varying lengths of time, depending on the history of the disease and/or individual being treated. As is evident to one skilled in the art, a sufficient or significant delay can, in effect, encompass prevention, in that the individual does not develop the disease. A method that "delays" development of pulmonary hypertension is a method that reduces probability of disease development in a given time frame and/or reduces the extent of the disease in a given time frame, when compared to not using the method. Such comparisons are typically based on clinical studies, using a statistically significant number of subjects. Pulmonary hypertension development can be detectable using standard methods, such as routine physical exams, x-ray, electrocardiogram, and echocardiogram. Development may also refer to disease progression that may be initially undetectable and includes occurrence and onset.

As used herein, an "at risk" individual is an individual who is at risk of developing pulmonary hypertension. An individual "at risk" may or may not have detectable disease, and may or may not have displayed detectable disease prior to the treatment methods described herein. "At risk" denotes that an individual has one or more so-called risk factors, which are measurable parameters that correlate with development of pulmonary hypertension, which are described herein. An individual having one or more of these risk factors has a higher probability of developing pulmonary hypertension than an individual without these risk factor(s).

As used herein, by "pharmaceutically active compound" is meant a chemical compound that induces a desired effect, *e.g*., treating, stabilizing, preventing, and/or delaying pulmonary hypertension.

As used herein, by "combination therapy" is meant a first therapy that includes nanoparticles comprising an mTOR inhibitor (*e.g.,* rapamycin or a derivative thereof, *e.g.,* rapamycin) and a carrier protein in conjunction with a second therapy (*e.g*., surgery or a therapeutic agent) useful for treating, stabilizing, preventing, and/or delaying pulmonary hypertension. Administration in "conjunction with" another compound includes administration in the same or different composition(s), either sequentially, simultaneously, or continuously. In some embodiments, the combination therapy optionally includes one or more pharmaceutically acceptable carriers or excipients, non-pharmaceutically active compounds, and/or inert substances.

As is understood in the art, an "effective amount" may be in one or more doses, *i.e.,* a single dose or multiple doses may be required to achieve the desired treatment endpoint. An effective amount may be considered in the context of administering one or more therapeutic agents, and a nanoparticle composition (*e.g.*, a composition including rapamycin and a carrier protein) may be considered to be given in an effective amount if, in conjunction with one or more other agents, a desirable or beneficial result may be or is achieved. The components (*e.g.,* the first and second therapies) in a combination therapy of the invention may be administered sequentially, simultaneously, or continuously using the same or different routes of administration for each component. Thus, an effective amount of a combination therapy includes an amount of the first therapy and an amount of the second therapy that when administered sequentially, simultaneously, or continuously produces a desired outcome.

A "therapeutically effective amount" refers to an amount of a composition (*e.g.*, nanoparticles that comprise an mTOR inhibitor (*e.g.,* rapamycin or a derivative thereof, *e.g.,* rapamycin) and a carrier protein), a therapy, or a combination therapy sufficient to produce a desired therapeutic outcome (*e.g.*, reducing the severity or duration of, stabilizing the severity of, or eliminating one or more symptoms of pulmonary hypertension). For therapeutic use, beneficial or desired results include, *e.g*., decreasing one or more symptoms resulting from the disease (biochemical, histologic and/or behavioral), including its complications and intermediate pathological phenotypes presenting during development of the disease, increasing the quality of life of those suffering from the disease, decreasing the dose of other medications required to treat the disease, enhancing effect of another medication, delaying the progression of the disease, and/or prolonging survival of patients.

A "prophylactically effective amount" refers to an amount of a composition (*e.g*., nanoparticles that comprise an mTOR inhibitor *(e.g.,* rapamycin or a derivative thereof, *e.g.,* rapamycin) and a carrier protein, a therapy, or a combination therapy sufficient to prevent or reduce the severity of one or more future symptoms of pulmonary hypertension when administered to an individual who is susceptible and/or who may develop pulmonary hypertension. For prophylactic use, beneficial or desired results include, *e.g*., results such as eliminating or reducing the risk, lessening the severity of future disease, or delaying the onset of the disease (e.g., delaying biochemical, histologic and/or behavioral symptoms of the disease, its complications, and intermediate pathological phenotypes presenting during future development of the disease).

As used herein, by "pharmaceutically acceptable" or "pharmacologically compatible" is meant a material that is not biologically or otherwise undesirable, *e.g.*, the material may be incorporated into a pharmaceutical composition administered to a patient without causing any significant undesirable biological effects or interacting in a deleterious manner with any of the other components of the composition in which it is contained. Pharmaceutically acceptable carriers or excipients have preferably met the required standards of toxicological and manufacturing testing and/or are included on the Inactive Ingredient Guide prepared by the U.S. Food and Drug administration.

Reference to "about" a value or parameter herein includes (and describes) embodiments that are directed to that value or parameter per se. For example, description referring to "about X" includes description of "X".

The term "about X-Y" used herein has the same meaning as "about X to about Y." The expression "about X, Y or Z" used herein has the same meaning as "about X, about Y, or about Z."

As used herein, reference to "not" a value or parameter generally means and describes "other than" a value or parameter. For example, the method is not used to treat cancer of type X means the method is used to treat cancer of types other than X.

The terms "a," "an," or "the" as used herein not only include aspects with one member, but also include aspects with more than one member. For instance, the singular forms "a," "an," and "the" include plural referents unless the context clearly dictates otherwise. Thus, for example, reference to "a cell" includes a plurality of such cells and reference to "the agent" includes reference to one or more agents known to those skilled in the art, and so forth.

### Methods of Treating Pulmonary Hypertension

Disclosed herein is a variety of methods of using nanoparticle compositions with an mTOR inhibitor *(e.g.,* rapamycin or a derivative thereof, *e.g.,* rapamycin) and a carrier protein (*e.g.,* albumin, *e.g.,* human albumin, *e.g.,* human serum albumin) to treat pulmonary hypertension (e.g., severe pulmonary arterial hypertension). In some examples, the dose of the mTOR inhibitor (*e.g.,* rapamycin) is no more than about 10 mg/m². In some examples, the dose of the mTOR inhibitor (*e.g.,* rapamycin) is no less than about 0.1 mg/m². In some examples, the nanoparticle composition is administered at least once a week. In some examples, the nanoparticle composition is administered no more than once a week. In some examples, the nanoparticle composition is administered for at least about four weeks. In some examples, the nanoparticle composition is administered parenterally (*e.g.,* intravenously or subcutaneously).

In some examples, a method is disclosed for delivering an effective amount of an mTOR inhibitor (such as sirolimus) to the lung of an individual, the method comprising subcutaneously administering a composition, such as a pharmaceutical composition, comprising nanoparticles comprising rapamycin and an albumin, wherein the dose of rapamycin in the nanoparticles to deliver an effective amount of rapamycin to the lung is any of about 0.1 mg/m² to about 10 mg/m² (such as about 0.1 mg/m² to about 5 mg/m² or about 5 mg/m² to about 10 mg/m²), and values and ranges therein. In some examples, the individual has pulmonary hypertension (*e.g.,* severe pulmonary arterial hypertension).

The present invention provides a composition comprising nanoparticles comprising rapamycin and a carrier protein for use in an individual, where the method comprises subcutaneously administering to the individual a dose of rapamycin in the composition of from about 1 mg/m² to about 10 mg/m² (such as about 1-2, 2-3, 3-4, 4-5, 5-6, 6-7, 7-8, 8-9, 9-10 mg/m²), for example, about 2.5 mg/m² to about 10 mg/m², or about 5 mg/m² to about 10 mg/m². In some embodiments, the dose of rapamycin in the composition is no more than about 10%, 9%, 8%, 7%, 6%, 5%, 4%, 3%, 2%, or 1% of the MTD of the mTOR inhibitor in the composition. In some embodiments, the dose of rapamycin in the composition is about 1 mg/m² to about 5 mg/m², or about 2.5 mg/m² to about 5 mg/m². In some embodiments, the dose of rapamycin in the composition is about any of 1, 2, 3, 4, 5, 6, 7, 8, 9, or 10 mg/m². . In some embodiments, the nanoparticle composition is administered about once a week. In some embodiments, the nanoparticle composition is administered for at least about four weeks (e.g., at least about eight, twelve, sixteen, twenty-four, thirty-two, forty, or forty-eight weeks). In some embodiments, the concentration of rapamycin in the blood is at least about 2 ng/ml five days after administration of the nanoparticle composition. In some embodiments, the concentration of rapamycin in the blood is no more than about 20 ng/ml seven days after administration of the nanoparticle composition. In some embodiments, the pulmonary hypertension is World Health Organization [WHO] Function Class II, III or IV pulmonary arterial hypertension. In some embodiments, the composition comprises more than about 50% of rapamycin in nanoparticle form. In some embodiments, the carrier protein is human serum albumin. In some embodiments, the average diameter of the nanoparticles in the composition is no greater than about 200 nm. In some embodiments, the weight ratio of the carrier protein to rapamycin in the nanoparticles is less than about 18:1. In some embodiments, the individual is human. In some embodiments, the individual has a high level of fibrosis in the lung. In some embodiments, the individual has a high level of angiogenesis in the lung. In some embodiments, the individual has increased fibrosis in the lung. In some embodiments, the individual has increased angiogenesis in the lung.

In some embodiments, the pulmonary hypertension is World Health Organization [WHO] Function Class III or IV pulmonary arterial hypertension. In some embodiments, the dose of rapamycin in the composition is about 1-2, 2-3, 3-4, 4-5, 5-6, 6-7, 7-8, 8-9, 9-10 mg/m², about 2.5 mg/m² to about 10mg/m², or about 5 mg/m² to about 10mg/m². In some embodiments, the dose of rapamycin in the composition is no more than about 10%, 9%, 8%, 7%, 6%, 5%, 4%, 3%, 2%, or 1% of the MTD of rapamycin in the composition. In some embodiments, the dose of rapamycin in the composition is about 1 mg/m² to about 5 mg/m², or about 2.5 mg/m² to about 5 mg/m². In some embodiments, the dose of rapamycin in the composition is about any of 1, 2, 3, 4, 5, 6, 7, 8, 9, or 10 mg/m². In some embodiments, the pulmonary hypertension is WHO Function Class III pulmonary arterial hypertension. In some embodiments, the nanoparticle composition is administered about once a week. In some embodiments, the nanoparticle composition is administered for at least about four weeks (e.g., at least about eight, twelve, sixteen, twenty-four, thirty-two, forty, or forty-eight weeks). In some embodiments, the concentration of rapamycin in the blood is at least about 2 ng/ml on the 5th day after administration of the nanoparticle composition. In some embodiments, the concentration of rapamycin in the blood is no more than about 20 ng/ml within 7 days or on the 7th day after administration of the nanoparticle composition. In some embodiments, the composition comprises more than about 50% of rapamycin in nanoparticle form. In some embodiments, rapamycin is the only pharmaceutically active agent useful for treating pulmonary hypertension that is administered to the individual. In some embodiments, the carrier protein is human serum albumin. In some embodiments, the average diameter of the nanoparticles in the composition is no greater than about 200 nm. In some embodiments, the weight ratio of the carrier protein to rapamycin in the nanoparticles is less than about 18:1. In some embodiments, the individual is human. In some embodiments, the individual has a high level of fibrosis in the lung. In some embodiments, the individual has a high level of angiogenesis in the lung. In some embodiments, the individual has increased fibrosis in the lung. In some embodiments, the individual has increased angiogenesis in the lung.

In some embodiments, the dose of rapamycin in the composition is about 1-2, 2-3, 3-4, 4-5, 5-6, 6-7, 7-8, 8-9, 9-10 mg/m², such as about 1 mg/m², about 2.5 mg/m², about 5 mg/m², or about 10 mg/m², and the pulmonary hypertension is World Health Organization [WHO] Function Class III or IV pulmonary arterial hypertension. In some embodiments, the dose of rapamycin in the composition is no more than about 10%, 9%, 8%, 7%, 6%, 5%, 4%, 3%, 2%, or 1% of the MTD of rapamycin in the composition. In some embodiments, the nanoparticle composition is administered about once a week. In some embodiments, the nanoparticle composition is administered for at least about four weeks (e.g., at least about eight, twelve, sixteen, twenty-four, thirty-two, forty, or forty-eight weeks). In some embodiments, the concentration of rapamycin in the blood is at least about 2 ng/ml on the 5th day after administration of the nanoparticle composition. In some embodiments, the concentration of rapamycin in the blood is no more than about 20 ng/ml within 7 days or on the 7th day after administration of the nanoparticle composition. In some embodiments, the composition comprises more than about 50% of rapamycin in nanoparticle form. In some embodiments, rapamycin is the only pharmaceutically active agent useful for treating pulmonary hypertension that is administered to the individual. In some embodiments, the carrier protein is human serum albumin. In some embodiments, the average diameter of the nanoparticles in the composition is no greater than about 200 nm. In some embodiments, the weight ratio of the carrier protein to rapamycin in the nanoparticles is less than about 18:1. In some embodiments, the individual is human. In some embodiments, the individual has a high level of fibrosis in the lung. In some embodiments, the individual has a high level of angiogenesis in the lung. In some embodiments, the individual has increased fibrosis in the lung. In some embodiments, the individual has increased angiogenesis in the lung.

In some embodiments, the individual has had at least one prior therapy for pulmonary hypertension. In some embodiments, the dose of rapamycin in the composition is about 1-2, 2-3, 3-4, 4-5, 5-6, 6-7, 7-8, 8-9, 9-10 mg/m², about 2.5 mg/m² to about 10 mg/m², or about 5 mg/m² to about 10 mg/m². In some embodiments, the dose of rapamycin in the composition is no more than about 10%, 9%, 8%, 7%, 6%, 5%, 4%, 3%, 2%, or 1% of the MTD of rapamycin in the composition. In some embodiments, the dose of rapamycin in the composition is about 1 mg/m² to about 5 mg/m², or about 2.5 mg/m² to about 5 mg/m². In some embodiments, the dose of rapamycin in the composition is about any of 1, 2, 3, 4, 5, 6, 7, 8, 9, or 10 mg/m². In some embodiments, the individual has at least two prior therapies for pulmonary hypertension. In some embodiments, the prior therapy comprises an agent selected from the group consisting of a prostacyclin analogue, an endothelin-1 receptor antagonist, a phosphodiesterase 5 (PDE-5) inhibitor and a soluble guanylate cyclase (sGC) stimulator. In some embodiments, the individual has progressed on the prior therapy. In some embodiments, the nanoparticle composition is administered about once a week. In some embodiments, the nanoparticle composition is administered for at least about four weeks (e.g., at least about eight, twelve, sixteen, twenty-four, thirty-two, forty, or forty-eight weeks). In some embodiments, the concentration of rapamycin in the blood is at least about 2 ng/ml on the 5th day after administration of the nanoparticle composition. In some embodiments, the concentration of rapamycin in the blood is no more than about 20 ng/ml within 7 days or on the 7th day after administration of the nanoparticle composition. In some embodiments, the composition comprises more than about 50% of rapamycin in nanoparticle form. In some embodiments, rapamycin is the only pharmaceutically active agent useful for treating pulmonary hypertension that is administered to the individual. In some embodiments, the carrier protein is human serum albumin. In some embodiments, the average diameter of the nanoparticles in the composition is no greater than about 200 nm. In some embodiments, the weight ratio of the carrier protein to rapamycin in the nanoparticles is less than about 18:1. In some embodiments, the individual is human. In some embodiments, the individual has a high level of fibrosis in the lung. In some embodiments, the individual has a high level of angiogenesis in the lung. In some embodiments, the individual has increased fibrosis in the lung. In some embodiments, the individual has increased angiogenesis in the lung.

In some embodiments, the individual has had at least one prior therapy for pulmonary hypertension, and wherein the pulmonary hypertension is World Health Organization [WHO] Function Class III or IV pulmonary arterial hypertension. In some embodiments, the dose of rapamycin in the composition is about 1-2, 2-3, 3-4, 4-5, 5-6, 6-7, 7-8, 8-9, 9-10 mg/m², about 2.5 mg/m² to about 10mg/m², or about 5 mg/m² to about 10 mg/m². In some embodiments, the dose of rapamycin in the composition is no more than about 10%, 9%, 8%, 7%, 6%, 5%, 4%, 3%, 2%, or 1% of the MTD of rapamycin in the composition. In some embodiments, the dose of rapamycin in the composition is about 1 mg/m² to about 5 mg/m², or about 2.5 mg/m² to about 5 mg/m². In some embodiments, the dose of rapamycin in the composition is about any of 1, 2, 3, 4, 5, 6, 7, 8, 9, or 10 mg/m². In some embodiments, the pulmonary hypertension is WHO Function Class III pulmonary arterial hypertension. In some embodiments, the individual has at least two prior therapies for pulmonary hypertension. In some embodiments, the prior therapy comprises an agent selected from the group consisting of a prostacyclin analogue, an endothelin-1 receptor antagonist, a phosphodiesterase 5 (PDE-5) inhibitor and a soluble guanylate cyclase (sGC) stimulator. In some embodiments, the individual has progressed on the prior therapy. In some embodiments, the nanoparticle composition is administered about once a week. In some embodiments, the nanoparticle composition is administered for at least about four weeks (e.g., at least about eight, twelve, sixteen, twenty-four, thirty-two, forty, or forty-eight weeks). In some embodiments, the concentration of rapamycin in the blood is at least about 2 ng/ml on the 5th day after administration of the nanoparticle composition. In some embodiments, the concentration of rapamycin in the blood is no more than about 20 ng/ml within 7 days or on the 7th day after administration of the nanoparticle composition. In some embodiments, the composition comprises more than about 50% of rapamycin in nanoparticle form. In some embodiments, rapamycin is the only pharmaceutically active agent useful for treating pulmonary hypertension that is administered to the individual. In some embodiments, the carrier protein is human serum albumin. In some embodiments, the average diameter of the nanoparticles in the composition is no greater than about 200 nm. In some embodiments, the weight ratio of the carrier protein to rapamycin in the nanoparticles is less than about 18:1. In some embodiments, the individual is human. In some embodiments, the individual has a high level of fibrosis in the lung. In some embodiments, the individual has a high level of angiogenesis in the lung. In some embodiments, the individual has increased fibrosis in the lung. In some embodiments, the individual has increased angiogenesis in the lung.

In some embodiments, the individual is resistant, refractory, or recurrent to at least one, two, or three prior therapies, and wherein the pulmonary hypertension is World Health Organization [WHO] Function Class III or IV pulmonary arterial hypertension. In some embodiments, the dose of rapamycin in the composition is about 1-2, 2-3, 3-4, 4-5, 5-6, 6-7, 7-8, 8-9, 9-10 mg/m², about 2.5 mg/m² to about 10mg/m², or about 5 mg/m² to about 10mg/m². In some embodiments, the dose of rapamycin in the composition is no more than about 10%, 9%, 8%, 7%, 6%, 5%, 4%, 3%, 2%, or 1% of the MTD of rapamycin in the composition. In some embodiments, the pulmonary hypertension is WHO Function Class III pulmonary arterial hypertension. In some embodiments, the individual is resistant, refractory or recurrent to at least two prior therapies for pulmonary hypertension. In some embodiments, the at least one, two or three prior therapies comprise an agent selected from the group consisting of a prostacyclin analogue, an endothelin-1 receptor antagonist, a phosphodiesterase 5 (PDE-5) inhibitor and a soluble guanylate cyclase (sGC) stimulator. In some embodiments, the nanoparticle composition is administered about once a week. In some embodiments, the nanoparticle composition is administered for at least about four weeks (e.g., at least about eight, twelve, sixteen, twenty-four, thirty-two, forty, or forty-eight weeks). In some embodiments, the concentration of rapamycin in the blood is at least about 2 ng/ml on the 5th day after administration of the nanoparticle composition. In some embodiments, the concentration of rapamycin in the blood is no more than about 20 ng/ml within 7 days or on the 7th day after administration of the nanoparticle composition. In some embodiments, the composition comprises more than about 50% of rapamycin in nanoparticle form. In some embodiments, rapamycin is the only pharmaceutically active agent useful for treating pulmonary hypertension that is administered to the individual. In some embodiments, the carrier protein is human serum albumin. In some embodiments, the average diameter of the nanoparticles in the composition is no greater than about 200 nm. In some embodiments, the weight ratio of the carrier protein to rapamycin in the nanoparticles is less than about 18:1. In some embodiments, the individual is human. In some embodiments, the individual has a high level of fibrosis in the lung. In some embodiments, the individual has a high level of angiogenesis in the lung. In some embodiments, the individual has increased fibrosis in the lung. In some embodiments, the individual has increased angiogenesis in the lung.

In some embodiments, the method is of ameliorating circulatory inadequacy (e.g., cardiac output) in an individual having pulmonary hypertension. In some embodiments, the dose of rapamycin in the composition is about 1-2, 2-3, 3-4, 4-5, 5-6, 6-7, 7-8, 8-9, 9-10 mg/m², about 2.5 mg/m² to about 10mg/m², or about 5 mg/m² to about 10 mg/m². In some embodiments, the dose of rapamycin in the composition is no more than about 10%, 9%, 8%, 7%, 6%, 5%, 4%, 3%, 2%, or 1% of the MTD of rapamycin in the composition. In some embodiments, the dose of rapamycin in the composition is about 1 mg/m² to about 5 mg/m², or about 2.5 mg/m² to about 5 mg/m². In some embodiments, the dose of rapamycin in the composition is about any of 1, 2, 3, 4, 5, 6, 7, 8, 9, or 10 mg/m². In some embodiments, the amount of rapamycin in the composition is an amount sufficient to produce a cardiac output (CO) of more than about 10%, 15%, 20%, 25, or 30% among a population of individuals treated with the composition. In some embodiments, the individual has a high level of fibrosis in the lung. In some embodiments, the individual has a high level of angiogenesis in the lung. In some embodiments, the individual has increased fibrosis in the lung. In some embodiments, the individual has increased angiogenesis in the lung. In some embodiments, the nanoparticle composition is administered for at least about four weeks (e.g., at least about eight, twelve, sixteen, twenty-four, thirty-two, forty, or forty-eight weeks).

In some embodiments, the method is of ameliorating circulatory inadequacy (e.g., cardiac output) in an individual having pulmonary hypertension, wherein the pulmonary hypertension is World Health Organization [WHO] Function Class III or IV pulmonary arterial hypertension. In some embodiments, the dose of rapamycin in the composition is about 1-2, 2-3, 3-4, 4-5, 5-6, 6-7, 7-8, 8-9, 9-10 mg/m², about 2.5 mg/m² to about 10 mg/m², or about 5 mg/m² to about 10 mg/m². In some embodiments, the dose of rapamycin in the composition is no more than about 10%, 9%, 8%, 7%, 6%, 5%, 4%, 3%, 2%, or 1% of the MTD of rapamycin in the composition. In some embodiments, the dose of rapamycin in the composition is about 1 mg/m² to about 5 mg/m², or about 2.5 mg/m² to about 5 mg/m². In some embodiments, the dose of rapamycin in the composition is about any of 1, 2, 3, 4, 5, 6, 7, 8, 9, or 10 mg/m². In some embodiments, the amount of rapamycin in the composition is an amount sufficient to produce a cardiac output (CO) of more than about 10%, 15%, 20%, 25, or 30% among a population of individuals treated with the composition. In some embodiments, the individual has a high level of fibrosis in the lung. In some embodiments, the individual has a high level of angiogenesis in the lung. In some embodiments, the individual has increased fibrosis in the lung. In some embodiments, the individual has increased angiogenesis in the lung. In some embodiments, the nanoparticle composition is administered for at least about four weeks (e.g., at least about eight, twelve, sixteen, twenty-four, thirty-two, forty, or forty-eight weeks).

In some embodiments, the method is of ameliorating circulatory inadequacy (e.g., cardiac output) in an individual having pulmonary hypertension, wherein the individual has had at least one prior therapy for pulmonary hypertension. In some embodiments, the dose of rapamycin in the composition is about 1-2, 2-3, 3-4, 4-5, 5-6, 6-7, 7-8, 8-9, 9-10 mg/m², about 2.5 mg/m² to about 10 mg/m², or about 5 mg/m² to about 10 mg/m². In some embodiments, the dose of rapamycin in the composition is no more than about 10%, 9%, 8%, 7%, 6%, 5%, 4%, 3%, 2%, or 1% of the MTD of rapamycin in the composition. In some embodiments, the dose of rapamycin in the composition is about 1 mg/m² to about 5 mg/m², or about 2.5 mg/m² to about 5 mg/m². In some embodiments, the dose of rapamycin in the composition is about any of 1, 2, 3, 4, 5, 6, 7, 8, 9, or 10 mg/m². In some embodiments, the amount of rapamycin in the composition is an amount sufficient to produce a cardiac output (CO) of more than about 10%, 15%, 20%, 25, or 30% among a population of individuals treated with the composition. In some embodiments, the individual has a high level of fibrosis in the lung. In some embodiments, the individual has a high level of angiogenesis in the lung. In some embodiments, the individual has increased fibrosis in the lung. In some embodiments, the individual has increased angiogenesis in the lung. In some embodiments, the nanoparticle composition is administered for at least about four weeks (e.g., at least about eight, twelve, sixteen, twenty-four, thirty-two, forty, or forty-eight weeks).

In some embodiments, the method is of ameliorating circulatory inadequacy (e.g., cardiac output) in an individual having pulmonary hypertension, wherein the individual has had at least one prior therapy for pulmonary hypertension, and wherein the pulmonary hypertension is World Health Organization [WHO] Function Class III or IV pulmonary arterial hypertension. In some embodiments, the dose of rapamycin in the composition is about 1-2, 2-3, 3-4, 4-5, 5-6, 6-7, 7-8, 8-9, 9-10 mg/m², about 2.5 mg/m² to about 10 mg/m², or about 5 mg/m² to about 10 mg/m². In some embodiments, the dose of rapamycin in the composition is no more than about 10%, 9%, 8%, 7%, 6%, 5%, 4%, 3%, 2%, or 1% of the MTD of rapamycin in the composition. In some embodiments, the dose of rapamycin in the composition is about 1 mg/m² to about 5 mg/m², or about 2.5 mg/m² to about 5 mg/m². In some embodiments, the dose of rapamycin in the composition is about any of 1, 2, 3, 4, 5, 6, 7, 8, 9, or 10 mg/m². In some embodiments, the amount of rapamycin in the composition is an amount sufficient to produce a cardiac output (CO) of more than about 10%, 15%, 20%, 25, or 30% among a population of individuals treated with the composition. In some embodiments, the individual has a high level of fibrosis in the lung. In some embodiments, the individual has a high level of angiogenesis in the lung. In some embodiments, the individual has increased fibrosis in the lung. In some embodiments, the individual has increased angiogenesis in the lung. In some embodiments, the nanoparticle composition is administered for at least about four weeks (e.g., at least about eight, twelve, sixteen, twenty-four, thirty-two, forty, or forty-eight weeks).

In some embodiments, the method is of reducing pulmonary vascular resistance (PVR) in an individual having pulmonary hypertension. In some embodiments, the dose of rapamycin in the composition is about 1-2, 2-3, 3-4, 4-5, 5-6, 6-7, 7-8, 8-9, 9-10 mg/m², about 2.5 mg/m² to about 10 mg/m², or about 5 mg/m² to about 10 mg/m². In some embodiments, the dose of rapamycin in the composition is no more than about 10%, 9%, 8%, 7%, 6%, 5%, 4%, 3%, 2%, or 1% of the MTD of rapamycin in the composition. In some embodiments, the dose of rapamycin in the composition is about 1 mg/m² to about 5 mg/m², or about 2.5 mg/m² to about 5 mg/m². In some embodiments, the dose of rapamycin in the composition is about any of 1, 2, 3, 4, 5, 6, 7, 8, 9, or 10 mg/m². In some embodiments, the amount of rapamycin in the composition is an amount sufficient to reduce pulmonary vascular resistance (PVR) by about 10%, 15%, 20%, 25%, or 30% among a population of individuals treated with the composition. In some embodiments, the individual has a high level of fibrosis in the lung. In some embodiments, the individual has a high level of angiogenesis in the lung. In some embodiments, the individual has increased fibrosis in the lung. In some embodiments, the individual has increased angiogenesis in the lung. In some embodiments, the nanoparticle composition is administered for at least about four weeks (e.g., at least about eight, twelve, sixteen, twenty-four, thirty-two, forty, or forty-eight weeks).

In some embodiments, the method is of reducing pulmonary vascular resistance (PVR) in an individual having pulmonary hypertension, wherein the pulmonary hypertension is World Health Organization [WHO] Function Class III or IV pulmonary arterial hypertension. In some embodiments, the dose of rapamycin in the composition is about 1-2, 2-3, 3-4, 4-5, 5-6, 6-7, 7-8, 8-9, 9-10 mg/m², about 2.5 mg/m² to about 10 mg/m², or about 5 mg/m² to about 10 mg/m². In some embodiments, the dose of rapamycin in the composition is no more than about 10%, 9%, 8%, 7%, 6%, 5%, 4%, 3%, 2%, or 1% of the MTD of rapamycin in the composition. In some embodiments, the dose of rapamycin in the composition is about 1 mg/m² to about 5 mg/m², or about 2.5 mg/m² to about 5 mg/m². In some embodiments, the dose of rapamycin in the composition is about any of 1, 2, 3, 4, 5, 6, 7, 8, 9, or 10 mg/m². In some embodiments, the amount of rapamycin in the composition is an amount sufficient to reduce pulmonary vascular resistance (PVR) by about 10%, 15%, 20%, 25%, or 30% among a population of individuals treated with the composition. In some embodiments, the individual has a high level of fibrosis in the lung. In some embodiments, the individual has a high level of angiogenesis in the lung. In some embodiments, the individual has increased fibrosis in the lung. In some embodiments, the individual has increased angiogenesis in the lung. In some embodiments, the nanoparticle composition is administered for at least about four weeks (e.g., at least about eight, twelve, sixteen, twenty-four, thirty-two, forty, or forty-eight weeks).

In some embodiments, the method is of reducing pulmonary vascular resistance (PVR) in an individual having pulmonary hypertension, wherein the individual has had at least one prior therapy for pulmonary hypertension. In some embodiments, the dose of rapamycin in the composition is about 1-2, 2-3, 3-4, 4-5, 5-6, 6-7, 7-8, 8-9, 9-10 mg/m², about 2.5 mg/m² to about 10 mg/m², or about 5 mg/m² to about 10 mg/m². In some embodiments, the dose of rapamycin in the composition is no more than about 10%, 9%, 8%, 7%, 6%, 5%, 4%, 3%, 2%, or 1% of the MTD of rapamycin in the composition. In some embodiments, the dose of rapamycin in the composition is about 1 mg/m² to about 5 mg/m², or about 2.5 mg/m² to about 5 mg/m². In some embodiments, the dose of rapamycin in the composition is about any of 1, 2, 3, 4, 5, 6, 7, 8, 9, or 10 mg/m². In some embodiments, the amount of rapamycin in the composition is an amount sufficient to reduce pulmonary vascular resistance (PVR) by about 10%, 15%, 20%, 25%, or 30% among a population of individuals treated with the composition. In some embodiments, the individual has a high level of fibrosis in the lung. In some embodiments, the individual has a high level of angiogenesis in the lung. In some embodiments, the individual has increased fibrosis in the lung. In some embodiments, the individual has increased angiogenesis in the lung. In some embodiments, the nanoparticle composition is administered for at least about four weeks (e.g., at least about eight, twelve, sixteen, twenty-four, thirty-two, forty, or forty-eight weeks).

In some embodiments, the method is of reducing pulmonary vascular resistance (PVR) in an individual having pulmonary hypertension, wherein the individual has had at least one prior therapy for pulmonary hypertension, and wherein the pulmonary hypertension is World Health Organization [WHO] Function Class III or IV pulmonary arterial hypertension. In some embodiments, the dose of rapamycin in the composition is about 1-2, 2-3, 3-4, 4-5, 5-6, 6-7, 7-8, 8-9, 9-10 mg/m², about 2.5 mg/m² to about 10 mg/m², or about 5 mg/m² to about 10 mg/m². In some embodiments, the dose of the rapamycin in the composition is no more than about 10%, 9%, 8%, 7%, 6%, 5%, 4%, 3%, 2%, or 1% of the MTD of rapamycin in the composition. In some embodiments, the dose of rapamycin in the composition is about 1 mg/m² to about 5 mg/m², or about 2.5 mg/m² to about 5 mg/m². In some embodiments, the dose of rapamycin in the composition is about any of 1, 2, 3, 4, 5, 6, 7, 8, 9, or 10 mg/m². In some embodiments, the amount of rapamycin in the composition is an amount sufficient to reduce pulmonary vascular resistance (PVR) by about 10%, 15%, 20%, 25%, or 30% among a population of individuals treated with the composition. In some embodiments, the individual has a high level of fibrosis in the lung. In some embodiments, the individual has a high level of angiogenesis in the lung. In some embodiments, the individual has increased fibrosis in the lung. In some embodiments, the individual has increased angiogenesis in the lung. In some embodiments, the nanoparticle composition is administered for at least about four weeks (e.g., at least about eight, twelve, sixteen, twenty-four, thirty-two, forty, or forty-eight weeks).

In some embodiments, the method is of improving six-minute walking distance (6MWD) performance in an individual having pulmonary hypertension. In some embodiments, the dose of rapamycin in the composition is about 1-2, 2-3, 3-4, 4-5, 5-6, 6-7, 7-8, 8-9, 9-10 mg/m², about 2.5 mg/m² to about 10 mg/m², or about 5 mg/m² to about 10 mg/m². In some embodiments, the dose of rapamycin in the composition is about 1 mg/m² to about 5 mg/m², or about 2.5 mg/m² to about 5 mg/m². In some embodiments, the dose of rapamycin in the composition is about any of 1, 2, 3, 4, 5, 6, 7, 8, 9, or 10 mg/m². In some embodiments, the amount of rapamycin in the composition is an amount sufficient to produce a six-minute walking distance (6MWD) performance of more than about 10%, 15%, 20%, 25%, 30%, or 35% among a population of individuals treated with the composition. In some embodiments, the dose of rapamycin in the composition is no more than about 10%, 9%, 8%, 7%, 6%, 5%, 4%, 3%, 2%, or 1% of the MTD of rapamycin in the composition. In some embodiments, the individual has a high level of fibrosis in the lung. In some embodiments, the individual has a high level of angiogenesis in the lung. In some embodiments, the individual has increased fibrosis in the lung. In some embodiments, the individual has increased angiogenesis in the lung. In some embodiments, the nanoparticle composition is administered for at least about four weeks (e.g., at least about eight, twelve, sixteen, twenty-four, thirty-two, forty, or forty-eight weeks).

In some embodiments, the method is of improving six-minute walking distance (6MWD) performance in an individual having pulmonary hypertension, wherein the pulmonary hypertension is World Health Organization [WHO] Function Class III or IV pulmonary arterial hypertension. In some embodiments, the dose of rapamycin in the composition is about 1-2, 2-3, 3-4, 4-5, 5-6, 6-7, 7-8, 8-9, 9-10 mg/m², about 2.5 mg/m² to about 10 mg/m², or about 5 mg/m² to about 10 mg/m². In some embodiments, the dose of rapamycin in the composition is no more than about 10%, 9%, 8%, 7%, 6%, 5%, 4%, 3%, 2%, or 1% of the MTD of rapamycin in the composition.. In some embodiments, the dose of rapamycin in the composition is about 1 mg/m² to about 5 mg/m², or about 2.5 mg/m² to about 5 mg/m². In some embodiments, the dose of rapamycin in the composition is about any of 1, 2, 3, 4, 5, 6, 7, 8, 9, or 10 mg/m². In some embodiments, the amount of rapamycin in the composition is an amount sufficient to produce a six-minute walking distance (6MWD) performance of more than about 10%, 15%, 20%, 25%, 30%, or 35% among a population of individuals treated with the composition. In some embodiments, the dose of rapamycin in the composition is no more than about 10%, 9%, 8%, 7%, 6%, 5%, 4%, 3%, 2%, or 1% of the MTD of rapamycin in the composition. In some embodiments, the individual has a high level of fibrosis in the lung. In some embodiments, the individual has a high level of angiogenesis in the lung. In some embodiments, the individual has increased fibrosis in the lung. In some embodiments, the individual has increased angiogenesis in the lung. In some embodiments, the nanoparticle composition is administered for at least about four weeks (e.g., at least about eight, twelve, sixteen, twenty-four, thirty-two, forty, or forty-eight weeks).

In some embodiments, the method is of improving six-minute walking distance (6MWD) performance in an individual having pulmonary hypertension, wherein the individual has had at least one prior therapy for pulmonary hypertension. In some embodiments, the dose of rapamycin in the composition is about 1-2, 2-3, 3-4, 4-5, 5-6, 6-7, 7-8, 8-9, 9-10 mg/m², about 2.5 mg/m² to about 10 mg/m², or about 5 mg/m² to about 10 mg/m². In some embodiments, the dose of rapamycin in the composition is about 1 mg/m² to about 5 mg/m², or about 2.5 mg/m² to about 5 mg/m². In some embodiments, the dose of rapamycin in the composition is about any of 1, 2, 3, 4, 5, 6, 7, 8, 9, or 10 mg/m². In some embodiments, the amount of rapamycin in the composition is an amount sufficient to produce a six-minute walking distance (6MWD) performance of more than about 10%, 15%, 20%, 25%, 30%, or 35% among a population of individuals treated with the composition. In some embodiments, the dose of rapamycin in the composition is no more than about 10%, 9%, 8%, 7%, 6%, 5%, 4%, 3%, 2%, or 1% of the MTD of rapamycin in the composition. In some embodiments, the individual has a high level of fibrosis in the lung. In some embodiments, the individual has a high level of angiogenesis in the lung. In some embodiments, the individual has increased fibrosis in the lung. In some embodiments, the individual has increased angiogenesis in the lung. In some embodiments, the nanoparticle composition is administered for at least about four weeks (e.g., at least about eight, twelve, sixteen, twenty-four, thirty-two, forty, or forty-eight weeks).

In some embodiments, the method is of improving six-minute walking distance (6MWD) performance in an individual having pulmonary hypertension, wherein the individual has had at least one prior therapy for pulmonary hypertension, and wherein the pulmonary hypertension is World Health Organization [WHO] Function Class III or IV pulmonary arterial hypertension. In some embodiments, the dose of rapamycin in the composition is about 1-2, 2-3, 3-4, 4-5, 5-6, 6-7, 7-8, 8-9, 9-10 mg/m², about 2.5 mg/m² to about 10 mg/m², or about 5 mg/m² to about 10 mg/m². In some embodiments, the dose of rapamycin in the composition is about 1 mg/m² to about 5 mg/m², or about 2.5 mg/m² to about 5 mg/m². In some embodiments, the dose of rapamycin in the composition is about any of 1, 2, 3, 4, 5, 6, 7, 8, 9, or 10 mg/m². In some embodiments, the amount of rapamycin in the composition is an amount sufficient to produce a six-minute walking distance (6MWD) performance of more than about 10%, 15%, 20%, 25%, 30%, or 35% among a population of individuals treated with the composition. In some embodiments, the dose of rapamycin in the composition is no more than about 10%, 9%, 8%, 7%, 6%, 5%, 4%, 3%, 2%, or 1% of the MTD of rapamycin in the composition. In some embodiments, the individual has a high level of fibrosis in the lung. In some embodiments, the individual has a high level of angiogenesis in the lung. In some embodiments, the individual has increased fibrosis in the lung. In some embodiments, the individual has increased angiogenesis in the lung. In some embodiments, the nanoparticle composition is administered for at least about four weeks (e.g., at least about eight, twelve, sixteen, twenty-four, thirty-two, forty, or forty-eight weeks).

In some embodiments, the method is of improving cardiac output (CO) or cardiac input (CI) in an individual having pulmonary hypertension. In some embodiments, the dose of rapamycin in the composition is about 1-2, 2-3, 3-4, 4-5, 5-6, 6-7, 7-8, 8-9, 9-10 mg/m², about 2.5 mg/m² to about 10 mg/m², or about 5 mg/m² to about 10 mg/m². In some embodiments, the dose of the mTOR inhibitor in the composition is about 1 mg/m² to about 5 mg/m², or about 2.5 mg/m² to about 5 mg/m². In some embodiments, the dose of rapamycin in the composition is about any of 1, 2, 3, 4, 5, 6, 7, 8, 9, or 10 mg/m². In some embodiments, the amount of rapamycin in the composition is an amount sufficient to produce a cardiac output (CO) or cardiac input (CI) of more than about 2.5%, 5%, 7.5%, or 10% among a population of individuals treated with the composition. In some embodiments, the dose of rapamycin in the composition is no more than about 10%, 9%, 8%, 7%, 6%, 5%, 4%, 3%, 2%, or 1% of the MTD of rapamycin in the composition. In some embodiments, the individual has a high level of fibrosis in the lung. In some embodiments, the individual has a high level of angiogenesis in the lung. In some embodiments, the individual has increased fibrosis in the lung. In some embodiments, the individual has increased angiogenesis in the lung. In some embodiments, the nanoparticle composition is administered for at least about four weeks (e.g., at least about eight, twelve, sixteen, twenty-four, thirty-two, forty, or forty-eight weeks).

In some embodiments, the method is of improving cardiac output (CO) or cardiac input (CI) in an individual having pulmonary hypertension, wherein the pulmonary hypertension is World Health Organization [WHO] Function Class III or IV pulmonary arterial hypertension. In some embodiments, the dose of rapamycin in the composition is about 1-2, 2-3, 3-4, 4-5, 5-6, 6-7, 7-8, 8-9, 9-10 mg/m², about 2.5 mg/m² to about 10 mg/m², or about 5 mg/m² to about 10 mg/m². In some embodiments, the dose of rapamycin in the composition is no more than about 10%, 9%, 8%, 7%, 6%, 5%, 4%, 3%, 2%, or 1% of the MTD of rapamycin in the composition. In some embodiments, the dose of rapamycin in the composition is about 1 mg/m² to about 5 mg/m², or about 2.5 mg/m² to about 5 mg/m². In some embodiments, the dose of rapamycin in the composition is about any of 1, 2, 3, 4, 5, 6, 7, 8, 9, or 10 mg/m². In some embodiments, the amount of rapamycin in the composition is an amount sufficient to produce a cardiac output (CO) or cardiac input (CI) of more than about 2.5%, 5%, 7.5%, or 10% among a population of individuals treated with the composition. In some embodiments, the dose of rapamycin in the composition is no more than about 10%, 9%, 8%, 7%, 6%, 5%, 4%, 3%, 2%, or 1% of the MTD of rapamycin in the composition. In some embodiments, the individual has a high level of fibrosis in the lung. In some embodiments, the individual has a high level of angiogenesis in the lung. In some embodiments, the individual has increased fibrosis in the lung. In some embodiments, the individual has increased angiogenesis in the lung. In some embodiments, the nanoparticle composition is administered for at least about four weeks (e.g., at least about eight, twelve, sixteen, twenty-four, thirty-two, forty, or forty-eight weeks).

In some embodiments, the method is of improving cardiac output (CO) or cardiac input (CI) in an individual having pulmonary hypertension, wherein the individual has had at least one prior therapy for pulmonary hypertension. In some embodiments, the dose of rapamycin in the composition is about 1-2, 2-3, 3-4, 4-5, 5-6, 6-7, 7-8, 8-9, 9-10 mg/m², about 2.5 mg/m² to about 10 mg/m², or about 5 mg/m² to about 10 mg/m². In some embodiments, the dose of rapamycin in the composition is about 1 mg/m² to about 5 mg/m², or about 2.5 mg/m² to about 5 mg/m². In some embodiments, the dose of rapamycin in the composition is about any of 1, 2, 3, 4, 5, 6, 7, 8, 9, or 10 mg/m². In some embodiments, the amount of rapamycin in the composition is an amount sufficient to produce a cardiac output (CO) or cardiac input (CI) of more than about 2.5%, 5%, 7.5%, or 10% among a population of individuals treated with the composition. In some embodiments, the dose of rapamycin in the composition is no more than about 10%, 9%, 8%, 7%, 6%, 5%, 4%, 3%, 2%, or 1% of the MTD of rapamycin in the composition. In some embodiments, the individual has a high level of fibrosis in the lung. In some embodiments, the individual has a high level of angiogenesis in the lung. In some embodiments, the individual has increased fibrosis in the lung. In some embodiments, the individual has increased angiogenesis in the lung. In some embodiments, the nanoparticle composition is administered for at least about four weeks (e.g., at least about eight, twelve, sixteen, twenty-four, thirty-two, forty, or forty-eight weeks).

In some embodiments, the method is of improving cardiac output (CO) or cardiac input (CI) in an individual having pulmonary hypertension, wherein the individual has had at least one prior therapy for pulmonary hypertension, and wherein the pulmonary hypertension is World Health Organization [WHO] Function Class III or IV pulmonary arterial hypertension. In some embodiments, the dose of rapamycin in the composition is about 1-2, 2-3, 3-4, 4-5, 5-6, 6-7, 7-8, 8-9, 9-10 mg/m², about 2.5 mg/m² to about 10 mg/m², or about 5 mg/m² to about 10 mg/m². In some embodiments, the dose of rapamycin in the composition is about 1 mg/m² to about 5 mg/m², or about 2.5 mg/m² to about 5 mg/m². In some embodiments, the dose of rapamycin in the composition is about any of 1, 2, 3, 4, 5, 6, 7, 8, 9, or 10 mg/m². In some embodiments, the amount of rapamycin in the composition is an amount sufficient to produce a cardiac output (CO) or cardiac input (CI) of more than about 2.5%, 5%, 7.5%, or 10% among a population of individuals treated with the composition. In some embodiments, the dose of rapamycin in the composition is no more than about 10%, 9%, 8%, 7%, 6%, 5%, 4%, 3%, 2%, or 1% of the MTD of rapamycin in the composition. In some embodiments, the individual has a high level of fibrosis in the lung. In some embodiments, the individual has a high level of angiogenesis in the lung. In some embodiments, the individual has increased fibrosis in the lung. In some embodiments, the individual has increased angiogenesis in the lung. In some embodiments, the nanoparticle composition is administered for at least about four weeks (e.g., at least about eight, twelve, sixteen, twenty-four, thirty-two, forty, or forty-eight weeks).

In some embodiments, the method is of delivering an effective amount of rapamycin to the lung in an individual having pulmonary hypertension, wherein the pulmonary hypertension is World Health Organization [WHO] Function Class III or IV pulmonary arterial hypertension. In some embodiments, the dose of rapamycin in the composition is no more than about 10%, 9%, 8%, 7%, 6%, 5%, 4%, 3%, 2%, or 1% of the MTD of rapamycin in the composition. In some embodiments, the individual has a lung concentration of rapamycin of at least about 250, 500, 750, 1000, 1100, or 1200 ng/g at 24 hours post administration. In some embodiments, the individual a lung concentration of rapamycin of at least about 50, 100, 150, 200, 250, 300, or 320 ng/g at 120 hours post administration. In some embodiments, the amount of rapamycin in the composition is an amount sufficient to produce a lung concentration of rapamycin of at least about 250, 500, 750, 1000, 1100, or 1200 ng/g at 24 hours post administration. In some embodiments, the amount of rapamycin in the composition is an amount sufficient to produce a lung concentration of rapamycin of at least about 50, 100, 150, 200, 250, 300, or 320 ng/g at 120 hours post administration. In some embodiments, the individual has a high level of fibrosis in the lung. In some embodiments, the individual has a high level of angiogenesis in the lung. In some embodiments, the individual has increased fibrosis in the lung. In some embodiments, the individual has increased angiogenesis in the lung. In some embodiments, the nanoparticle composition is administered for at least about four weeks (e.g., at least about eight, twelve, sixteen, twenty-four, thirty-two, forty, or forty-eight weeks).

In some embodiments, the method is of delivering an effective amount of rapamycin to the lung in an individual having pulmonary hypertension, wherein the individual has had at least one prior therapy for pulmonary hypertension, and wherein the pulmonary hypertension is World Health Organization [WHO] Function Class III or IV pulmonary arterial hypertension. In some embodiments, the dose of rapamycin in the composition is no more than about 10%, 9%, 8%, 7%, 6%, 5%, 4%, 3%, 2%, or 1% of the MTD of rapamycin in the composition. In some embodiments, the individual has a lung concentration of rapamycin of at least about 250, 500, 750, 1000, 1100, or 1200 ng/g at 24 hours post administration. In some embodiments, the individual a lung concentration of rapamycin of at least about 50, 100, 150, 200, 250, 300, or 320 ng/g at 120 hours post administration. In some embodiments, the amount of rapamycin in the composition is an amount sufficient to produce a lung concentration of rapamycin of at least about 250, 500, 750, 1000, 1100, or 1200 ng/g at 24 hours post administration. In some embodiments, the amount of rapamycin in the composition is an amount sufficient to produce a lung concentration of rapamycin of at least about 50, 100, 150, 200, 250, 300, or 320 ng/g at 120 hours post administration. In some embodiments, the individual has a high level of fibrosis in the lung. In some embodiments, the individual has a high level of angiogenesis in the lung. In some embodiments, the individual has increased fibrosis in the lung. In some embodiments, the individual has increased angiogenesis in the lung. In some embodiments, the nanoparticle composition is administered for at least about four weeks (e.g., at least about eight, twelve, sixteen, twenty-four, thirty-two, forty, or forty-eight weeks).

In some embodiments, the method is of improving quality of life in an individual having pulmonary hypertension. In some embodiments, the dose of rapamycin in the composition is about 1-2, 2-3, 3-4, 4-5, 5-6, 6-7, 7-8, 8-9, 9-10 mg/m², about 2.5 mg/m² to about 10 mg/m², or about 5 mg/m² to about 10 mg/m². In some embodiments, the pulmonary hypertension is World Health Organization [WHO] Function Class III or IV pulmonary arterial hypertension. In some embodiments, the individual has a high level of fibrosis in the lung. In some embodiments, the individual has a high level of angiogenesis in the lung. In some embodiments, the individual has increased fibrosis in the lung. In some embodiments, the individual has increased angiogenesis in the lung. In some embodiments, the nanoparticle composition is administered for at least about four weeks (e.g., at least about eight, twelve, sixteen, twenty-four, thirty-two, forty, or forty-eight weeks). In some embodiments, the improved quality of life is characterized by an improved quality of life score after treatment as compared to corresponding score assessed prior to the administration of mTOR inhibitor nanoparticle. In some embodiments, the quality of life score is based upon a self-assessing questionnaire (e.g., emPHasis-10). *See* Yorke et al., Eur Respir J. 2014 Apr; 43(4): 1106-1113. In some embodiments, the total quality of life score is reduced by at least about 10%, 20%, 30%, 40%, or 50% after mTOR administration as compared to the corresponding baseline score prior to the administration.

### Dosing and Method of Administration

The dose of the inventive composition administered to an individual (such as a human) may vary with the particular composition, the method of administration, and the particular stage of pulmonary hypertension being treated. The amount should be sufficient to produce a desirable response, such as a therapeutic or prophylactic response against pulmonary hypertension. In some embodiments, the amount of the composition is a therapeutically effective amount. In some embodiments, that amount of the composition is a prophylactically effective amount. In some embodiments, the amount of the mTOR inhibitor in the composition is below the level that induces a toxicological effect (*i.e.,* an effect above a clinically acceptable level of toxicity) or is at a level where a potential side effect can be controlled or tolerated when the composition is administered to the individual.

In some embodiments, the amount of the mTOR inhibitor in the composition is an amount sufficient to increase basal AKT activity, increase AKT phosphorylation, increase PI3-kinase activity, increase the length of activation of AKT (e.g., activation induced by exogenous IGF-1), inhibit serine phosphorylation of IRS-1, inhibit IRS-1 degradation, inhibit or alter CXCR4 subcellular localization, inhibit VEGF secretion, decrease expression of cyclin D2, decrease expression of survivin, inhibit IL-6-induced multiple myeloma cell growth, inhibit cell proliferation, increase apoptosis, increase cell cycle arrest, increase cleavage of poly(ADPribose) polymerase, increase cleavage of caspase-8/caspase-9, alter or inhibit signaling in the phosphatidylinositol 3-kinase/AKT/mTOR and/or cyclin D1/retinoblastoma pathways, inhibit angiogenesis, and/or inhibit osteoclast formation.

In some embodiments, the amount of the in the composition is an amount sufficient to produce a cardiac output (CO) or cardiac input (CI) of more than about any of 10%, 15%, 20%, 25%, 30%, 35%, 40%, 45% or 50% among a population of individuals treated with the mTOR inhibitor nanoparticle composition. In some embodiments, the individual is administered with the composition for a period of no more than about 4, 6, 8, 10, 12, 14, or 16 weeks when an about 10%, 15%, 20%, 25%, 30%, 35%, 40%, 45% or 50% increase in CO or CI appears. In some embodiments, the individual is administered with the composition for a period of more than about 4, 6, 8, 10, 12, 14, or 16 weeks when an about 10%, 15%, 20%, 25%, 30%, 35%, 40%, 45% or 50% increase in CO or CI appears. In some embodiments, the individual is administered with the composition for a period of more than about 1, 2, 3, 4, 5, 6, 7, 8, 9, 10, 11, or 12 months when an about 10%, 15%, 20%, 25%, 30%, 35%, 40%, 45% or 50% increase in CO or CI appears. In some embodiments, the individual is administered with the composition for a period of more than about 1, 2, 3, 4, 5, 6, or 7 years when an about 10%, 15%, 20%, 25%, 30%, 35%, 40%, 45% or 50% increase in CO or CI appears.

In some embodiments, the amount of the mTOR inhibitor in the composition is an amount sufficient to reduce pulmonary vascular resistance (PVR) by about any of 10%, 15%, 20%, 25%, 30%, 35%, 40%, 45% or 50% among a population of individuals treated with the composition (such as rapamycin/albumin nanoparticle composition). In some embodiments, the individual is administered with the composition for a period of no more than about 4, 6, 8, 10, 12, 14, or 16 weeks when an about 10%, 15%, 20%, 25%, 30%, 35%, 40%, 45% or 50% decrease in PVR appears. In some embodiments, the individual is administered with the composition for a period of more than about 4, 6, 8, 10, 12, 14, or 16 weeks when an about 10%, 15%, 20%, 25%, 30%, 35%, 40%, 45% or 50% decrease in PVR appears. In some embodiments, the individual is administered with the composition for a period of more than about 1, 2, 3, 4, 5, 6, 7, 8, 9, 10, 11, or 12 months when an about 10%, 15%, 20%, 25%, 30%, 35%, 40%, 45% or 50% decrease in PVR appears. In some embodiments, the individual is administered with the composition for a period of more than about 1, 2, 3, 4, 5, 6, or 7 years when an about 10%, 15%, 20%, 25%, 30%, 35%, 40%, 45% or 50% decrease in PVR appears.

In some embodiments, the amount of the mTOR inhibitor in the composition is an amount sufficient to produce a six-minute walking distance (6MWD) performance of more than about any of 10%, 15%, 20%, 25%, 30%, 35%, 40%, 45% or 50% among a population of individuals treated with the composition. In some embodiments, the individual is administered with the composition for a period of no more than about 4, 6, 8, 10, 12, 14, or 16 weeks when an about 10%, 15%, 20%, 25%, 30%, 35%, 40%, 45% or 50% increase in 6MWD appears. In some embodiments, the individual is administered with the composition for a period of more than about 4, 6, 8, 10, 12, 14, or 16 weeks when an about 10%, 15%, 20%, 25%, 30%, 35%, 40%, 45% or 50% increase in 6MWD appears. In some embodiments, the individual is administered with the composition for a period of more than about 1, 2, 3, 4, 5, 6, 7, 8, 9, 10, 11, or 12 months when an about 10%, 15%, 20%, 25%, 30%, 35%, 40%, 45% or 50% increase in 6MWD appears. In some embodiments, the individual is administered with the composition for a period of more than about 1, 2, 3, 4, 5, 6, or 7 years when an about 10%, 15%, 20%, 25%, 30%, 35%, 40%, 45% or 50% increase in 6MWD appears.

In some embodiments, , the amount of the mTOR inhibitor in the composition is an amount sufficient to produce a favorable result in an individual or among a population of individuals treated with the composition (such as rapamycin/albumin nanoparticle composition) in any one or more of the assessment as following: 1) Doppler-echocardiographic assessment of right ventricular structure and function, 2) Pulmonary function test (such as forced vital capacity (FVC)); 3) NT Pro-BNP; 4) CRP; 5) Troponin; 6) fasting lipids; 7) WHO Functional class; 8) pulmonary artery pressure (PAP); 9) pulmonary artery occlusion pressure (PAOP); 10) pulmonary capillary wedge pressure (PCWP); or 11) central venous pressure (CVP). In some embodiments, the favorable result comprises an improvement in WHO Function class. In some embodiments, the improvement comprises a change from WHO Function class III PAH to WHO Function Class II PAH. In some embodiments, the individual is administered with the composition for a period of no more than about 4, 6, 8, 10, 12, 14, or 16 weeks when a favorable result appears. In some embodiments, the individual is administered with the composition for a period of more than about 4, 6, 8, 10, 12, 14, or 16 weeks when a favorable result appears. In some embodiments, the individual is administered with the composition for a period of more than about 1, 2, 3, 4, 5, 6, 7, 8, 9, 10, 11, or 12 months when a favorable result appears. In some embodiments, the individual is administered with the composition for a period of more than about 1, 2, 3, 4, 5, 6, or 7 years when a favorable result appears. In some embodiments, the individual exhibits a change of about at least 2%, 5%, 10%, 15%, 20%, 25%, 30%, 35%, 40%, 45% or 50% in any of the assessments as described above after being treated with the composition as compared to the baseline (*i.e.,* prior to the treatment). In some embodiments, the individual has a change of about at least 2%, 5%, 10%, 15%, 20%, 25%, 30%, 35%, 40%, 45% or 50% in any of the symptoms as described above after being treated with the composition for a period of no more than about 4, 6, 8, 10, 12, 14, or 16 weeks.

In some embodiments, the amount of the mTOR inhibitor in the composition is an amount that produces a favorable safety profile in the individual having pulmonary hypertension. In some embodiments, the favorable safety profile is maintained for at least about 1, 2, 3, 4, 5, 6, 7, 8, 9, 10, 11, 12, 13, 14, 15, or 16 weeks. In some embodiments, the favorable safety profile does not comprise a serious adverse event (SAE). In some embodiments, the serious adverse event comprises a fatal condition, a life-threatening condition, a condition requires in-patient hospitalization or prolongation of existing hospitalization, a condition resulting in persistent or significant disability or incapacity, a condition causing congenital anomaly or birth defect, and/or other medically important serious event. In some embodiments, the favorable safety profile does not comprise more than about 0, 1, 2, 3, 4, 5, 6, 7, or 8 adverse events in a period of about 1, 2, 3, 4, 5, 6, 7, 8, 9, 10, 11, 12, 13, 14, 15, or 16 weeks after initiation of the mTOR composition administration. In some embodiments, the adverse event comprises some of the adverse events defined in Common Terminology Criteria for Adverse Events (CTCAE) version 4.0 or a higher version. For example, in some embodiments, the adverse event comprises all Grade 2 or above adverse event defined in Common Terminology Criteria for Adverse Events (CTCAE) version 4.0 or a higher version. In some embodiments, the adverse event comprises all Grade 3 or above adverse event defined in Common Terminology Criteria for Adverse Events (CTCAE) version 4.0 or a higher version. In some embodiments, the adverse event comprises all of the adverse events (*i.e.*, all Grade 1 or above adverse events) defined in Common Terminology Criteria for Adverse Events (CTCAE) version 4.0 or a higher version. In some embodiments, the adverse event comprises Grade 1 or above thrombocytopenia, Grade 2 or above rash, Grade 1 or above paresthesia, Grade 1 or above hypertriglyceridemia or hypercholesterolemia, Grade 1 or above diarrhea, Grade 3 or above cellulitis/infection requiring IV antibodies.

In some embodiments, the amount of the mTOR inhibitor in the composition is included in any of the following ranges: about 0.1 to about 1 mg, about 1 to about 3 mg, about 3 to about 6 mg, about 6 to about 9 mg, about 9 to about 12 mg, about 12 to about 15 mg, or about 15 to about 18 mg. In some embodiments, the amount of rapamycin in the effective amount of the composition (*e.g.*, a unit dosage form) is in the range of about 0.1 mg to about 18 mg, such as about 1 mg to about 18 mg. In some embodiments, the concentration of the rapamycin in the composition is dilute (about 0.1 mg/ml) or concentrated (about 100 mg/ml), including for example any of about 0.1 to about 50 mg/ml, about 0.1 to about 20 mg/ml, about 1 to about 10 mg/ml, about 2 mg/ml to about 8 mg/ml, about 4 to about 6 mg/ml, about 5 mg/ml. In some embodiments, the concentration of the rapamycin is at least about any of 0.5 mg/ml, 1.3 mg/ml, 1.5 mg/ml, 2 mg/ml, 3 mg/ml, 4 mg/ml, 5 mg/ml, 6 mg/ml, 7 mg/ml, 8 mg/ml, 9 mg/ml, 10 mg/ml, 15 mg/ml, 20 mg/ml, 25 mg/ml, 30 mg/ml, 40 mg/ml, or 50 mg/ml.

Exemplary effective amounts of the mTOR inhibitor in the nanoparticle composition include, but are not limited to, about any of 1 mg/m², 1.5 mg/m², 2 mg/m², 2.5 mg/m², 3 mg/m², 3.5 mg/m², 4 mg/m², 4.5 mg/m², 5 mg/m², 5.5 mg/m², 6 mg/m², 6.5 mg/m², 7 mg/m², 7.5 mg/m², 8 mg/m², 8.5 mg/m², 9 mg/m², 9.5 mg/m², or 10 mg/m². In various embodiments, the composition includes no more than about any of 9.5 mg/m², 9 mg/m², 8.5 mg/m², 8 mg/m², 7.5 mg/m², 7 mg/m², 6.5 mg/m², 6 mg/m², 5.5 mg/m², 5 mg/m², 4.5 mg/m², 4 mg/m², 3.5 mg/m², 3 mg/m², 2.5 mg/m², 2 mg/m², 1.5 mg/m², or 1 mg/m² of the mTOR inhibitor. In some embodiments, the amount of the mTOR inhibitor per administration is less than about any of 10 mg/m², 9.5 mg/m², 9 mg/m², 8.5 mg/m², 8 mg/m², 7.5 mg/m², 7 mg/m², 6.5 mg/m², 6 mg/m², 5.5 mg/m², 5 mg/m², 4.5 mg/m², 4 mg/m², 3.5 mg/m², 3 mg/m², 2.5 mg/m², 2 mg/m², or 1.5 mg/m². In some embodiments, the effective amount of the mTOR inhibitor in the composition is included in any of the following ranges: about 1-2, 2-3, 3-4, 4-5, 5-6, 6-7, 7-8, 8-9, 9-10 mg/m², about 1 to about 2.5 mg/m², about 2.5 to about 5 mg/m², about 5 to about 7.5 mg/m², or about 7.5 to about 10 mg/m². In some embodiments, the effective amount of the mTOR inhibitor in the composition is about 1 to about 5 mg/m², or about 5 mg/m², about 10 mg/m². In some embodiments, the effective amount of the mTOR inhibitor in the composition is about 5 mg/m².

In some embodiments of any of the above aspects, the effective amount of the mTOR inhibitor in the composition includes at least about any of 0.001 mg/kg, 0.005 mg/kg, 0.01 mg/kg, 0.02 mg/kg, 0.05 mg/kg, 0.1 mg/kg, 0.12 mg/kg, 0.14 mg/kg, 0.16 mg/kg, 0.18 mg/kg, 0.20 mg/kg, 0.22 mg/kg or 0.24 mg/kg. In various embodiments, the effective amount of the mTOR inhibitor in the composition includes no more than about or less than about any of 0.24 mg/kg, 0.22 mg/kg, 0.2 mg/kg, 0.18 mg/kg, 0.16 mg/kg, 0.14 mg/kg, 0.12 mg/kg, 0.10 mg/kg, 0.05 mg/kg, 0.02 mg/kg, or 0.01 mg/kg of the mTOR inhibitor.

In some embodiments, the dose of the mTOR inhibitor in the composition is no more than about 50%, 40%, 30%, 20%, 15%, 10%, 9%, 8%, 7%, 6%, 5%, 4%, 3%, 2%, or 1% of the MTD of the mTOR inhibitor in the composition.

In some embodiments, the concentration of the mTOR inhibitor in the blood is at least about 2, 3, 4, 5, 6, 7, or 8 ng/ml upon or within 1, 2, 3, 4, 5, 6, or 7 days after administration of the nanoparticle composition. In some embodiments, the concentration of the mTOR inhibitor in the blood is at least about 2 ng/ml upon on the 5th day after administration of the nanoparticle composition. In some embodiments, the concentration of the mTOR inhibitor in the blood is at least about 2, 3, 4, 5, 6, 7, or 8 ng/ml at 1, 2, 3, or 4 days before administration of next dose of the nanoparticle composition.

In some embodiments, the concentration of the mTOR inhibitor in the blood is no more than about 30, 28, 26, 24, 22, 20, 19, 18, 17, 16, 15, 14, 13, 12, 11, 10, 9, or 8 ng/ml upon or within 3, 4, 5, 6, or 7 days after administration of the nanoparticle composition. In some embodiments, the concentration of the mTOR inhibitor in the blood is no more than about 20 ng/ml upon or within 7 days after administration of the nanoparticle composition. In some embodiments, the concentration of the mTOR inhibitor in the blood is no more than about 30, 28, 26, 24, 22, 20, 19, 18, 17, 16, 15, 14, 13, 12, 11, 10, 9, or 8 ng/ml at 1, 2, 3, or 4 days before administration of next dose of the nanoparticle composition.

In some embodiments, the individual has or maintains the mTOR inhibitor trough level (such as an average trough level) of at least about 0.1 ng/ml (such as at least about 0.5 ng/ml, or 1 ng/ml) during a treatment period. In some embodiments, the individual has or maintains the mTOR inhibitor trough level (such as an average trough level) of no more about 300 ng/ml (such as no more than about 100 ng/ml, 50 ng/ml, or 20 ng/ml) during a treatment period. In some embodiments, the individual has or maintains the mTOR inhibitor trough level (such as an average trough level) of about 0.1-300 ng/ml (such as about 0.5-50 ng/ml, or 1-20 ng/ml) during a treatment period. In some embodiments, the individual is administered the mTOR inhibitor nanoparticle composition at a frequency of about daily to once every two weeks (such as a frequency of about once a week) during the treatment period.

In some embodiments, the trough concentration of the mTOR inhibitor in the blood is at least about 2, 3, 4, 5, 6, 7, or 8 ng/ml. In some embodiments, the trough concentration of the mTOR inhibitor in the blood is at least about 2 ng/ml. In some embodiments, the trough concentration of the mTOR inhibitor in the blood is no more than about 30, 28, 26, 24, 22, 20, 19, 18, 17, 16, 15, 14, 13, 12, 11, 10, 9, or 8 ng/ml. In some embodiments, the trough concentration of the mTOR inhibitor in the blood is no more than about 20 ng/ml.

In some embodiments, the nanoparticle composition is administered for no more than once a week, for example, weekly without break; weekly, three out of four weeks; once every three weeks; once every two weeks; or weekly, two out of three weeks. In some embodiments, the composition is administered about once every 2 weeks, once every 3 weeks, once every 4 weeks, once every 6 weeks, or once every 8 weeks. In some embodiments, the nanoparticle composition is administered no more than twice a week, three times a week, four times a week, five times a week, six times a week. In some embodiments, the composition is administered at least once a week. In some embodiments, the composition is administered at least about any of 1x, 2x, 3x, 4x, 5x, 6x, or 7x (*i.e.,* daily) a week. In some embodiments, the intervals between each administration are less than about any of 3 months, 1 month, 20 days, 15, days, 12 days, 10 days, 9 days, 8 days, 7 days, 6 days, 5 days, 4 days, 3 days, 2 days, or 1 day. In some embodiments, the intervals between each administration are more than about any of 1 month, 2 months, 3 months, 4 months, 5 months, 6 months, 8 months, or 12 months. In some embodiments, there is no break in the dosing schedule. In some embodiments, the interval between each administration is no more than about a week.

The administration of the composition can be extended over an extended period of time, such as from about four weeks up to about seven years. In some embodiments, the composition is administered over a period of at least about 1, 2, 3, 4, 5, 6, 7, 8, 9, 10, 11, 12, 14, 16, 20, 24, 28, 32, 36, 40, 44, or 48 weeks. In some embodiments, the composition is administered over a period of at least about 1, 2, 3, 4, 5, 6, 7, 8, 9, 10, 11, 12, 18, 24, 30, 36, 48, 60, 72, or 84 months. In some embodiments, the rapamycin is administered over a period of at least four weeks, wherein the interval between each administration is no more than about a week, and wherein the dose of the rapamycin at each administration is about 1 mg/m² to about 10 mg/m² or about 5 mg/m² to about 10 mg/m². In some embodiments, the composition is administered no more than about 2, 3, 4, 5, 6, 7, 8, 9, 10, 11, 12, 14, 16, 20, 24, 28, or 32 weeks. In some embodiments, the composition is administered no more than about 1, 2, 3, 4, 5, 6, 7, 8, 9, 10, 11, 12, 18, 24, 30, 36, 48, 60, 72, or 84 months.

In some embodiments, the rapamycin is administered over a period of at least four weeks (e.g., at least about eight, twelve, sixteen, twenty-four, thirty-two, forty, or forty-eight weeks), wherein the interval between each administration is no more than about a week, and wherein the dose of the rapamycin at each administration is about 1 mg/m² to about 10 mg/m² or about 5 mg/m² to about 10 mg/m².

The dose of the nanoparticle composition may be discontinued or interrupted, with or without dose reduction, to manage adverse drug reactions.

In some embodiments, the method comprises an induction phase and a maintenance phase. In some embodiments, the induction phase comprises administering the composition weekly. In some embodiments, the maintenance phase comprises administering the composition less than once every week *(e.g.,* once every two week, *e.g.,* once every three weeks). In some embodiments, the maintenance phase comprises at least 1, 2, 4, 6, 8, 10, 12, 14, 16, 18, or 20 weeks.

According to the invention, the route of administration is subcutaneous. In some embodiments, an effective amount of the composition is administered over a period of less than 30 minutes. In some embodiments, an effective amount of the composition is administered over a period of about any of 30 minutes, 20 minutes, 15 minutes, 10 minutes, 5 minutes, or 1 minute.

In some embodiments, the composition allows infusion of the composition to an individual over an infusion time that is shorter than about 24 hours. For example, in some embodiments, the composition (such as rapamycin/albumin nanoparticle composition) is administered over an infusion period of less than about any of 24 hours, 12 hours, 8 hours, 5 hours, 3 hours, 2 hours, 1 hour, 30 minutes, 20 minutes, or 10 minutes. In some embodiments, the composition (such as rapamycin/albumin nanoparticle composition) is administered over an infusion period of about 30 minutes.

In some embodiments, a taxane is not contained in the composition. In some embodiments, the rapamycin is the only pharmaceutically active agent for the treatment of pulmonary hypertension that is contained in the composition.

In some embodiments, sustained continuous release formulation of the composition may be used. In some embodiments, the rapamycin is coating a stent or is administered using a stent. In some embodiments, the rapamycin is not coating a stent or is not administered using a stent.

### Nanoparticle Compositions

The mTOR inhibitor nanoparticle compositions described herein comprise nanoparticles comprising (in various embodiments consisting essentially of or consisting of) an mTOR inhibitor (such as a limus drug, *e.g.*, rapamycin or a derivative thereof) and an albumin (such as human serum albumin). The nanoparticle composition for use of the present invention comprises rapamycin and a carrier protein. Nanoparticles of poorly water soluble drugs (such as macrolides) have been disclosed in, for example, U. S. Pat. Nos.5,916,596; 6,506,405; 6,749,868, 6,537,579, 7,820,788, and 8,911,786, and also in U. S. Pat. Pub. Nos. 2006/0263434, and 2007/0082838; PCT Patent Application WO08/137148.

In some embodiments, the pharmaceutical compositions further comprise an agent or agents for enhancing dissolution of dried forms of the compositions and/or enhancing the stability of the composition. In some embodiments, the additional agent or agents comprise a saccharide. The saccharide may be, but is not limited to, monosaccharides, disaccharides, polysaccharides, and derivatives or modifications thereof. The saccharide may be, for example, any of mannitol, sucrose, fructose, lactose, maltose, dextrose, or trehalose. In some embodiments, the additional agent or agents comprise glycine. The present invention therefore in one aspect provides a pharmaceutical composition for use of the invention suitable for subcutaneous administration to an individual comprising a) nanoparticles comprising rapamycin and an albumin, and b) a saccharide.

In some embodiments, the saccharide is present in an amount that is effective to increase the stability of the nanoparticles in the composition as compared to a nanoparticle composition without the saccharide. In some embodiments, the saccharide is in an amount that is effective to improve filterability of the nanoparticle composition as compared to a composition without the saccharide.

In some embodiments, the saccharide is present in an amount effective to enhance the solubility of the pharmaceutical composition. In some embodiments, the enhanced solubility comprises improved rate of dissolution of a dried form of the nanoparticle composition after addition of a reconstituting solution.

In some embodiments, the saccharide is present in an amount that reduces the incidence or severity of post-administration side effects when the nanoparticle composition is administered subcutaneously. For example, in some embodiments, the side effect is rash and the composition comprises nanoparticles comprising rapamycin and an albumin and the saccharide is present in an amount that reduces the incidence of rash after subcutaneous administration of the nanoparticle composition.

In some embodiments, the composition comprises nanoparticles with an average or mean diameter of no greater than about 1000 nanometers (nm), such as no greater than about any of 900, 800, 700, 600, 500, 400, 300, 200, and 100 nm. In some embodiments, the average or mean diameters of the nanoparticles is no greater than about 200 nm. In some embodiments, the average or mean diameters of the nanoparticles is no greater than about 150 nm. In some embodiments, the average or mean diameters of the nanoparticles is no greater than about 100 nm. In some embodiments, the average or mean diameter of the nanoparticles is about 10 to about 400 nm. In some embodiments, the average or mean diameter of the nanoparticles is about 10 to about 150 nm. In some embodiments, the average or mean diameter of the nanoparticles is about 40 to about 120 nm. In some embodiments, the average or mean diameter of the nanoparticles are no less than about 50 nm. In some embodiments, the nanoparticles are sterile-filterable.

In some embodiments, the particles (such as nanoparticles) described herein have an average or mean diameter of no greater than about any of 1000, 900, 800, 700, 600, 500, 400, 300, 200, 150, 120, and 100 nm. In some embodiments, the average or mean diameter of the particles is no greater than about 200 nm. In some embodiments, the average or mean diameter of the particles is between about 20 nm to about 400 nm. In some embodiments, the average or mean diameter of the particles is between about 40 nm to about 200 nm. In some embodiments, the average or mean diameter of the nanoparticles is about 100-120 nm, for example about 100 nm. In some embodiments, the average mean diameter of the particles is less than or equal to 120 nm. In some embodiments, the average mean diameter of the particles is about 100-120 nm, for example about 100 nm. In some embodiments, the particles are sterile-filterable.

In some embodiments, the nanoparticles in the composition described herein have an average diameter of no greater than about 200 nm, including for example no greater than about any one of 190, 180, 170, 160, 150, 140, 130, 120, 110, 100, 90, 80, 70, or 60 nm. In some embodiments, at least about 50% (for example at least about any one of 60%, 70%, 80%, 90%, 95%, or 99%) of the nanoparticles in the composition have a diameter of no greater than about 200 nm, including for example no greater than about any one of 190, 180, 170, 160, 150, 140, 130, 120, 110, 100, 90, 80, 70, or 60 nm. In some embodiments, at least about 50% (for example at least any one of 60%, 70%, 80%, 90%, 95%, or 99%) of the nanoparticles in the composition fall within the range of about 10 nm to about 400 nm, including for example about 10 nm to about 200 nm, about 20 nm to about 200 nm, about 30 nm to about 180 nm, about 40 nm to about 150 nm, about 40 nm to about 120 nm, and about 60 nm to about 100 nm.

Methods of determining average particle sizes are known in the art, for example, dynamic light scattering (DLS) has been routinely used in determining the size of submicrometre-sized particles based. International Standard ISO22412 Particle Size Analysis - Dynamic Light Scattering, International Organisation for Standardisation (ISO) 2008 and Dynamic Light Scattering Common Terms Defined, Malvern Instruments Limited, 2011. In some embodiments, the particle size is measured as the volume-weighted mean particle size (Dv50) of the nanoparticles in the composition.

In some embodiments, the nanoparticles comprise the mTOR inhibitor associated with the albumin. In some embodiments, the nanoparticles comprise the mTOR inhibitor coated with the albumin.

In some embodiments, the albumin has sulfhydryl groups that can form disulfide bonds. In some embodiments, at least about 5% (including for example at least about any one of 10%, 15%, 20%, 25%, 30%, 40%, 50%, 60%, 70%, 80%, or 90%) of the albumin in the nanoparticle portion of the composition are crosslinked (for example crosslinked through one or more disulfide bonds).

In some embodiments, the nanoparticles comprising the mTOR inhibitor are associated *(e.g.,* coated) with an albumin (such as human albumin or human serum albumin). In some embodiments, the composition comprises the mTOR inhibitor in both nanoparticle and non-nanoparticle forms *(e.g.,* in the form of solutions or in the form of soluble albumin/nanoparticle complexes), wherein at least about any one of 50%, 60%, 70%, 80%, 90%, 95%, or 99% of the mTOR inhibitor in the composition are in nanoparticle form. In some embodiments, the mTOR inhibitor in the nanoparticles constitutes more than about any one of 50%, 60%, 70%, 80%, 90%, 95%, or 99% of the nanoparticles by weight. In some embodiments, the nanoparticles have a non-polymeric matrix. In some embodiments, the nanoparticles comprise a core of the mTOR inhibitor that is substantially free of polymeric materials (such as polymeric matrix).

In some embodiments, the composition comprises an albumin in both nanoparticle and non-nanoparticle portions of the composition, wherein at least about any one of 50%, 60%, 70%, 80%, 90%, 95%, or 99% of the albumin in the composition are in non-nanoparticle portion of the composition.

In some embodiments, the weight ratio of an albumin (such as human albumin or human serum albumin) and the mTOR inhibitor in the composition is about 18:1 or less, such as about 15:1 or less, for example about 10:1 or less. In some embodiments, the weight ratio of an albumin (such as human albumin or human serum albumin) and the mTOR inhibitor in the composition falls within the range of any one of about 1:1 to about 18:1, about 2:1 to about 15:1, about 3:1 to about 13:1, about 4:1 to about 12:1, about 5:1 to about 10:1. In some embodiments, the weight ratio of an albumin and the mTOR inhibitor in the nanoparticle portion of the composition is about any one of 1:2, 1:3, 1:4, 1:5, 1:9, 1:10, 1:15, or less. In some embodiments, the weight ratio of the albumin (such as human albumin or human serum albumin) and the mTOR inhibitor in the composition is any one of the following: about 1:1 to about 18:1, about 1:1 to about 15:1, about 1:1 to about 12:1, about 1:1 to about 10:1, about 1:1 to about 9:1, about 1:1 to about 8:1, about 1:1 to about 7:1, about 1:1 to about 6:1, about 1:1 to about 5:1, about 1:1 to about 4:1, about 1:1 to about 3:1, about 1:1 to about 2:1, about 1:1 to about 1:1.

In some embodiments, the composition (such as rapamycin/albumin nanoparticle composition) comprises one or more of the above characteristics.

The nanoparticles described herein may be present in a dry formulation (such as lyophilized composition) or suspended in a biocompatible medium. Suitable biocompatible media include, but are not limited to, water, buffered aqueous media, saline, buffered saline, optionally buffered solutions of amino acids, optionally buffered solutions of proteins, optionally buffered solutions of sugars, optionally buffered solutions of vitamins, optionally buffered solutions of synthetic polymers, lipid-containing emulsions, and the like.

In some embodiments, the pharmaceutically acceptable carrier comprises an albumin (such as human albumin or human serum albumin). The albumin may either be natural in origin or synthetically prepared. In some embodiments, the albumin is human albumin or human serum albumin. In some embodiments, the albumin is a recombinant albumin.

Human serum albumin (HSA) is a highly soluble globular protein of Mᵣ 65K and consists of 585 amino acids. HSA is the most abundant protein in the plasma and accounts for 70-80 % of the colloid osmotic pressure of human plasma. The amino acid sequence of HSA contains a total of 17 disulfide bridges, one free thiol (Cys 34), and a single tryptophan (Trp 214). Intravenous use of HSA solution has been indicated for the prevention and treatment of hypovolemic shock (see, *e.g.,* Tullis, JAMA, 237: 355-360, 460-463, (1977)) and Houser et al., Surgery, Gynecology and Obstetrics, 150: 811-816 (1980)) and in conjunction with exchange transfusion in the treatment of neonatal hyperbilirubinemia (see, *e.g*., Finlayson, Seminars in Thrombosis and Hemostasis, 6, 85-120, (1980)). Other albumins are contemplated, such as bovine serum albumin. Use of such non-human albumins could be appropriate, for example, in the context of use of these compositions in non-human mammals, such as the veterinary (including domestic pets and agricultural context). Human serum albumin (HSA) has multiple hydrophobic binding sites (a total of eight for fatty acids, an endogenous ligand of HSA) and binds a diverse set of drugs, especially neutral and negatively charged hydrophobic compounds (Goodman et al., The Pharmacological Basis of Therapeutics, 9th ed, McGraw-Hill New York (1996)). Two high affinity binding sites have been proposed in subdomains IIA and IIIA of HSA, which are highly elongated hydrophobic pockets with charged lysine and arginine residues near the surface which function as attachment points for polar ligand features (see, *e.g.,* Fehske et al., Biochem. Pharmcol., 30, 687-92 (198a), Vorum, Dan. Med. Bull., 46, 379-99 (1999), Kragh-Hansen, Dan. Med. Bull., 1441, 131-40 (1990), Curry et al., Nat. Struct. Biol., 5, 827-35 (1998), Sugio et al., Protein. Eng., 12, 439-46 (1999), He et al., Nature, 358, 209-15 (199b), and Carter et al., Adv. Protein. Chem., 45, 153-203 (1994)). Rapamycin and propofol have been shown to bind HSA (see, *e.g.,* Paal et al., Eur. J. Biochem., 268(7), 2187-91 (200a), Purcell et al., Biochem. Biophys. Acta, 1478(a), 61-8 (2000), Altmayer et al., Arzneimittelforschung, 45, 1053-6 (1995), and Garrido et al., Rev. Esp. Anestestiol. Reanim., 41, 308-12 (1994)). In addition, docetaxel has been shown to bind to human plasma proteins (see, *e.g.,* Urien et al., Invest. New Drugs, 14(b), 147-51 (1996)).

In some embodiments, the composition described herein is substantially free (such as free) of surfactants, such as Cremophor (or polyoxyethylated castor oil, including Cremophor EL^{®} (BASF) or Tween 80). In some embodiments, the composition (such as rapamycin/albumin nanoparticle composition) is substantially free (such as free) of surfactants. A composition is "substantially free of Cremophor" or "substantially free of surfactant" if the amount of Cremophor or surfactant in the composition is not sufficient to cause one or more side effect(s) in an individual when the composition (such as rapamycin/albumin nanoparticle composition) is administered to the individual. In some embodiments, the composition (such as rapamycin/albumin nanoparticle composition) contains less than about any one of 20%, 15%, 10%, 7.5%, 5%, 2.5%, or 1% organic solvent or surfactant. In some embodiments, the albumin is human albumin or human serum albumin. In some embodiments, the albumin is recombinant albumin.

The amount of an albumin in the composition described herein will vary depending on other components in the composition. In some embodiments, the composition comprises an albumin in an amount that is sufficient to stabilize the mTOR inhibitor in an aqueous suspension, for example, in the form of a stable colloidal suspension (such as a stable suspension of nanoparticles). In some embodiments, the albumin is in an amount that reduces the sedimentation rate of the mTOR inhibitor in an aqueous medium. For particle-containing compositions, the amount of the albumin also depends on the size and density of nanoparticles of the mTOR inhibitor.

The mTOR inhibitor is "stabilized" in an aqueous suspension if it remains suspended in an aqueous medium (such as without visible precipitation or sedimentation) for an extended period of time, such as for at least about any of 0.1, 0.2, 0.25, 0.5, 1, 2, 3, 4, 5, 6, 7, 8, 9, 10, 11, 12, 24, 36, 48, 60, or 72 hours. The suspension is generally, but not necessarily, suitable for administration to an individual (such as a human). Stability of the suspension is generally (but not necessarily) evaluated at a storage temperature (such as room temperature (such as 20-25 °C) or refrigerated conditions (such as 4 °C)). For example, a suspension is stable at a storage temperature if it exhibits no flocculation or particle agglomeration visible to the naked eye or when viewed using an optical microscope at 1000 times, at about fifteen minutes after preparation of the suspension. Stability can also be evaluated under accelerated testing conditions, such as at a temperature that is about 40 °C or higher.

The compositions described herein may be a stable aqueous suspension of the mTOR inhibitor, such as a stable aqueous suspension of the mTOR inhibitor at a concentration of any of about 0.1 to about 200 mg/ml, about 0.1 to about 150 mg/ml, about 0.1 to about 100 mg/ml, about 0.1 to about 50 mg/ml, about 0.1 to about 20 mg/ml, about 1 to about 10 mg/ml, about 2 mg/ml to about 8 mg/ml, about 4 to about 6 mg/ml, and about 5 mg/ml. In some embodiments, the concentration of the mTOR inhibitor is at least about any of 0.2 mg/ml, 1.3 mg/ml, 1.5 mg/ml, 2 mg/ml, 3 mg/ml, 4 mg/ml, 5 mg/ml, 6 mg/ml, 7 mg/ml, 8 mg/ml, 9 mg/ml, 10 mg/ml, 15 mg/ml, 20 mg/ml, 25 mg/ml, 30 mg/ml, 40 mg/ml, 50 mg/ml, 100 mg/ml, 150 mg/ml, or 200 mg/ml.

In some embodiments, the albumin is present in an amount that is sufficient to stabilize the mTOR inhibitor in an aqueous suspension at a certain concentration. For example, the concentration of the mTOR inhibitor in the composition is about 0.1 to about 100 mg/ml, including for example about any of 0.1 to about 50 mg/ml, about 0.1 to about 20 mg/ml, about 1 to about 10 mg/ml, about 2 mg/ml to about 8 mg/ml, about 4 to about 6 mg/ml, or about 5 mg/ml. In some embodiments, the concentration of the mTOR inhibitor is at least about any of 1.3 mg/ml, 1.5 mg/ml, 2 mg/ml, 3 mg/ml, 4 mg/ml, 5 mg/ml, 6 mg/ml, 7 mg/ml, 8 mg/ml, 9 mg/ml, 10 mg/ml, 15 mg/ml, 20 mg/ml, 25 mg/ml, 30 mg/ml, 40 mg/ml, and 50 mg/ml. In some embodiments, the albumin is present in an amount that avoids use of surfactants (such as Cremophor), so that the composition is free or substantially free of surfactant (such as Cremophor).

In some embodiments, the composition, in liquid form, comprises from about 0.1% to about 50% (w/v) (*e.g*., about 0.5% (w/v), about 5% (w/v), about 10% (w/v), about 15% (w/v), about 20% (w/v), about 30% (w/v), about 40% (w/v), or about 50% (w/v)) of an albumin. In some embodiments, the composition, in liquid form, comprises about 0.5% to about 5% (w/v) of albumin.

In some embodiments, the weight ratio of the albumin to the mTOR inhibitor in the composition is such that a sufficient amount of the mTOR inhibitor binds to, or is transported by, the cell. While the weight ratio of an albumin to the mTOR inhibitor will have to be optimized for different albumin and mTOR inhibitor combinations, generally the weight ratio of an albumin to the mTOR inhibitor (w/w) is about 0.01:1 to about 100:1, about 0.02:1 to about 50:1, about 0.05:1 to about 20:1, about 0.1:1 to about 20:1, about 1:1 to about 18:1, about 2:1 to about 15:1, about 3:1 to about 12: 1, about 4:1 to about 10:1, about 5:1 to about 9:1, or about 9:1. In some embodiments, the albumin to the mTOR inhibitor weight ratio is about any of 18:1 or less, 15:1 or less, 14:1 or less, 13:1 or less, 12:1 or less, 11:1 or less, 10:1 or less, 9:1 or less, 8:1 or less, 7:1 or less, 6:1 or less, 5:1 or less, 4:1 or less, and 3:1 or less. In some embodiments, the weight ratio of the albumin (such as human albumin or human serum albumin) to the mTOR inhibitor in the composition is any one of the following: about 1:1 to about 18:1, about 1:1 to about 15:1, about 1:1 to about 12:1, about 1:1 to about 10:1, about 1:1 to about 9:1, about 1:1 to about 8:1, about 1:1 to about 7:1, about 1:1 to about 6:1, about 1:1 to about 5:1, about 1:1 to about 4:1, about 1:1 to about 3:1, about 1:1 to about 2:1, about 1:1 to about 1:1.

In some embodiments, the pharmaceutical composition comprises nanoparticles comprising an mTOR inhibitor and an albumin, wherein the weight ratio of the albumin to the mTOR inhibitor in the composition is about 0.01:1 to about 100:1. In some embodiments, the composition comprises nanoparticles comprising the mTOR inhibitor and an albumin, wherein the weight ratio of the albumin to the mTOR inhibitor in the composition is about 18:1 or less (including for example any of about 1:1 to about 18:1, about 2:1 to about 15:1, about 3:1 to about 12:1, about 4:1 to about 10:1, about 5:1 to about 9:1, and about 9:1). In some embodiments, the composition comprises nanoparticles comprising rapamycin, or a derivative thereof, and an albumin, wherein the weight ratio of the albumin to the rapamycin or derivative thereof in the composition is about 18:1 or less (including for example any of about 1:1 to about 18:1, about 2:1 to about 15:1, about 3:1 to about 12:1, about 4:1 to about 10:1, about 5:1 to about 9:1, and about 9:1). In some embodiments, the mTOR inhibitor is coated with albumin.

In some embodiments, the albumin allows the composition to be administered to an individual (such as a human) without significant side effects. In some embodiments, the albumin (such as human serum albumin or human albumin) is in an amount that is effective to reduce one or more side effects of administration of the mTOR inhibitor to a human. The term "reducing one or more side effects" of administration of the mTOR inhibitor refers to reduction, alleviation, elimination, or avoidance of one or more undesirable effects caused by the mTOR inhibitor, as well as side effects caused by delivery vehicles (such as solvents that render the mTOR inhibitor suitable for injection) used to deliver the mTOR inhibitor. Such side effects include, for example, myelosuppression, neurotoxicity, hypersensitivity, inflammation, venous irritation, phlebitis, pain, skin irritation, peripheral neuropathy, neutropenic fever, anaphylactic reaction, venous thrombosis, extravasation, and combinations thereof. These side effects, however, are merely exemplary and other side effects, or combination of side effects, associated with the mTOR inhibitor can be reduced.

In some embodiments, the compositions for use provided herein comprise nanoparticles comprising the mTOR inhibitor and an albumin (such as human albumin or human serum albumin), wherein the nanoparticles have an average diameter of no greater than about 200 nm. In some embodiments, the compositions for use provided herein comprise nanoparticles comprising the mTOR inhibitor and an albumin (such as human albumin or human serum albumin), wherein the nanoparticles have an average diameter of no greater than about 150 nm. In some embodiments, the compositions for use provided herein comprise nanoparticles comprising the mTOR inhibitor and an albumin (such as human albumin or human serum albumin), wherein the nanoparticles have an average diameter of no greater than about 150 nm (for example about 100-120 nm, for example about 100 nm). In some embodiments, the compositions for use provided herein comprise nanoparticles comprising rapamycin and human albumin (such as human serum albumin), wherein the nanoparticles have an average diameter of no greater than about 150 nm (for example about 100-120 nm, for example about 100 nm). In some embodiments, the compositions for use provided herein comprise nanoparticles comprising rapamycin and human albumin (such as human serum albumin), wherein the average or mean diameter of the nanoparticles is about 10 to about 150 nm. In some embodiments, the compositions for use provided herein comprise nanoparticles comprising rapamycin and human albumin (such as human serum albumin), wherein the average or mean diameter of the nanoparticles is about 40 to about 120 nm. In some embodiments, the average or mean diameter of the nanoparticles is about 100-120 nm, for example about 100 nm.

In some embodiments, the compositions for use provided herein comprise nanoparticles comprising the mTOR inhibitor and an albumin (such as human albumin or human serum albumin), wherein the nanoparticles have an average diameter of no greater than about 200 nm, wherein the weight ratio of the albumin and the mTOR inhibitor in the composition is no greater than about 9:1 (for example, from about 3:1 to about 9:1, such as about 9:1 or about 8:1). In some embodiments, the compositions for use provided herein comprise nanoparticles comprising the mTOR inhibitor and an albumin (such as human albumin or human serum albumin), wherein the nanoparticles have an average diameter of no greater than about 150 nm, wherein the weight ratio of the albumin and the mTOR inhibitor in the composition is no greater than about 9:1 (such as about 9:1 or about 8:1). In some embodiments, the compositions for use provided herein comprise nanoparticles comprising the mTOR inhibitor and an albumin (such as human albumin or human serum albumin), wherein the nanoparticles have an average diameter of about 150 nm, wherein the weight ratio of the albumin and the mTOR inhibitor in the composition is no greater than about 9:1 (such as about 9:1 or about 8:1). In some embodiments, the compositions for use provided herein comprise nanoparticles comprising rapamycin and human albumin (such as human serum albumin), wherein the nanoparticles have an average diameter of no greater than about 150 nm (for example about 100-120 nm, for example about 100 nm), wherein the weight ratio of albumin and mTOR inhibitor in the composition is about 9:1 or about 8:1. In some embodiments, the average or mean diameter of the nanoparticles is about 10 nm to about 150 nm. In some embodiments, the average or mean diameter of the nanoparticles is about 40 nm to about 120 nm. In some embodiments, the average or mean diameter of the nanoparticles is about 100-120 nm, for example about 100 nm.

In some embodiments, the compositions for use provided herein comprise nanoparticles comprising the mTOR inhibitor associated (*e.g.*, coated) with an albumin (such as human albumin or human serum albumin). In some embodiments, the compositions for use provided herein comprise nanoparticles comprising the mTOR inhibitor associated (*e.g.*, coated) with an albumin (such as human albumin or human serum albumin), wherein the nanoparticles have an average diameter of no greater than about 200 nm. In some embodiments, the compositions for use provided herein comprise nanoparticles comprising the mTOR inhibitor associated (*e.g*., coated) with an albumin (such as human albumin or human serum albumin), wherein the nanoparticles have an average diameter of no greater than about 150 nm. In some embodiments, the compositions for use provided herein comprise nanoparticles comprising the mTOR inhibitor associated (*e.g.,* coated) with an albumin (such as human albumin or human serum albumin), wherein the nanoparticles have an average diameter of about 10 nm to about 150 nm. In some embodiments, the compositions for use provided herein comprise nanoparticles comprising the mTOR inhibitor associated (*e.g.,* coated) with an albumin (such as human albumin or human serum albumin), wherein the nanoparticles have an average diameter of about 40 nm to about 120 nm. In some embodiments, the compositions for use provided herein comprise nanoparticles comprising rapamycin associated (*e.g.,* coated) with human albumin (such as human serum albumin), wherein the nanoparticles have an average diameter of no greater than about 150 nm (for example about 100-120 nm, for example about 100 nm). In some embodiments, the compositions for use provided herein comprise nanoparticles comprising rapamycin associated (*e.g.,* coated) with human albumin (such as human serum albumin), wherein the nanoparticles have an average diameter of about 10 nm to about 150 nm. In some embodiments, the compositions for use provided herein comprise nanoparticles comprising rapamycin associated (*e.g.,* coated) with human albumin (such as human serum albumin), wherein the nanoparticles have an average diameter of about 40 nm to about 120 nm. In some embodiments, the average or mean diameter of the nanoparticles is about 100-120 nm, for example about 100 nm.

In some embodiments, the compositions for use provided herein comprise nanoparticles comprising the associated (*e.g.,* coated) with an albumin (such as human albumin or human serum albumin), wherein the weight ratio of the albumin and the mTOR inhibitor in the composition is no greater than about 9:1 (for example, from about 3:1 to about 9:1, such as about 9:1 or about 8:1). In some embodiments, the compositions for use provided herein comprise nanoparticles comprising the mTOR inhibitor associated (*e.g.,* coated) with an albumin (such as human albumin or human serum albumin), wherein the nanoparticles have an average diameter of no greater than about 200 nm, wherein the weight ratio of the albumin and the mTOR inhibitor in the composition is no greater than about 9:1 (such as about 9:1 or about 8:1). In some embodiments, the compositions for use described herein comprise nanoparticles comprising the mTOR inhibitor associated (*e.g.,* coated) with an albumin (such as human albumin or human serum albumin), wherein the nanoparticles have an average diameter of no greater than about 150 nm, wherein the weight ratio of the albumin and the mTOR inhibitor in the composition is no greater than about 9:1 (such as about 9:1 or about 8:1). In some embodiments, the compositions for use provided herein comprise nanoparticles comprising the mTOR inhibitor associated (*e.g.,* coated) with an albumin (such as human albumin or human serum albumin), wherein the nanoparticles have an average diameter of about 150 nm, wherein the weight ratio of the albumin and the mTOR inhibitor in the composition is no greater than about 9:1 (such as about 9:1 or about 8:1). In some embodiments, the compositions for use provided herein comprise nanoparticles comprising rapamycin associated (*e.g.,* coated) with human albumin (such as human serum albumin), wherein the nanoparticles have an average diameter of no greater than about 150 nm (for example about 100-120 nm, for example about 100 nm), wherein the weight ratio of albumin and the rapamycin in the composition is about 9:1 or about 8:1. In some embodiments, the average or mean diameter of the nanoparticles is about 10 nm to about 150 nm. In some embodiments, the average or mean diameter of the nanoparticles is about 40 nm to about 120 nm. In some embodiments, the average or mean diameter of the nanoparticles is about 100-120 nm, for example about 100 nm.

In some embodiments, the compositions for use provided herein comprise nanoparticles comprising the mTOR inhibitor stabilized by an albumin (such as human albumin or human serum albumin). In some embodiments, the compositions for use provided herein comprise nanoparticles comprising the mTOR inhibitor stabilized by an albumin (such as human albumin or human serum albumin), wherein the nanoparticles have an average diameter of no greater than about 200 nm. In some embodiments, the compositions for use provided herein comprise nanoparticles comprising the mTOR inhibitor stabilized by an albumin (such as human albumin or human serum albumin), wherein the nanoparticles have an average diameter of no greater than about 150 nm. In some embodiments, the compositions for use provided herein comprise nanoparticles comprising the mTOR inhibitor stabilized by an albumin (such as human albumin or human serum albumin), wherein the nanoparticles have an average diameter of no greater than about 150 nm (for example about 100-120 nm, for example about 100 nm). In some embodiments, the compositions for use provided herein comprise nanoparticles comprising rapamycin stabilized by human albumin (such as human serum albumin), wherein the nanoparticles have an average diameter of no greater than about 150 nm (for example about 100-120 nm, for example about 100 nm). In some embodiments, the average or mean diameter of the nanoparticles is about 10 nm to about 150 nm. In some embodiments, the average or mean diameter of the nanoparticles is about 40 nm to about 120 nm. In some embodiments, the average or mean diameter of the nanoparticles is about 100-120 nm, for example about 100 nm.

In some embodiments, the compositions for use provided herein comprise nanoparticles comprising the mTOR inhibitor stabilized by an albumin (such as human albumin or human serum albumin), wherein the weight ratio of the albumin and the mTOR inhibitor in the composition is no greater than about 9:1 (for example, from about 3:1 to about 9:1, such as about 9:1 or about 8:1). In some embodiments, the compositions for use provided herein comprise nanoparticles comprising the mTOR inhibitor stabilized by an albumin (such as human albumin or human serum albumin), wherein the nanoparticles have an average diameter of no greater than about 200 nm, wherein the weight ratio of the albumin and the mTOR inhibitor in the composition is no greater than about 9:1 (such as about 9:1 or about 8:1). In some embodiments, the compositions for use provided herein comprise nanoparticles comprising the mTOR inhibitor stabilized by an albumin (such as human albumin or human serum albumin), wherein the nanoparticles have an average diameter of no greater than about 150 nm, wherein the weight ratio of the albumin and the mTOR inhibitor in the composition is no greater than about 9:1 (such as about 9:1 or about 8:1). In some embodiments, the compositions for use provided herein comprise nanoparticles comprising the mTOR inhibitor stabilized by an albumin (such as human albumin or human serum albumin), wherein the nanoparticles have an average diameter of about 150 nm, wherein the weight ratio of the albumin and the mTOR inhibitor in the composition is no greater than about 9:1 (such as about 9:1 or about 8:1). In some embodiments, the compositions for use provided herein comprise nanoparticles comprising rapamycin stabilized by human albumin (such as human serum albumin), wherein the nanoparticles have an average diameter of no greater than about 150 nm (for example about 100-120 nm, for example about 100 nm), wherein the weight ratio of albumin and the rapamycin in the composition is about 9:1 or about 8:1. In some embodiments, the average or mean diameter of the nanoparticles is about 10 nm to about 150 nm. In some embodiments, the average or mean diameter of the nanoparticles is about 40 nm to about 120 nm. In some embodiments, the average or mean diameter of the nanoparticles is about 100-120 nm, for example about 100 nm.

In some embodiments, the compositions for use provided herein comprise nanoparticles comprising the mTOR inhibitor and an albumin (such as human albumin or human serum albumin), wherein the composition further comprises a saccharide, wherein the nanoparticles have an average diameter of no greater than about 200 nm. In some embodiments, the compositions for use provided herein comprise nanoparticles comprising the mTOR inhibitor and an albumin (such as human albumin or human serum albumin), wherein the composition further comprises a saccharide, wherein the nanoparticles have an average diameter of no greater than about 150 nm. In some embodiments, the compositions for use provided herein comprise nanoparticles comprising the mTOR inhibitor and an albumin (such as human albumin or human serum albumin), wherein the composition further comprises a saccharide, wherein the nanoparticles have an average diameter of no greater than about 150 nm (for example about 100 nm). In some embodiments, the average or mean diameter of the nanoparticles is about 100-120 nm, for example about 100 nm. In some embodiments, the compositions for use provided herein comprise nanoparticles comprising rapamycin and human albumin (such as human serum albumin), wherein the composition further comprises a saccharide, wherein the nanoparticles have an average diameter of no greater than about 150 nm (for example about 100 nm). In some embodiments, the average or mean diameter of the nanoparticles is about 100-120 nm, for example about 100 nm. In some embodiments, the compositions for use provided herein comprise nanoparticles comprising rapamycin and human albumin (such as human serum albumin), wherein the composition further comprises a saccharide, wherein the average or mean diameter of the nanoparticles is about 10 to about 150 nm. In some embodiments, the compositions for use provided herein comprise nanoparticles comprising rapamycin and human albumin (such as human serum albumin), wherein the average or mean diameter of the nanoparticles is about 40 to about 120 nm. In some embodiments, the average or mean diameter of the nanoparticles is about 100-120 nm, for example about 100 nm.

In some embodiments, the compositions for use provided herein comprise nanoparticles comprising the mTOR inhibitor and an albumin (such as human albumin or human serum albumin), wherein the composition further comprises a saccharide, wherein the nanoparticles have an average diameter of no greater than about 200 nm, wherein the weight ratio of the albumin and the mTOR inhibitor in the composition is no greater than about 9:1 (such as about 9:1 or about 8:1). In some embodiments, the compositions for use provided herein comprise nanoparticles comprising the mTOR inhibitor and an albumin (such as human albumin or human serum albumin), wherein the composition further comprises a saccharide, wherein the nanoparticles have an average diameter of no greater than about 150 nm, wherein the weight ratio of the albumin and the mTOR inhibitor in the composition is no greater than about 9:1 (such as about 9:1 or about 8:1). In some embodiments, the compositions for use provided herein comprise nanoparticles comprising the mTOR inhibitor and an albumin (such as human albumin or human serum albumin), wherein the composition further comprises a saccharide, wherein the nanoparticles have an average diameter of about 150 nm, wherein the weight ratio of the albumin and the mTOR inhibitor in the composition is no greater than about 9:1 (such as about 9:1 or about 8:1). In some embodiments, the compositions for use provided herein comprise nanoparticles comprising rapamycin and human albumin (such as human serum albumin), wherein the composition further comprises a saccharide, wherein the nanoparticles have an average diameter of no greater than about 150 nm (for example about 100 nm), wherein the weight ratio of albumin and mTOR inhibitor in the composition is about 9:1 or about 8:1. In some embodiments, the average or mean diameter of the nanoparticles is about 10 nm to about 150 nm. In some embodiments, the average or mean diameter of the nanoparticles is about 40 nm to about 120 nm. In some embodiments, the average or mean diameter of the nanoparticles is about 100-120 nm, for example about 100 nm.

In some embodiments, the compositions for use provided herein comprise nanoparticles comprising the mTOR inhibitor stabilized by an albumin (such as human albumin or human serum albumin), wherein the composition further comprises a saccharide, wherein the weight ratio of the albumin and the mTOR inhibitor in the composition is no greater than about 9:1 (such as about 9:1 or about 8:1). In some embodiments, the compositions for use provided herein comprise nanoparticles comprising the mTOR inhibitor stabilized by an albumin (such as human albumin or human serum albumin), wherein the composition further comprises a saccharide, wherein the nanoparticles have an average diameter of no greater than about 200 nm, wherein the weight ratio of the albumin and the mTOR inhibitor in the composition is no greater than about 9:1 (such as about 9:1 or about 8:1). In some embodiments, the compositions for use provided herein comprise nanoparticles comprising the mTOR inhibitor stabilized by an albumin (such as human albumin or human serum albumin), wherein the composition further comprises a saccharide, wherein the nanoparticles have an average diameter of no greater than about 150 nm, wherein the weight ratio of the albumin and the mTOR inhibitor in the composition is no greater than about 9:1 (such as about 9:1 or about 8:1). In some embodiments, the compositions for use provided herein comprise nanoparticles comprising the mTOR inhibitor stabilized by an albumin (such as human albumin or human serum albumin), wherein the composition further comprises a saccharide, wherein the nanoparticles have an average diameter of about 150 nm, wherein the weight ratio of the albumin and the mTOR inhibitor in the composition is no greater than about 9:1 (such as about 9:1 or about 8:1). In some embodiments, the compositions for use provided herein comprise nanoparticles comprising rapamycin stabilized by human albumin (such as human serum albumin), wherein the composition further comprises a saccharide, wherein the nanoparticles have an average diameter of no greater than about 150 nm (for example about 100 nm), wherein the weight ratio of albumin and the rapamycin in the composition is about 9:1 or about 8:1. In some embodiments, the average or mean diameter of the nanoparticles is about 10 nm to about 150 nm. In some embodiments, the average or mean diameter of the nanoparticles is about 40 nm to about 120 nm. In some embodiments, the average or mean diameter of the nanoparticles is about 100-120 nm, for example about 100 nm.

In some embodiments, the compositions for use provided herein comprise nanoparticles comprising the mTOR inhibitor associated (*e.g.,* coated) with an albumin (such as human albumin or human serum albumin), wherein the composition further comprises a saccharide. In some embodiments, the compositions for use provided herein comprise nanoparticles comprising the mTOR inhibitor associated (*e.g.,* coated) with an albumin (such as human albumin or human serum albumin), wherein the composition further comprises a saccharide, wherein the nanoparticles have an average diameter of no greater than about 200 nm. In some embodiments, the compositions for use provided herein comprise nanoparticles comprising the mTOR inhibitor associated (*e.g.,* coated) with an albumin (such as human albumin or human serum albumin), wherein the composition further comprises a saccharide, wherein the nanoparticles have an average diameter of no greater than about 150 nm. In some embodiments, the compositions for use provided herein comprise nanoparticles comprising the mTOR inhibitor associated (*e.g.,* coated) with an albumin (such as human albumin or human serum albumin), wherein the composition further comprises a saccharide, wherein the nanoparticles have an average diameter of about 10 nm to about 150 nm. In some embodiments, the compositions for use provided herein comprise nanoparticles comprising the mTOR inhibitor associated (*e.g.,* coated) with an albumin (such as human albumin or human serum albumin), wherein the composition further comprises a saccharide, wherein the nanoparticles have an average diameter of about 40 nm to about 120 nm. In some embodiments, the compositions for use provided herein comprise nanoparticles comprising rapamycin associated (*e.g.,* coated) with human albumin (such as human serum albumin), wherein the composition further comprises a saccharide, wherein the nanoparticles have an average diameter of no greater than about 150 nm (for example about 100 nm). In some embodiments, the compositions for use provided herein comprise nanoparticles comprising rapamycin associated (*e.g.,* coated) with human albumin (such as human serum albumin), wherein the composition further comprises a saccharide, wherein the nanoparticles have an average diameter of about 10 nm to about 150 nm. In some embodiments, the compositions for use provided herein comprise nanoparticles comprising rapamycin associated *(e.g.,* coated) with human albumin (such as human serum albumin), wherein the composition further comprises a saccharide, wherein the nanoparticles have an average diameter of about 40 nm to about 120 nm. In some embodiments, the average or mean diameter of the nanoparticles is about 100-120 nm, for example about 100 nm.

In some embodiments, the compositions for use provided herein comprise nanoparticles comprising the mTOR inhibitor associated (e.g., coated) with an albumin (such as human albumin or human serum albumin), wherein the composition further comprises a saccharide, wherein the weight ratio of the albumin and the mTOR inhibitor in the composition is no greater than about 9:1 (such as about 9:1 or about 8:1). In some embodiments, the compositions for use provided herein comprise nanoparticles comprising the mTOR inhibitor associated (*e.g.,* coated) with an albumin (such as human albumin or human serum albumin), wherein the composition further comprises a saccharide, wherein the nanoparticles have an average diameter of no greater than about 200 nm, wherein the weight ratio of the albumin and the mTOR inhibitor in the composition is no greater than about 9:1 (such as about 9:1 or about 8:1). In some embodiments, the compositions for use provided herein comprise nanoparticles comprising the mTOR inhibitor associated (*e.g.,* coated) with an albumin (such as human albumin or human serum albumin), wherein the composition further comprises a saccharide, wherein the nanoparticles have an average diameter of no greater than about 150 nm, wherein the weight ratio of the albumin and the mTOR inhibitor in the composition is no greater than about 9:1 (such as about 9:1 or about 8:1). In some embodiments, the compositions for use provided herein comprise nanoparticles comprising the mTOR inhibitor associated (*e.g.,* coated) with an albumin (such as human albumin or human serum albumin), wherein the composition further comprises a saccharide, wherein the nanoparticles have an average diameter of about 150 nm, wherein the weight ratio of the albumin and the mTOR inhibitor in the composition is no greater than about 9:1 (such as about 9:1 or about 8:1). In some embodiments, the compositions for use provided herein comprise nanoparticles comprising rapamycin associated (*e.g.,* coated) with human albumin (such as human serum albumin), wherein the composition further comprises a saccharide, wherein the nanoparticles have an average diameter of no greater than about 150 nm (for example about 100 nm), wherein the weight ratio of albumin and the rapamycin in the composition is about 9:1 or about 8:1. In some embodiments, the average or mean diameter of the nanoparticles is about 10 nm to about 150 nm. In some embodiments, the average or mean diameter of the nanoparticles is about 40 nm to about 120 nm. In some embodiments, the average or mean diameter of the nanoparticles is about 100-120 nm, for example about 100 nm.

In some embodiments, the compositions for use provided herein comprise nanoparticles comprising the mTOR inhibitor stabilized by an albumin (such as human albumin or human serum albumin), wherein the composition further comprises a saccharide. In some embodiments, the compositions for use provided herein comprise nanoparticles comprising the mTOR inhibitor stabilized by an albumin (such as human albumin or human serum albumin), wherein the composition further comprises a saccharide, wherein the nanoparticles have an average diameter of no greater than about 200 nm. In some embodiments, the compositions for use provided herein comprise nanoparticles comprising the mTOR inhibitor stabilized by an albumin (such as human albumin or human serum albumin), wherein the composition further comprises a saccharide, wherein the nanoparticles have an average diameter of no greater than about 150 nm. In some embodiments, the compositions for use provided herein comprise nanoparticles comprising the mTOR inhibitor stabilized by an albumin (such as human albumin or human serum albumin), wherein the composition further comprises a saccharide, wherein the nanoparticles have an average diameter of no greater than about 150 nm (for example about 100 nm). In some embodiments, the compositions for use provided herein comprise nanoparticles comprising rapamycin stabilized by human albumin (such as human serum albumin), wherein the composition further comprises a saccharide, wherein the nanoparticles have an average diameter of no greater than about 150 nm (for example about 100 nm). In some embodiments, the average or mean diameter of the nanoparticles is about 10 nm to about 150 nm. In some embodiments, the average or mean diameter of the nanoparticles is about 40 nm to about 120 nm. In some embodiments, the average or mean diameter of the nanoparticles is about 100-120 nm, for example about 100 nm.

In some embodiments, the composition comprises nab-rapamycin. In some embodiments, the composition is *nab*-rapamycin. *Nab*-rapamycin is a formulation of rapamycin stabilized by human albumin USP, which can be dispersed in directly injectable physiological solution. The weight ratio of human albumin and rapamycin is from about 3:1 to about 9:1, for example, about 8:1 to about 9:1. When dispersed in a suitable aqueous medium such as 0.9% sodium chloride injection or 5% dextrose injection, *nab*-rapamycin forms a stable colloidal suspension of rapamycin. The mean particle size of the nanoparticles in the colloidal suspension is about 100 nanometers. Since HSA is freely soluble in water, *nab*-rapamycin can be reconstituted in a wide range of concentrations ranging from dilute (0.1 mg/ml rapamycin or a derivative thereof) to concentrated (e.g., 50 mg/ml rapamycin or a derivative thereof), including for example about 2 mg/ml to about 8 mg/ml, or about 5 mg/ml.

Methods of making nanoparticle compositions are known in the art. For example, nanoparticles containing the mTOR inhibitor and an albumin (such as human serum albumin or human albumin) can be prepared under conditions of high shear forces (*e.g.,* sonication, high pressure homogenization, or the like). These methods are disclosed in, for example, U. S. Pat. Nos.5,916,596; 6,506,405; 6,749,868, 6,537,579, 7,820,788, and 8,911,786, and also in U. S. Pat. Pub. Nos. 2007/0082838, 2006/0263434 and PCT Application WO08/137148.

Briefly, the mTOR inhibitor is dissolved in an organic solvent, and the solution can be added to an albumin solution. The mixture is subjected to high pressure homogenization. The organic solvent can then be removed by evaporation. The dispersion obtained can be further lyophilized. Suitable organic solvent include, for example, ketones, esters, ethers, chlorinated solvents, and other solvents known in the art. For example, the organic solvent can be methylene chloride or chloroform/ethanol (for example with a ratio of 1:9, 1:8, 1:7, 1:6, 1:5, 1:4, 1:3, 1:2, 1:1, 2:1, 3:1, 4:1, 5:1, 6:1, 7:1, 8:1, or 9:1).

In some embodiments, the composition is a dry (such as lyophilized) composition that can be reconstituted, resuspended, or rehydrated to form generally a stable aqueous suspension of the nanoparticles comprising the mTOR inhibitor and an albumin. In some embodiments, the composition is a liquid (such as aqueous) composition obtained by reconstituting or resuspending a dry composition. In some embodiments, the composition is an intermediate liquid (such as aqueous) composition that can be dried (such as lyophilized).

### A. mTOR inhibitor

"mTOR inhibitor" used herein refers to an inhibitor of mTOR. mTOR is a serine/threonine-specific protein kinase downstream of the phosphatidylinositol 3-kinase (PI3K)/Akt (protein kinase B) pathway, and a key regulator of cell survival, proliferation, stress, and metabolism. mTOR pathway dysregulation has been found in many human carcinomas, and mTOR inhibition produced substantial inhibitory effects on tumor progression.

The mammalian target of the mTOR inhibitor (mTOR) (also known as mechanistic target of the mTOR inhibitor or FK506 binding protein 12-the mTOR inhibitor associated protein 1 (FRAP1)) is an atypical serine/threonine protein kinase that is present in two distinct complexes, mTOR Complex 1 (mTORC1) and mTOR Complex 2 (mTORC2). mTORC1 is composed of mTOR, regulatory-associated protein of mTOR (Raptor), mammalian lethal with SEC13 protein 8 (MLST8), PRAS40 and DEPTOR (Kim et al. (2002). Cell 110: 163-75; Fang et al. (2001). Science 294 (5548): 1942-5). mTORC1 integrates four major signal inputs: nutrients (such as amino acids and phosphatidic acid), growth factors (insulin), energy and stress (such as hypoxia and DNA damage). Amino acid availability is signaled to mTORC1 via a pathway involving the Rag and Ragulator (LAMTOR1-3) Growth factors and hormones (*e.g.,* insulin) signal to mTORC1 via Akt, which inactivates TSC2 to prevent inhibition of mTORC1. Alternatively, low ATP levels lead to the AMPK-dependent activation of TSC2 and phosphorylation of raptor to reduce mTORC1 signaling proteins.

Active mTORC1 has a number of downstream biological effects including translation of mRNA via the phosphorylation of downstream targets (4E-BP1 and p70 S6 Kinase), suppression of autophagy (Atg13, ULK1), ribosome biogenesis, and activation of transcription leading to mitochondrial metabolism or adipogenesis. Accordingly, mTORC1 activity promotes either cellular growth when conditions are favorable or catabolic processes during stress or when conditions are unfavorable.

mTORC2 is composed of mTOR, rapamycin-insensitive companion of mTOR (RICTOR), GβL, and mammalian stress-activated protein kinase interacting protein 1 (mSIN1). In contrast to mTORC1, for which many upstream signals and cellular functions have been defined (see above), relatively little is known about mTORC2 biology. mTORC2 regulates cytoskeletal organization through its stimulation of F-actin stress fibers, paxillin, RhoA, Rac1, Cdc42, and protein kinase C α (PKCα). It had been observed that knocking down mTORC2 components affects actin polymerization and perturbs cell morphology (Jacinto et al. (2004). Nat. Cell Biol. 6, 1122-1128; Sarbassov et al. (2004). Curr. Biol. 14, 1296-1302). This suggests that mTORC2 controls the actin cytoskeleton by promoting protein kinase Cα (PKCα) phosphorylation, phosphorylation of paxillin and its relocalization to focal adhesions, and the GTP loading of RhoA and Rac1. The molecular mechanism by which mTORC2 regulates these processes has not been determined.

In some embodiments, the mTOR inhibitor is an inhibitor of mTORC1. In some embodiments, the mTOR inhibitor is an inhibitor of mTORC2. In some embodiments, the mTOR inhibitor is an inhibitor of both mTORC1 and mTORC2.

In some examples, the mTOR inhibitor is a limus drug. Examples of limus drugs include, but are not limited to, temrapamycin (CCI-779), everolimus (RAD001), ridaforolimus (AP-23573), deforolimus ( MK-8669), zotarolimus (ABT-578), pimecrolimus, and tacrolimus (FK-506). In some embodiments, the limus drug is selected from the group consisting of temrapamycin (CCI-779), everolimus (RAD001), ridaforolimus (AP-23573), deforolimus (MK-8669), zotarolimus (ABT-578), pimecrolimus, and tacrolimus (FK-506). In some examples, the mTOR inhibitor is an mTOR kinase inhibitor, such as CC-115 or CC-223.

According to the present invention, the mTOR inhibitor is rapamycin. Rapamycin is macrolide antibiotic that complexes with FKBP-12 and inhibits the mTOR pathway by binding mTORC1.

In some examples, the mTOR inhibitor is selected from the group consisting of BEZ235 (NVP-BEZ235), everolimus (also known as RAD001, Zortress, Certican, and Afinitor), AZD8055,temrapamycin (also known as CCI-779 and Torisel), CC-115, CC-223, PI-103, Ku-0063794, INK 128, AZD2014, NVP-BGT226, PF-04691502, CH5132799, GDC-0980 (RG7422), Torin 1, WAY-600, WYE-125132, WYE-687, GSK2126458, PF-05212384 (PKI-587), PP-121, OSI-027, Palomid 529, PP242, XL765, GSK1059615, WYE-354, and ridaforolimus (also known as deforolimus).

BEZ235 (NVP-BEZ235) is an imidazoquilonine derivative that is an mTORC1 catalytic inhibitor (Roper J, et al. PLoS One, 2011, 6(9), e25132). Everolimus is the 40-O-(2-hydroxyethyl) derivative of rapamycin and binds the cyclophilin FKBP-12, and this complex also mTORC1. AZD8055 is a small molecule that inhibits the phosphorylation of mTORC1 (p70S6K and 4E-BP1). Temrapamycin is a small molecule that forms a complex with the FK506-binding protein and prohibits the activation of mTOR when it resides in the mTORC1complex. PI-103 is a small molecule that inhibits the activation of the rapamycin-sensitive (mTORC1) complex (Knight et al. (2006) Cell. 125: 733-47). KU-0063794 is a small molecule that inhibits the phosphorylation of mTORC1 at Ser2448 in a dose-dependent and time-dependent manner. INK 128, AZD2014, NVP-BGT226, CH5132799, WYE-687, and are each small molecule inhibitors of mTORC1. PF-04691502 inhibits mTORC1 activity. GDC-0980 is an orally bioavailable small molecule that inhibits Class I PI3 Kinase and TORC1. Torin 1 is a potent small molecule inhibitor of mTOR. WAY-600 is a potent, ATP-competitive and selective inhibitor of mTOR. WYE-125132 is an ATP-competitive small molecule inhibitor of mTORC1. GSK2126458 is an inhibitor of mTORC1. PKI-587 is a highly potent dual inhibitor of PI3Kα, PI3Kγ and mTOR. PP-121 is a multi-target inhibitor of PDGFR, Hck, mTOR, VEGFR2, Src and Abl. OSI-027 is a selective and potent dual inhibitor of mTORC1 and mTORC2 with IC50 of 22 nM and 65 nM, respectively. Palomid 529 is a small molecule inhibitor of mTORC1 that lacks affinity for ABCB1/ABCG2 and has good brain penetration (Lin et al. (2013) Int J Cancer DOI: 10.1002/ijc. 28126 (e-published ahead of print). PP242 is a selective mTOR inhibitor. XL 765 is a dual inhibitor of mTOR/PI3k for mTOR, p110α, p110β, p110γ and p110δ. GSK1059615 is a novel and dual inhibitor of PI3Kα, PI3Kβ, PI3Kδ, PI3Kγ and mTOR. WYE-354 inhibits mTORC1 in HEK293 cells (0.2 µM-5 µM) and in HUVEC cells (10 nM-1µM). WYE-354 is a potent, specific and ATP-competitive inhibitor of mTOR. Deforolimus (Ridaforolimus, AP23573, MK-8669) is a selective mTOR inhibitor.

### B. Carrier Protein

The composition for use of the invention comprises nanoparticles comprising rapamycin and a carrier protein. The term "proteins" refers to polypeptides or polymers of amino acids of any length (including full length or fragments), which may be linear or branched, comprise modified amino acids, and/or be interrupted by non-amino acids. The term also encompasses an amino acid polymer that has been modified naturally or by intervention; for example, disulfide bond formation, glycosylation, lipidation, acetylation, phosphorylation, or any other manipulation or modification. Also included within this term are, for example, polypeptides containing one or more analogs of an amino acid (including, for example, unnatural amino acids, etc.), as well as other modifications known in the art. The proteins described herein may be naturally occurring, *i.e.,* obtained or derived from a natural source (such as blood), or synthesized (such as chemically synthesized or by synthesized by recombinant DNA techniques). Examples of suitable carrier proteins include proteins normally found in blood or plasma, which include, but are not limited to, albumin, immunoglobulin including IgA, lipoproteins, apolipoprotein B, alpha-acid glycoprotein, beta-2-macroglobulin, thyroglobulin, transferin, fibronectin, factor VII, factor VIII, factor IX, factor X, and the like. In some embodiments, the carrier protein is non-blood protein, such as casein, α-lactalbumin, and β-lactoglobulin. The carrier proteins may either be natural in origin or synthetically prepared.

In some embodiments, the carrier protein is an albumin. In some embodiments, the albumin is serum albumin. In some embodiments, the albumin is human serum albumin.

### C. Other Components in the mTOR inhibitor Nanoparticle Composition

The nanoparticles described herein can be present in a composition that includes other agents, excipients, or stabilizers. For example, to increase stability by increasing the negative zeta potential of nanoparticles, certain negatively charged components may be added. Such negatively charged components include, but are not limited to bile salts of bile acids consisting of glycocholic acid, cholic acid, chenodeoxycholic acid, taurocholic acid, glycochenodeoxycholic acid, taurochenodeoxycholic acid, litocholic acid, ursodeoxycholic acid, dehydrocholic acid and others; phospholipids including lecithin (egg yolk) based phospholipids which include the following phosphatidylcholines: palmitoyloleoylphosphatidylcholine, palmitoyllinoleoylphosphatidylcholine, stearoyllinoleoylphosphatidylcholine stearoyloleoylphosphatidylcholine, stearoylarachidoylphosphatidylcholine, and dipalmitoylphosphatidylcholine. Other phospholipids including L-α-dimyristoylphosphatidylcholine (DMPC), dioleoylphosphatidylcholine (DOPC), distearyolphosphatidylcholine (DSPC), hydrogenated soy phosphatidylcholine (HSPC), and other related compounds. Negatively charged surfactants or emulsifiers are also suitable as additives, *e.g*., sodium cholesteryl sulfate and the like.

In some embodiments, the composition is suitable for administration to a human. In some embodiments, the composition is suitable for administration to a mammal such as, in the veterinary context, domestic pets and agricultural animals. There are a wide variety of suitable formulations of the composition (such as rapamycin/albumin nanoparticle composition) (see, *e.g.,* U. S. Pat. Nos.5,916,596 and 6,096,331). The following formulations and methods are merely exemplary and are in no way limiting. Formulations suitable for oral administration can consist of (a) liquid solutions, such as an effective amount of the compound dissolved in diluents, such as water, saline, or orange juice, (b) capsules, sachets or tablets, each containing a predetermined amount of the active ingredient, as solids or granules, (c) suspensions in an appropriate liquid, and (d) suitable emulsions. Tablet forms can include one or more of lactose, mannitol, corn starch, potato starch, microcrystalline cellulose, acacia, gelatin, colloidal silicon dioxide, croscarmellose sodium, talc, magnesium stearate, stearic acid, and other excipients, colorants, diluents, buffering agents, moistening agents, preservatives, flavoring agents, and pharmacologically compatible excipients. Lozenge forms can comprise the active ingredient in a flavor, usually sucrose and acacia or tragacanth, as well as pastilles comprising the active ingredient in an inert base, such as gelatin and glycerin, or sucrose and acacia, emulsions, gels, and the like containing, in addition to the active ingredient, such excipients as are known in the art.

Examples of suitable carriers, excipients, and diluents include, but are not limited to, lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, saline solution, syrup, methylcellulose, methyl- and propylhydroxybenzoates, talc, magnesium stearate, and mineral oil. In some embodiments, the nanoparticle composition with a carrier as discussed herein is present in a dry formulation (such as lyophilized composition). The formulations can additionally include lubricating agents, wetting agents, emulsifying and suspending agents, preserving agents, sweetening agents or flavoring agents.

Formulations suitable for parenteral administration include aqueous and non-aqueous, isotonic sterile injection solutions, which can contain anti-oxidants, buffers, bacteriostats, and solutes that render the formulation compatible with the blood of the intended recipient, and aqueous and non-aqueous sterile suspensions that can include suspending agents, solubilizers, thickening agents, stabilizers, and preservatives. The formulations can be presented in unit-dose or multi-dose sealed containers, such as ampules and vials, and can be stored in a freeze-dried (lyophilized) condition requiring only the addition of the sterile liquid excipient, for example, water, for injections, immediately prior to use. Extemporaneous injection solutions and suspensions can be prepared from sterile powders, granules, and tablets of the kind previously described. Injectable formulations are preferred.

In some embodiments, the composition is formulated to have a pH range of about 4.5 to about 9.0, including for example pH ranges of about any of 5.0 to about 8.0, about 6.5 to about 7.5, and about 6.5 to about 7.0. In some embodiments, the pH of the composition is formulated to no less than about 6, including for example no less than about any of 6.5, 7, or 8 (such as about 8). The composition can also be made to be isotonic with blood by the addition of a suitable tonicity modifier, such as glycerol.

### Pulmonary hypertension

In some embodiments, the pulmonary hypertension is pulmonary arterial hypertension. In some embodiments, the pulmonary hypertension is selected from the group consisting of idiopathic pulmonary arterial hypertension (IPAH), heritable pulmonary arterial hypertension (HPAH), drug and toxin induced PAH, PAH associated with connective tissue disease, and PAH associated with congenital heart defects.

In some embodiments, the pulmonary hypertension is severe pulmonary arterial hypertension. In some embodiments, the pulmonary hypertension is World Health Organization [WHO] Function Class II, III, or IV pulmonary arterial hypertension. In some embodiments, the pulmonary hypertension is WHO Function Class II pulmonary arterial hypertension. In some embodiments, the pulmonary hypertension is WHO Function Class III pulmonary arterial hypertension. In some embodiments, the pulmonary hypertension is WHO Function Class IV pulmonary arterial hypertension.

### Individual

In some embodiments, the individual is human. In some embodiments, the individual is an adult.

In some embodiments, the individual has had at least one prior therapy for pulmonary hypertension. In some embodiments, the individual has had at least two prior therapy for pulmonary hypertension. In some embodiments, the individual has had one or two prior therapy for pulmonary hypertension.

In some embodiments, the prior therapy comprises a standard or commonly used pulmonary hypertension therapy. In some embodiments, the prior therapy comprises a vasodilator. In some embodiments, the prior therapy regulates vasodilation and/or vasoconstriction. In some embodiments, the prior therapy is selected from the group consisting of a prostacyclin analogue, an endothelin-1 receptor antagonist, a phosphodiesterase 5 (PDE-5) inhibitor and a soluble guanylate cyclase (sGC) stimulator. In some embodiments, the prior therapy is selected from the group consisting of epoprostenol, iloprost, treprostinil, bosentan, macitentan, ambrisentan, sildenafil, tadalafil, and riociguat. In some embodiments, the second therapy comprises a tyrosine kinase inhibitor (*e.g.,* imatinib).

In some embodiments, the individual has progressed on the prior therapy. In some embodiments, the individual did not respond to the prior therapy. In some embodiments, the individual relapsed after the prior therapy.

In some embodiments, the individual is resistant, refractory, or recurrent to at least one, two, or three prior therapies.

In some embodiments, the individual has a high level of fibrosis in the lung. In some embodiments, the individual has a high level of angiogenesis in the lung. In some embodiments, the individual has increased fibrosis in the lung. In some embodiments, the individual has increased angiogenesis in the lung.

In some embodiments, the individual has a baseline (measured shortly prior to initiation of the administration) 6MWD of about 150-450 meters. In some embodiments, the individual has a baseline forced vital capacity ratio of no less than 0.60. In some embodiments, the individual has a forced expiratory volume in one second (FEV1) of no less than about 55% of a reference level (such as a predicted normal level). In some embodiments, the individual has a mean PAP of no less than about 25 mmHg. In some embodiments, the individual has a PCWP or left ventricular end diastolic pressure (LVEDP) of no more than about15 mm. In some embodiments, the individual has a PVR of more than about 5 mmHg/L/min (Woods unit).

### Combination therapy

In some embodiments, the method further comprises administering a second therapy. In some embodiments, the second therapy is a standard or commonly used pulmonary hypertension therapy. In some embodiments, the second therapy comprises a vasodilator. In some embodiments, the second therapy regulates vasodilation and/or vasoconstriction. In some embodiments, the second therapy is selected from the group consisting of a prostacyclin analogue, an endothelin-1 receptor antagonist, a phosphodiesterase 5 (PDE-5) inhibitor and a soluble guanylate cyclase (sGC) stimulator. In some embodiments, the second therapy is selected from the group consisting of epoprostenol, iloprost, treprostinil, bosentan, macitentan, ambrisentan, sildenafil, tadalafil, and riociguat. In some embodiments, the second therapy comprises a tyrosine kinase inhibitor (*e.g.,* imatinib).

In some embodiments, the nanoparticle composition is administered simultaneously with the second therapy. In some embodiments, the nanoparticle composition is administered concurrently with the second therapy. In some embodiments, the nanoparticle composition is administered sequentially with the second therapy.

### Kits

Further disclosed herein are kits comprising the compositions, formulations, unit dosages, and articles of manufacture described herein for use in the methods of treatment, methods of administration, and dosage regimes described herein. The present invention provides a kit for use in a method of treating pulmonary hypertension in an individual, wherein the kit comprises: (a) a composition comprising nanoparticles comprising rapamycin and a carrier protein; and (b) instructions for said use of said composition, wherein the method comprises subcutaneously administering to the individual a dose of from about 1 mg/m² to about 10 mg/m² of rapamycin in said composition. In some embodiments, the pulmonary hypertension is IPAH, FPAH, or APAH. In some embodiments, the kit comprises i) a composition comprising nanoparticles comprising a rapamycin and a carrier protein (such as albumin) and ii) instructions for administering the nanoparticles and the chemotherapeutic agents simultaneously and/or sequentially, for treatment of pulmonary hypertension. In some embodiments, the pulmonary hypertension is pulmonary arterial hypertension. In some embodiments, the pulmonary hypertension is severe pulmonary arterial hypertension. In some embodiments, the pulmonary arterial hypertension is idiopathic pulmonary arterial hypertension. In various embodiments, the amount of the mTOR inhibitor in the kit is included in any of the following ranges: about 0.1 mg to about 1 mg, about 1 to about 3 mg, about 3 to about 6 mg, about 6 to about 9 mg, about 9 to about 12 mg, about 12 to about 15 mg, or about 15 to about 18 mg. In some embodiments, the amount of the mTOR inhibitor in the kit is in the range of about 0.1 mg to about 10 mg, such as about 1 mg to about 5 mg or about 5 mg to about 10 mg.

Instructions supplied in the kits of the invention are typically written instructions on a label or package insert (*e.g.,* a paper sheet included in the kit), but machine-readable instructions (*e.g.,* instructions carried on a magnetic or optical storage disk) are also acceptable. The instructions relating to the use of the nanoparticle compositions generally include information as to dosage, dosing schedule, and route of administration for the intended treatment. The kit may further comprise a description of selecting an individual suitable or treatment.

In some embodiments, the kit of the invention comprises the packaging described above. In other embodiments, the kit of the invention comprises the packaging described above and a second packaging comprising a buffer. It may further include other materials desirable from a commercial and user standpoint, including other buffers, diluents, filters, needles, syringes, and package inserts with instructions for performing any methods described herein.

For combination therapies of the invention, the kit may contain instructions for administering the first and second therapies simultaneously and/or sequentially for the effective treatment of pulmonary hypertension. The first and second therapies can be present in separate containers or in a single container. It is understood that the kit may comprise one distinct composition or two or more compositions wherein one composition comprises a first therapy and one composition comprises a second therapy.

Kits may also be provided that contain sufficient dosages of the mTOR inhibitor as disclosed herein to provide effective treatment for an individual for an extended period, such as any of a week, 2 weeks, 3 weeks, 4 weeks, 6 weeks, 8 weeks, 3 months, 4 months, 5 months, 6 months, 7 months, 8 months, 9 months or more. Kits may also include multiple unit doses of the compositions, pharmaceutical compositions, and formulations for use of the invention and instructions for use and packaged in quantities sufficient for storage and use in pharmacies, for example, hospital pharmacies and compounding pharmacies. In some embodiments, the kit comprises a dry (*e.g.,* lyophilized) composition that can be reconstituted, resuspended, or rehydrated to form generally a stable aqueous suspension of nanoparticles comprising the mTOR inhibitor and albumin (*e.g.,* serum albumin, *e.g.,* human serum albumin). In some embodiments, the kit comprises a dry (*e.g.,* lyophilized) composition that can be reconstituted, resuspended, or rehydrated to form generally a stable aqueous suspension of nanoparticles comprising the mTOR inhibitor and albumin.

The kits are in suitable packaging. Suitable packaging include, but is not limited to, vials, bottles, jars, flexible packaging (*e.g.,* seled Mylar or plastic bags), and the like. Kits may optionally provide additional components such as buffers and interpretative information.

Kits of the invention may include one or more containers comprising the nanoparticle composition (such as rapamycin/albumin nanoparticle composition) suitable for sub-cutaneous administration. According to the invention, the kit further comprises instructions for use in accordance with the invention. The kit may further comprise a description of selection of individuals suitable for treatment. Instructions supplied in the kits of the invention are typically written instructions on a label or package insert (e.g., a paper sheet included in the kit), but machine-readable instructions (e.g., instructions carried on a magnetic or optical storage disk) are also acceptable.

### Methods of Making the Compositions (disclosed herein as reference)

Methods of making compositions containing carrier proteins and poorly water soluble pharmaceutical agents are known in the art. For example, nanoparticles containing poorly watersoluble pharmaceutical agents and carrier proteins (*e.g.,* albumin) can be prepared under conditions of high shear forces (*e.g.,* sonication, high pressure homogenization, or the like). These methods are disclosed in, for example, U.S. Pat. Nos. 5,916,596; 6,506,405; and 6,537,579 and also in U.S. Pat. Pub. No. 2005/0004002A1.

Briefly, an mTOR inhibitor *(e.g.,* rapamycin or a derivative thereof, *e.g.,* rapamycin) is dissolved in an organic solvent. Suitable organic solvents include, for example, ketones, esters, ethers, chlorinated solvents, and other solvents known in the art. For example, the organic solvent can be methylene chloride, chloroform/ethanol, or chloroform/t-butanol (for example with a ratio of about any of 1:9, 1:8, 1:7, 1:6, 1:5, 1:4, 1:3, 1:2, 1:1, 2:1, 3:1, 4:1, 5:1, 6:1, 7:1, 8:1, or 9:1 or with a ratio of about any of 3:7, 5:7, 4:6, 5:5, 6:5, 8:5, 9:5, 9.5:5, 5:3, 7:3, 6:4, or 9.5:0.5). The solution is added to a carrier protein (e.g., human serum albumin). The mixture is subjected to high pressure homogenization (e.g., using an Avestin, APV Gaulin, Microfluidizer^{™} such as a Microfluidizer^{™} Processor M-110EH from Microfluidics, Stansted, or Ultra Turrax homogenizer). The emulsion may be cycled through the high pressure homogenizer for between about 2 to about 100 cycles, such as about 5 to about 50 cycles or about 8 to about 20 cycles (*e.g.,* about any of 8, 10, 12, 14, 16, 18 or 20 cycles). The organic solvent can then be removed by evaporation utilizing suitable equipment known for this purpose, including, but not limited to, rotary evaporators, falling film evaporators, wiped film evaporators, spray driers, and the like that can be operated in batch mode or in continuous operation. The solvent may be removed at reduced pressure (such as at about any of 25 mm Hg, 30 mm Hg, 40 mm Hg, 50 mm Hg, 100 mm Hg, 200 mm Hg, or 300 mm Hg). The amount of time used to remove the solvent under reduced pressure may be adjusted based on the volume of the formulation. For example, for a formulation produced on a 300 mL scale, the solvent can be removed at about 1 to about 300 mm Hg *(e.g.,* about any of 5-100 mm Hg, 10-50 mm Hg, 20-40 mm Hg, or 25 mm Hg) for about 5 to about 60 minutes (*e.g.,* about any of 7, 8, 9, 10, 11, 12, 13, 14, 15 16, 18, 20, 25, or 30 minutes).

If desired, human albumin solution may be added to the dispersion to adjust the human serum albumin to rapamycin ratio or to adjust the concentration of rapamycin in the dispersion. For example, human serum albumin solution (*e.g.,* 25 % w/v) can be added to adjust the human serum albumin to rapamycin ratio to about any of 18:1, 15,:1 14:1, 13:1, 12:1, 11:1, 10:1, 9:1, 8:1, 7.5:1, 7:1, 6:1, 5:1, 4:1, or 3:1. For example, human serum albumin solution (*e.g.,* 25 % w/v) can be added to adjust the human serum albumin to an mTOR inhibitor (e.g., rapamycin) ratio to about any of 18:1, 15,:1 14:1, 13:1, 12:1, 11:1, 10:1, 9:1, 8:1, 7.5:1, 7:1, 6:1, 5:1, 4:1, or 3:1. For example, human serum albumin solution (*e.g.,* 25 % w/v) or another solution is added to adjust the concentration of rapamycin in the dispersion to about any of 0.5 mg/ml, 1.3 mg/ml, 1.5 mg/ml, 2 mg/ml, 3 mg/ml, 4 mg/ml, 5 mg/ml, 6 mg/ml, 7 mg/ml, 8 mg/ml, 9 mg/ml, 10 mg/ml, 15 mg/ml, 20 mg/ml, 25 mg/ml, 30 mg/ml, 40 mg/ml, or 50 mg/ml. The dispersion may be serially filtered through multiple filters, such as a combination of 1.2 µm and 0.8/0.2 µm filters; the combination of 1.2 µm, 0.8 µm, 0.45 µm, and 0.22 µm filters; or the combination of any other filters known in the art. The dispersion obtained can be further lyophilized. The nanoparticle compositions may be made using a batch process or a continuous process (*e.g.,* the production of a composition on a large scale).

Unless defined otherwise, the meanings of all technical and scientific terms used herein are those commonly understood by one of skill in the art to which this invention belongs. One of skill in the art will also appreciate that any methods and materials similar or equivalent to those described herein can also be used to practice or test the invention.

The following Examples are provided to illustrate, but not limit, the invention.

### EXAMPLES

### Reference Example 1: Clinical Use of Nab-rapamycin for treatment of Severe Pulmonary Arterial Hypertension

This study was a dose-finding prospective phase 1, single arm, open-label, multi-institutional study to determine the maximum tolerated dose (MTD), dose limiting toxicity (DLT), safety, and preliminary efficacy of 16 weeks of IV ABI-009 (nab-rapamycin) treatment in patients with severe PAH who are WHO Functional Class III despite best available background therapy.

### Treatment and results

### 1 Part A.

ABI-009 was IV administered weekly to four subjects for up to 16 weeks. Two dose levels of ABI-009 were used (5 mg/m² and 10 mg/m²).

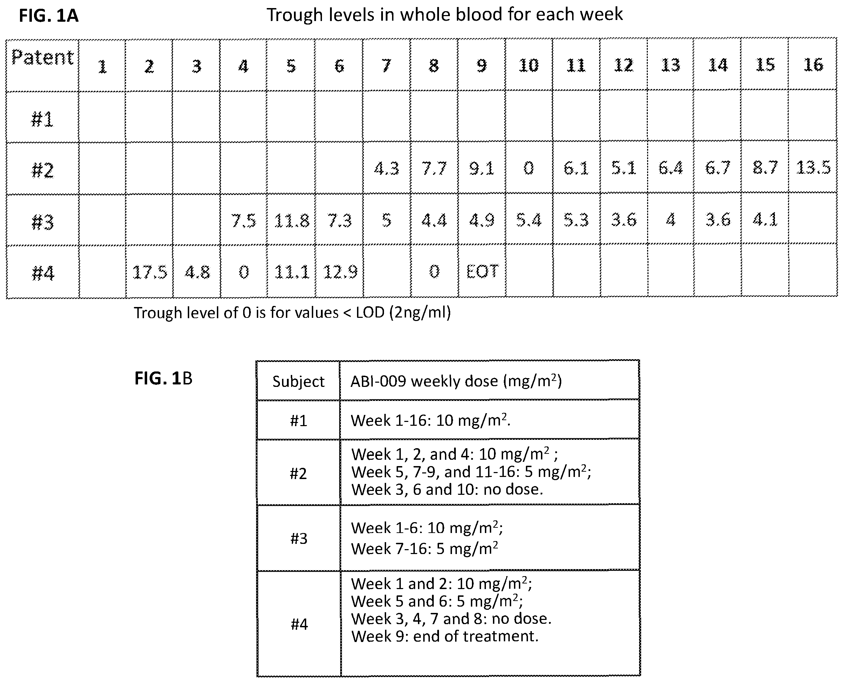

All four subjects had Functional class III according to the WHO set forth at the Dana Point Classification 2008 Meeting prior to the treatment and failed at least two PAH therapies. Specific dosages of ABI-009 for each subject is shown in FIG. 1B. All four subjects started with 10 mg/m² ABI-009 while three subjects had dose reduction during the treatment due to adverse events. Specifically, subject #1 was administered with 10 mg/m² ABI-009 weekly for 16 weeks. Subject #2 was administered with 10 mg/m² ABI-009 on week 1, 2, and 4, and was administered with 5 mg/m² ABI-009 at week 5, 7-9 and 11-16. No dose of ABI-009 was administered at week 3, 6, and 10 for subject #2. Subject #3 was administered with 10 mg/m² ABI-009 at week 1-6 and with 5 mg/m² ABI-009 at week 7-16. Subject 4 was administered with 10 mg/m² ABI-009 at week 1 and 2 and was administered with 5 mg/m² ABI-009 at week 5 and 6. No dose of ABI-009 was administered at week 3, 4, 7, and 8 and the treatment was ended at week 9. Trough concentration of rapamycin in whole blood for each week is shown in FIG. 1A.

In the four subjects who were treated with 10 mg/m² ABI-009, two of them developed Grade 1 thrombocytopenia, which results in a temporary interruption of ABI-009 administration in one subject. Grade 2 rash was developed in two subjects. As a result, dose of ABI-009 was reduced in one subject, and administration of ABI-009 was interrupted in one subject. Other adverse events reported include Grade 1 paresthesia, Grade 1 and Grade 2 hypertriglyceridemia or hypercholesterolemia, Grade 1 diarrhea, Grade 3 Cellulitis/infection requiring IV antibiotics.

As shown in Table 1, subject #3 had an unexpected improvement in 6 minute walking distance (6MWD) (m). The major improvement was observed only after four weeks of treatment. The 6MWD was increased from 290 m to 397.5 m. After 16 weeks of treatment, the 6MWD was increased to 425 m. The WHO Functional class for both subject #2 and subject #3 decreased from class III to class II after 8 weeks of treatment or 12 weeks of treatment.

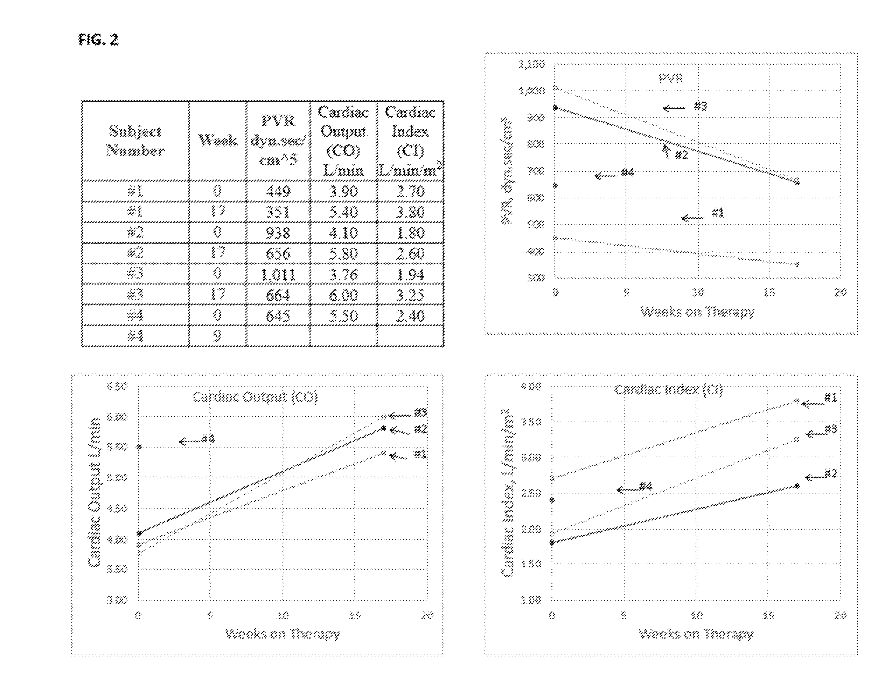

The level of pulmonary vascular pressure (PVR), cardiac output (CO), and cardiac input (CI) at the end of the ABI-009 treatment were compared to that at week 0. In all three subjects who had completed the sixteen-week treatment of ABI-009, PVR were significantly decreased. Surprisingly, all three subjects also had remarkable increase in cardiac output (CO). *See* FIG. 2.

**Table 1.**

| **Subject Number** | **Visit** | **Week** | **NT proBNP Result (pg/mL)** (normal range < 300 pg/ml) | **WHO Functional Class** | **6 Minute walking distance (m)** |
|---|---|---|---|---|---|
| **#1** | SCR | 0 | 1,041 | 3 | **311.0** |
| | WK5 | 5 | | 3 | 293.0 |
| | WK9 | 9 | 644 | 3 | 308.0 |
| | WK13 | 13 | 420 | 3 | 253.0 |
| | WK17 | 17 | 359 | 3 | 305.0 |
| **#2** | WK0 | 0 | 1,397 | 3 | **378.0** |
| | WK5 | 5 | 1,716 | 3 | N/A¹ |
| | WK9 | 9 | 1,367 | 3 | N/A |
| | WK13 | 13 | 844 | 2 | N/A |
| | WK17 | 17 | 2,510 | 2 | N/A |
| **#3** | SCR | 0 | 2,485 | 3 | **290.0** |
| | WK5 | 5 | 1,880 | 3 | 397.5 |
| | WK9 | 9 | 2,188 | 2 | 362.5 |
| | WK13 | 13 | 1,349 | 2 | 392.5 |
| | WK17 | 17 | 1,268 | 2 | 425.0 |
| **#4** | SCR | 0 | 251 | 3 | **340.0** |
| | WK5 | 5 | 259 | 3 | 337.5 |
| | EOT | 9 | 170 | 3 | 317.5 |

| | | | | | |
|---|---|---|---|---|---|
| SCR: at screening; WK: week; EOT: end of treatment. ¹ Subject #2 had injured ankle in car crash after screening visit, so no data of 6MWD for the duration of the treatment. | | | | | |

### 2 Part B.

In a modified dose-finding portion of the study, 3 dose levels of ABI-009 are tested in cohorts of 3 patients each (1 mg/m², 2.5 mg/m², and 5 mg/m²) using the 3+3 dose escalation de-escalation design.

### 3 Part C.

PAH patients with WHO FC III symptoms despite treatment with ≥2 PAH-specific therapies are eligible. ABI-009 was given IV, weekly, for 16 weeks at 1 mg/m², 2.5 mg/m², 5 mg/m², and 10 mg/m² using a 3+3 dose-finding design. Primary endpoints include dose-limiting toxicities and adverse events. Secondary endpoints include changes in WHO FC, 6MWD, and hemodynamics.

Six patients received ABI-009 and enrollment is ongoing. Five patients completed 16 weeks of therapy. Four patients received ABI-009 at the original starting dose of 10 mg/m²: one patient had no safety concerns, two patients required dose reduction to 5mg/m² due to rash (week 5) or paresthesia (week 7), and 1 patient discontinued treatment at week 8 due to cellulitis. Subsequently, dose-escalation scheme was modified to start at a lower dose of 1 mg/m². Thus far, 2 patients have completed 16 weeks of ABI-009 at 1 mg/m² without significant safety concerns.

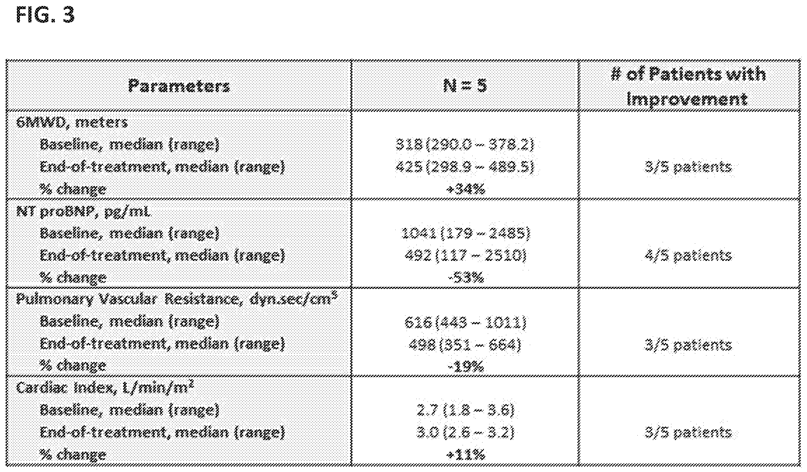

Functional and hemodynamic parameters were summarized in FIG. 3 for the 5 patients who completed 16 weeks of ABI-009. Each of the 5 patients had an improvement in some of the parameters, even at the lowest dose.

### 4 Part D.

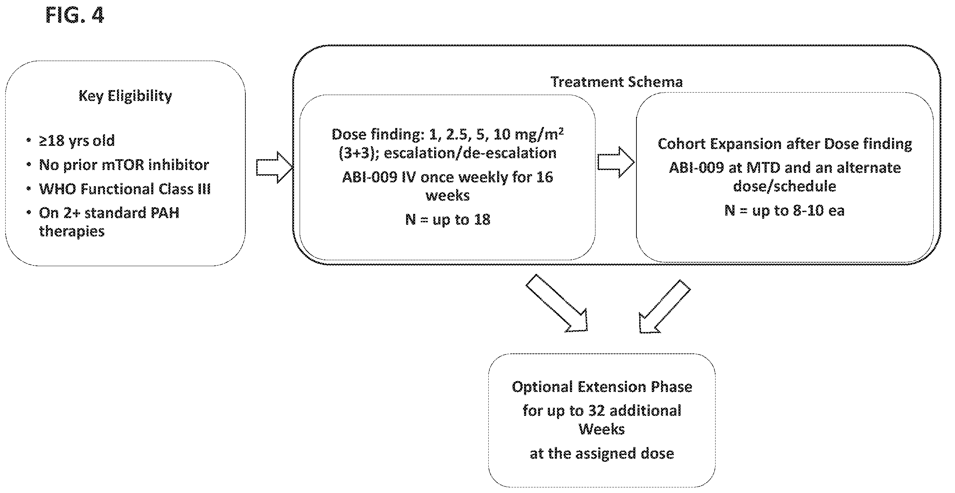

As of Feb 22, 2019, 9 patients have received treatment with IV administration of ABI-009. Four patients were treated with ABI-009 at a dose of 5-10 mg/m², three at 1 mg/m², and two at 2.5 mg/m². Among the nine patients, five patients have completed the 16 weeks of ABI-009 treatment; one patient (treated with ABI-009 at a dose of 10mg/m²) discontinued early at week 8. Three patients (including one patient treated with ABI-009 at the dose of 1 mg/m² and 2 patients treated with ABI-009 at the dose of 2.5 mg/m²) are currently in active treatment and have not completed 16 weeks of therapy. *See* FIG. 4 for study design.

Primary endpoints include a) MTD, DLT, and safety profile of 16 weeks of IV ABI-0009 and b) safety profile of up to 48 weeks of treatment. Secondary endpoints include a) changes in hemodynamics from baseline to end of treatment ("EOT") (baseline and week 17): pulmonary vascular resistance (PVR) (such as by right heart catheterization (RHC)), pulmonary artery pressure (PAP), pulmonary capillary wedge pressure (PCWP), central venous pressure (CVP) and right atrial pressure; b) changes at every-4-week assessments (baseline and weeks 5, 9, 13, and 17): doppler-echocardiography of right ventricular structure/function, 6-min walk distance (6MWD), WHO FC, pulmonary function testing; and c) 6MWD, WHO FC, and pulmonary function test at every eight weeks of the extension phase (*i.e.,* at week E9, E17, E23, and E23). Exploratory endpoints include: a) PK and trough levels of rapamycin for weekly treatment in patients with PAH; b) changes in PAH biomarkers: N-terminal pro brain natriuretic peptide (NTproBNP), C-reactive protein, troponin; c) changes in quality of life (emPHasis-10 questionnaire); and d) optional blood biomarkers for mTOR, correlative assessment with PAH biomarkers, clinical efficacy/safety.

Safety parameters were assessed among all the nine treated patients. Among the four patients received ABI-009 at 10 mg/m² (original starting dose), one patient had no safety concerns; two patients had the dose reduced to 5 mg/m² due to rash (week 5) or paresthesia (week 7) and completed therapy at 5 mg/m² without further safety concerns; the remaining one patient discontinued treatment at week 8 due to cellulitis.

Subsequently, the dosing schema was modified to escalate dosing from 1 mg/m², followed by 2.5 and 5 mg/m² if there were no safety concerns at each step. Two patients completed 16-week ABI-009 at 1 mg/m² without significant safety concerns. One patient treated at the same dose level (*i.e.,* 1 mg/m²) is ongoing at week 15 and no safety concerns were noted. The 2.5 mg/m² dose cohort is now enrolling with 2 patients treated to date.

The most common adverse events (all grade 1 and 2) to date have been diarrhea (4 patients), and thrombocytopenia, rash, and fatigue (2 patients for each). All occurred at the 10 mg/m² dose level and were managed with dose modifications and standard of care.

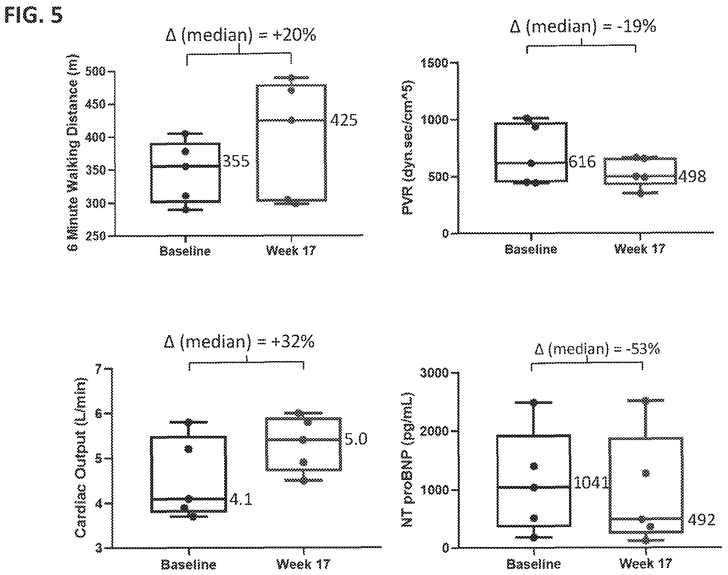

Efficacy parameters were assessed among the five patients that have completed sixteen weeks of therapy. As shown in FIG. 5, three out of five patients improved from WHO Functional Class III to WHO Functional Class II. Three out of five patients showed 16% to 47% increase in 6MWD. Among them, two patients improved more than 130 meters in 6MWD. Four out of five patients had reduction in PVR. The median PVR was reduced by 19% after sixteen weeks of treatment from 616 dyn.sec/cm⁵ to 498 dyn.sec/cm⁵. Two patients have shown a more than 30% decrease in PVR post-treatment. Three patients at the 10 mg/m² dose level had 38% to 62% increase in cardiac output, along with an improvement in cardiac index. Forced vital capacity (FVC) was also measured during pulmonary function test. The median FVC were increased by 10% post 16-week treatment. A median of NT-proBNP was reduced by 53% from 1041 pg/mL to 492 pg/mL post 16-week treatment. Four out of five patients have had a decrease in the NT-proBNP levels suggesting an improvement in left ventricular function. Note that one patient at dose level 10 mg/m² was in a car accident that resulted in a fractured foot during course of therapy. For this particular patient, while most parameters improved, the 6MWD did not.

Changes in quality of life was assessed by EmPHasis10. The total score for five patients improved from 146 at baseline to 99 at week 17. Per patient median (range) improved by 30%.

Taken together, all patients completing 16 weeks of therapy with ABI-009 combined with standard PAH therapy showed some improvement in functional capacity and/or hemodynamics.

Dose finding is ongoing, however, interim safety and efficacy results, including functional and hemodynamic measures support the ongoing investigation of ABI-009 in patients with severe PAH.

During the study, sirolimus trough levels (whole blood) in patients treated with different doses of ABI-009 were assessed. Results were shown in Table 2.

**Table 2. Sirolimus trough level (whole blood) data from Clinical study**

| **Dose** | **10 mg/m²** | **5 mg/m²** | **1 mg/m²** |
|---|---|---|---|
| **Number of Patients** | 2 | 3 | 3 |
| **Adult or Pediatric** | Adult | Adult | Adult |
| **N (# trough readings)** | 4 | 20 | 43 |
| **mean, ng/ml** | 11.0 | 6.6 | 2.8 |
| **stdev** | 4.8 | 3.0 | 2.3 |
| **min, ng/ml** | 7.3 | 3.6 | 1 |
| **max, ng/ml** | 17.5 | 13.5 | 10.9 |

### Study Population

Inclusion Criteria includes the following. 1. Male or female age >18 years old with a current diagnosis of WHO Group 1 PAH including idiopathic pulmonary arterial hypertension (IPAH), heritable pulmonary arterial hypertension (HPAH), drug and toxin induced PAH, or PAH associated with connective tissue disease, or congenital heart defects (repaired greater than 1 year prior to Screening). 2. Must meet following hemodynamic definition prior to initiation of study drug a. Mean PAP of ≥25 mmHg; b. PCWP or left ventricular end diastolic pressure (LVEDP) of ≤ 15 mm; c. PVR >5 mmHg/L/min (Woods unit). 3. Functional class III according to the WHO set forth at the Dana Point Classification 2008 Meeting. 4. On 2 or more specific standard PAH therapies (for ≥12 consecutive weeks and at stable dose for ≥8 consecutive weeks) unless documented inability to tolerate 2 standard therapies. 5. Meet the following criteria determined by pulmonary function tests completed at screening: a. Forced expiratory volume in one second (FEV1) ≥55% of predicted normal; b. FEV1: forced vital capacity (FVC) ratio ≥0.60. 6. 6MWD ≥ 150 meters and ≤450 meters.

A patient was not eligible for inclusion in this study if any of the following criteria apply. 1. History of heart disease including left ventricular ejection fraction (LVEF) ≤40% or clinically significant valvular constrictive or atherosclerotic heart disease (myocardial infarction, angina, cerebrovascular accident). 2. History of malignancy in 2 years prior to enrollment. 3. Pulmonary hypertension (PH) belonging to groups 2 to 5 of the 2013 Nice classification. 4. Current or recent (<3 months) use of intravenous inotropic or vasopressor agents for the treatment of PAH. 5. Recent (<3 months) PAH related hospital admission. 6. History of allergic reactions attributed to compounds of similar chemical or biologic composition including macrolide (e.g., azithromycin, clarithromycin, dirithromycin, and erythromycin) and ketolide antibiotics. 7. Uncontrolled diabetes mellitus as defined by HbA1c >8% despite adequate therapy. 8. Uncontrolled hyperlipidemia (serum triglyceride ≥300 mg/dL). 9. Serum cholesterol ≥350 mg/dL. 10. Surgery within 3 months of start date of study drug. 11. Baseline cytopenias: a. Absolute Neutrophil Count ≤1.5 x 109/L; b. Hemoglobin ≤9 g/dL; c. Platelet count ≤ 100,000/mm³. 12. Baseline liver disease: ALT/AST, total bilirubin, alkaline phosphatase ≥1.5 x ULN. 13. Creatinine clearance (Cockroft formula) ≤30 mL/min. 14. Prior use of an mTOR inhibitor within previous 6 months from enrollment. 15. Previous lung transplant. 16. Use of strong inhibitors and inducers of CYP3A4 within the 14 days prior to receiving the first dose of ABI-009. Additionally, use of any known CYP3A4 substrates with narrow therapeutic window (such as fentanyl, alfentanil, astemizole, cisapride, dihydroergotamine, pimozide, quinidine, terfanide) within the 14 days prior to receiving the first dose of ABI-009.

### Key Safety Assessment

The AE toxicity grading scale used was the NCI CTCAE Version 4.1. A serious adverse event (SAE) was defined as an AE that meets at least 1 of the following serious criteria: 1) fatal; 2) life-threatening (places the patient at immediate risk of death); 3) requires in-patient hospitalization or prolongation of existing hospitalization; 4) results in persistent or significant disability/incapacity; 5) congenital anomaly/birth defect; and 6) other medically important serious event.

### Key Efficacy Assessment

The following were measured every week, every four weeks at baseline and at 5, 9, 13, and 17 weeks or at baseline and at 17 weeks: 1) Doppler-echocardiographic assessments of right ventricular structure and function; 2) 6-minute walk distance (6MWD); 3) Pulmonary function test; 4) NT Pro-BNP; 5) CRP; 6) Troponin; 7) fasting lipids; 8) WHO Functional class; 9) rapamycin PK; 10) pulmonary vascular resistance (PVR) by right heart catheterization; 11) pulmonary artery pressure (PAP); 12) pulmonary artery occlusion pressure (PAOP); 13) pulmonary capillary wedge pressure (PCWP); 14) central venous pressure (CVP); 15) cardiac output; 16) cardiac input.

### Reference Example 2: Preclinical Study of nab-rapamycin for Treating Pulmonary Arterial Hypertension (PAH)

Preclinical studies were performed to evaluate the biodistribution of ABI-009.

### 1 Part A.

Single-dose ABI-009 IV at 1.7mg/kg (10mg/m²) was administered to rats (3 rats/group). Blood and organs were collected at 2, 8, 24, 72, and 120 hours to measure sirolimus concentrations.

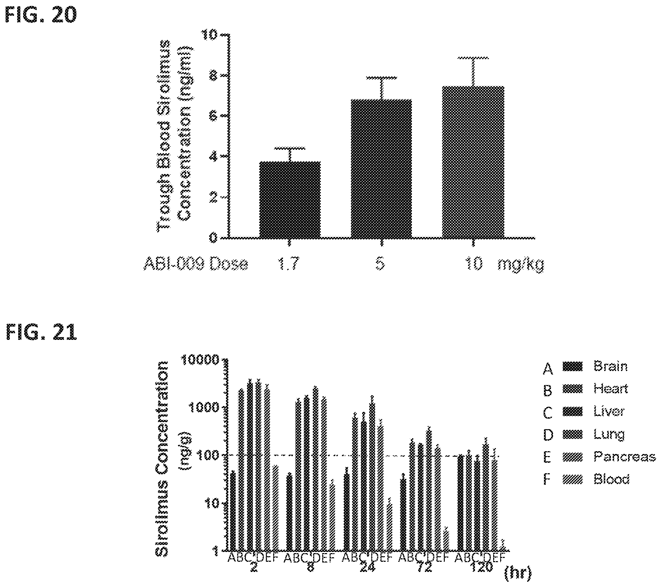

The total tissue exposure (AUC) was significantly higher in the lung versus other tissues over 120 hours (P<0.0001, ANOVA). Sirolimus lung concentrations were 3358, 2436, 1190, 322, and 171 ng/g at 2, 8, 24, 72, and 120 hours and lung/blood ratios were 57, 98, 125, 121, and 140, respectively. *See* FIG. 21.

### 2 Part B.

The whole blood PK and tissue distribution at 24 hrs after nab-rapamycin IV administration (dose 1 mg/kg) in rats (N=5) to oral rapamycin (dose 1.6 mg/kg) in rats (N=5) *(See* Napoli KL, Wang ME, Stepkowski SM, et al: Distribution of sirolimus in rat tissue. Clin Biochem 30:135-42, 1997) were compared to determine relative uptake into the lung (target organ for PAH) and liver (major excretion route for rapamycin). Blood and tissue levels were measured by LC/MS/MS or HPLC.

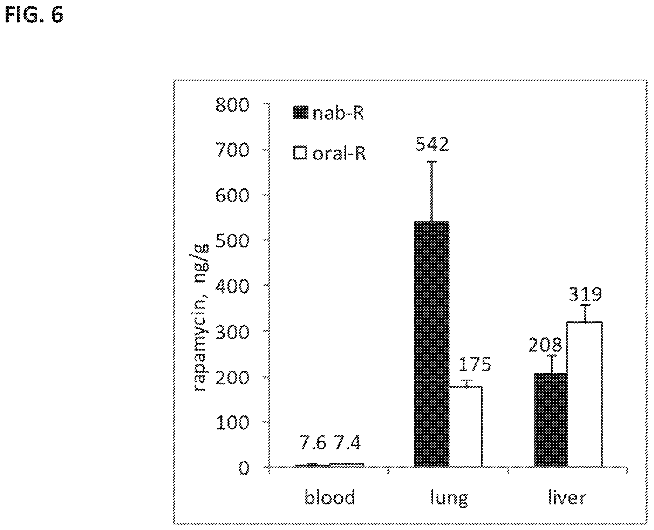

As shown in FIG. 6, Blood levels at 24 hours were comparable. *Nab*-R (nab-rapamycin) levels in lung were significantly higher than in liver (p<0.05); *nab*-R lung levels were significantly higher than estimated Oral-R in lungs (p<0.05); *nab*-R liver levels were significantly lower than estimated Oral-R in liver (p<0.05). (Paired Student's t-Test, used for all comparisons.) The calculated tissue extraction ratios (concentration of rapamycin in tissue/concentration of rapamycin in blood) in the lung were 72 and 24 respectively for nab-rapamycin and oral rapamycin indicating a 3-fold higher lung targeting for nab-rapamycin (P<0.05, student's t-test). In contrast, the extraction ratios for the liver were 27 and 43 respectively for nab-rapamycin and oral rapamycin, a 1.6 fold decrease for nab-rapamycin suggesting a lower rate of hepatic uptake due to the albumin bound formulation and supporting a longer persistence in the circulation. The roughly 2-fold higher levels in liver compared to lung for oral rapamycin are expected since hepatic metabolism is the major excretion pathway for oral rapamycin and confirms that there are no specific mechanisms to increase lung uptake for oral rapamycin. The 2.6-fold higher levels in lung compared to liver for nab-rapamycin strongly support specific transport mechanisms in the lung tissue resulting on increased lung uptake.

The results demonstrate a high penetration of nab-rapamycin in lung tissue.

### Example 3: Pharmacokinetics study following subcutaneous and intravenous dosing of ABI-009 in Sprague Dawley (SD) rats

Female SD rats received a single dose of nab-rapamycin (ABI-009) subcutaneously (i.e., "SC" or "subQ") or intravenously (IV). The study design is summarized below in Table 3. No inflammation or toxicity was observed after administration at the subcutaneous injection sites at any time point compared with the saline control (vehicle).

| **Table 3. Study Design of Single Dose of ABI-009 in Rats** | | | | | |
|---|---|---|---|---|---|
| **Group** | **No. mice** | **Test material** | **Route of administration** | **Dose** | **Euthanasia time point (hours)** |
| 1 | 3 | vehicle | SC | 0.5 ml/kg | 168 |
| 2 | 3 | ABI-009 | SC | 0.56 mg/kg | 24 |
| 3 | 3 | ABI-009 | SC | 0.56 mg/kg | 168 |
| 4 | 3 | ABI-009 | SC | 1.7 mg/kg | 24 |
| 5 | 3 | ABI-009 | SC | 1.7 mg/kg | 168 |
| 6 | 3 | ABI-009 | SC | 5 mg/kg | 24 |
| 7 | 3 | ABI-009 | SC | 5 mg/kg | 168 |
| 8 | 3 | ABI-009 | SC | 9.5 mg/kg | 24 |
| 9 | 3 | ABI-009 | SC | 9.5 mg/kg | 168 |
| 10 | 3 | ABI-009 | IV | 1.7 mg/kg | 24 |
| 11 | 3 | ABI-009 | IV | 1.7 mg/kg | 168 |

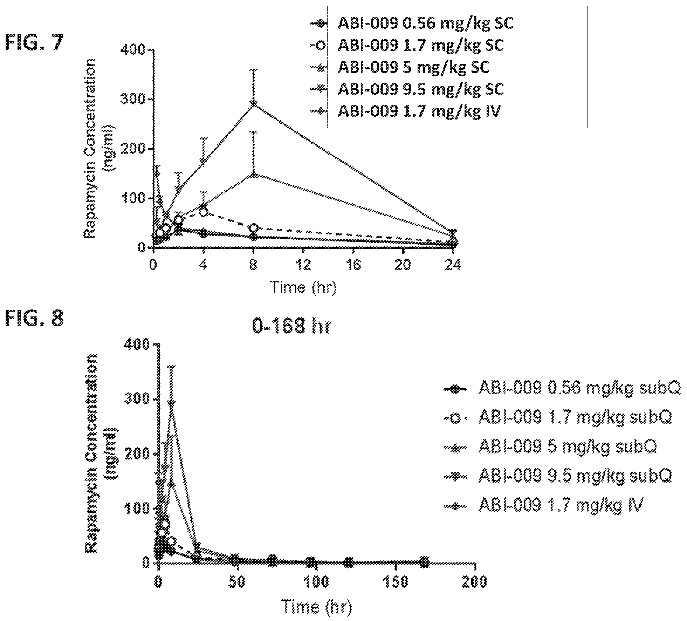

After subcutaneous or intravenous injection of ABI-009, rapamycin concentrations in the whole blood were measured at different time points. The results of the whole blood collections are shown in FIGS. 7-9 and summarized in Tables 4 and 5 below.

| **Table 4. Rapamycin Concentration after ABI-009 Administration** | | | | | | | | | |
|---|---|---|---|---|---|---|---|---|---|
| | **ABI-009 0.56 mg/kg SC** | | | **ABI-009 1.7 mg/kg SC** | | | **ABI-009 5 mg/kg SC** | | |
| Time (hr) | Average (ng/ml) | SD | N | Average (ng/ml) | SD | N | Average (ng/ml) | SD | N |
| 0.25 | 14.70 | 3.66 | 3 | 24.63 | 4.74 | 3 | 21.40 | 5.39 | 3 |
| 0.5 | 16.77 | 3.66 | 3 | 30.93 | 6.37 | 3 | 19.20 | 6.92 | 3 |
| 1 | 22.53 | 4.27 | 3 | 40.23 | 6.55 | 3 | 30.17 | 5.91 | 3 |
| 2 | 37.40 | 10.02 | 3 | 56.67 | 1.62 | 3 | 61.73 | 9.81 | 3 |
| 4 | 28.37 | 4.58 | 3 | 72.60 | 14.10 | 3 | 86.60 | 26.54 | 3 |
| 8 | 22.70 | 5.22 | 3 | 40.57 | 3.56 | 3 | 149.70 | 84.47 | 3 |
| 24 | 6.95 | 1.29 | 3 | 11.80 | 1.80 | 3 | 24.17 | 11.65 | 3 |
| 48 | 4.13 | 1.10 | 3 | 5.75 | 0.80 | 3 | 6.87 | 2.04 | 3 |
| 72 | 4.57 | 3.51 | 3 | 7.32 | 5.96 | 3 | 3.59 | 0.27 | 3 |
| 96 | 1.89 | 0.52 | 3 | 2.37 | 0.80 | 3 | 1.80 | 0.54 | 3 |
| 120 | 1.40 | 0.44 | 3 | 1.75 | 0.60 | 3 | 1.48 | 0.29 | 3 |
| 168 | 1.01 | 0.28 | 3 | 1.18 | 0.19 | 3 | 0.90 | 0.39 | 3 |

| **Table 5. Rapamycin Concentration after ABI-009 Administration** | | | | | | |
|---|---|---|---|---|---|---|
| | **ABI-009 9.5 mg/kg SC** | | | **ABI-009 1.7 mg/kg IV** | | |
| Time (hr) | Average (ng/ml) | SD | N | Average (ng/ml) | SD | N |
| 0.25 | 51.70 | 31.20 | 3 | 149.00 | 16.64 | 3 |
| 0.5 | 37.83 | 8.17 | 3 | 93.00 | 10.75 | 3 |
| 1 | 64.93 | 7.43 | 3 | 66.30 | 5.48 | 3 |
| 2 | 116.27 | 36.19 | 3 | 40.07 | 8.59 | 3 |
| 4 | 171.67 | 49.57 | 3 | 34.80 | 0.85 | 3 |
| 8 | 289.33 | 70.88 | 3 | 22.13 | 3.86 | 3 |
| 24 | 30.03 | 4.82 | 3 | 8.85 | 1.46 | 3 |
| 48 | 8.93 | 1.20 | 3 | 4.66 | 1.53 | 3 |
| 72 | 5.09 | 2.08 | 3 | 2.95 | 0.85 | 3 |
| 96 | 2.58 | 0.84 | 3 | 1.78 | 0.42 | 3 |
| 120 | 1.76 | 0.44 | 3 | 1.39 | 0.36 | 3 |
| 168 | 4.09 | 5.06 | 3 | 0.87 | 0.30 | 3 |

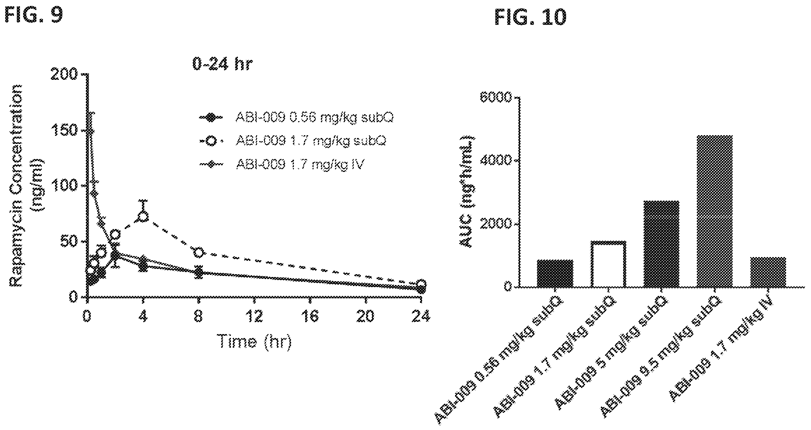

Surprisingly, as summarized in FIG. 10 and Table 6, below, subcutaneous administration enhanced bioavailability as indicated by total area under the curve (AUC) compared with intravenous administration. Subcutaneous administration of only 0.56 mg/kg ABI-009 produced similar drug exposure at 1/3rd the dose of IV ABI-009 (1.7 mg/kg). Further, subcutaneous administration reduced the maximum concentration achieved (Cmax) and delayed the time to reach the maximum concentration (Cmax time). Rapamycin peak levels and AUC in blood increased with higher subcutaneous ABI-009 doses.

| **Table 6. Pharmacokinetics of ABI-009 Administration in Rats** | | | | | |
|---|---|---|---|---|---|
| **Route** | **SC** | **SC** | **SC** | **SC** | **IV** |
| Dose (mg/kg) | 0.56 | 1.7 | 5 | 9.5 | 1.7 |
| Cmax (ng/mL) | 37.40 | 72.60 | 149.70 | 289.33 | 149.00 |
| Cmax Time (h) | 2 | 4 | 8 | 8 | 0.25 |
| AUC (ng*h/mL) | 860.8 | 1451 | 2734 | 4813 | 962.6 |

### Example 4: Biodistribution of ABI-009 after administration in rats

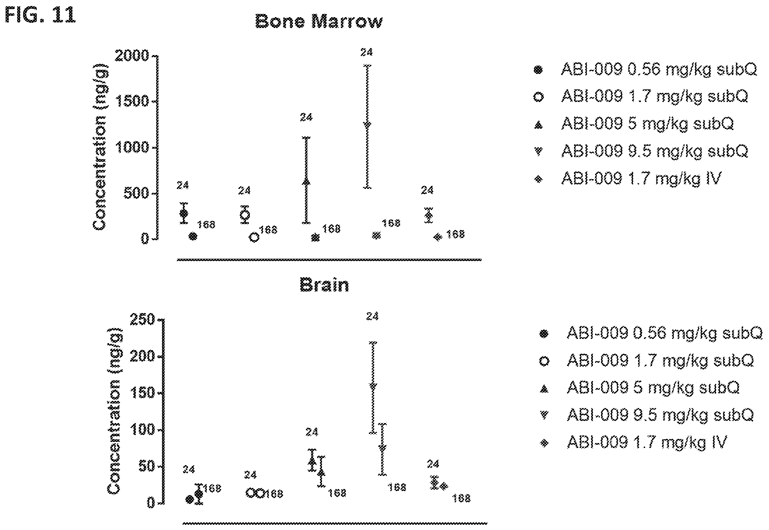

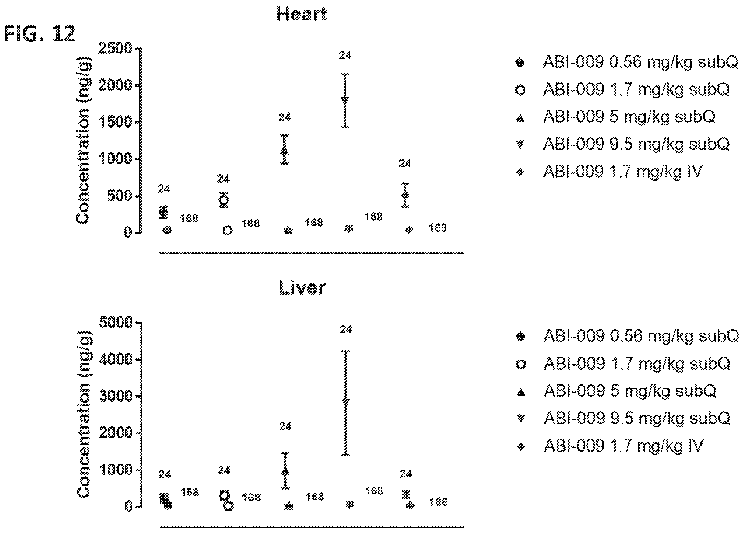

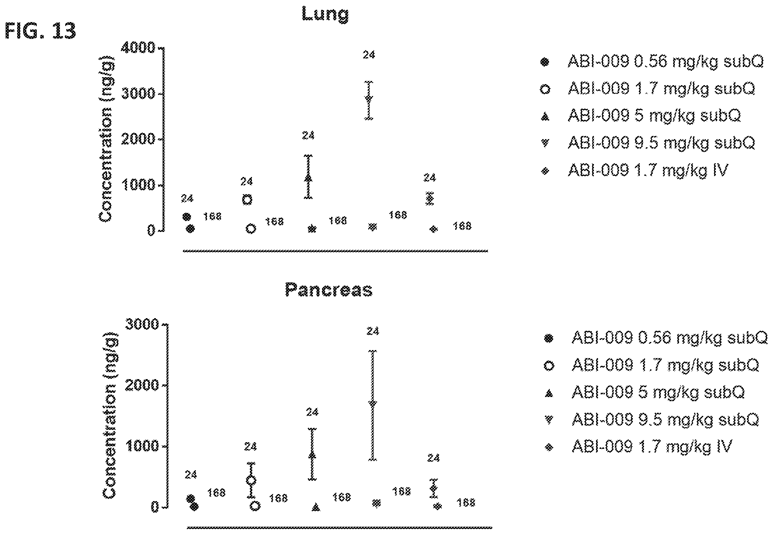

Tissues were harvested from the rats described above in Example 3 at either 24 hours or 168 hours (see Table 3 for study design) post-administration by subcutaneous (subQ) or intravenous (IV) route of ABI-009. The concentration of rapamycin in particular rat tissues 24 or 168 hours post-administration is indicated in FIG. 11 (bone marrow and brain), FIG. 12 (heart and liver), and FIG. 13 (lung and pancreas).

The subcutaneous route of administration resulted in significant distribution to all organs tested, including bone marrow, brain, heart, liver, lung, and pancreas. The pattern of organ distribution was similar between subcutaneous and intravenous but subcutaneous administration at 0.56 mg/kg dose was able to produce similar tissue concentrations as intravenous administration at 1.7 mg/kg dose. There was a significant drop in rapamycin concentration between 24 and 168 hours in well-perfused organs including the heart, liver, lung, and pancreas. However, the brain concentration was relatively stable between 24 and 168 hours.

### Example 5: Toxicology study following repeated subcutaneous dosing of ABI-009 in SD rats

The objectives of the study were to assess the overall safety and local toxicity at injection sites following repeated ABI-009 SC injections in SD rats. The signs of clinical distress were observed to determine toxicity. Skin samples from the injection sites were analyzed for signs of inflammation and necrosis by histopathology.

Fifteen female Sprague Dawley (SD) rats weighing 160-180g were used in the study. ABI-009 was dissolved in saline to prepare a stock solution (10 mg/ml), then further diluted in HSA 0.9% saline solution to prepare subcutaneous (volume: 1.0 ml/kg).

### A. Study design

Rats were divided into 5 groups of 3 animals each. Rats were weighed and dosed SC as specified in Table 7 every 4 days for 4 weeks (7 injections).

**Table 7. Treatment Groups**

| Group | Number of Rats | Test articles | ROA | Dose | Dose volume | Schedul e |
|---|---|---|---|---|---|---|
| 1 | 3 | 0.9% Saline | SC | - | 1.0 ml/kg | Once every 4 days for 4 weeks |
| 2 | 3 | HSA in 0.9% Saline | SC | 90 mg HSA/kg | | |
| 3 | 3 | ABI-009 | SC | 1.7 mg/kg | | |
| 4 | 3 | ABI-009 | SC | 5 mg/kg | | |
| 5 | 3 | ABI-009 | SC | 10 mg/kg | | |

| | | | | | | |
|---|---|---|---|---|---|---|
| SC = subcutaneous injection | | | | | | |

Animals were examined daily for clinical signs of overall toxicity and the local injection sites examined for reactions to subcutaneous injection.

Whole blood samples were collected prior to each injection for animals receiving ABI-009 (Groups 3, 4, and 5) and analyzed for trough sirolimus levels.

All animals were euthanized after 4 weeks and skin samples from local injection sites were examined by histopathology for signs of local toxicity.

### B. Experiment procedures

### 1. Dosing solution preparation

Vehicle controls consist of 0.9% saline solution and HSA in 0.9% saline solution. Final concentration of HSA solution is 90 mg/ml, based on the albumin:sirolimus ratio of 9:1 of the test article ABI-009 (manufacture lot # C345-001, Fisher lot #51394.2). Each vial of ABI-009 (C345-001) contains 97.4 mg sirolimus and 874 mg human albumin. HSA saline solution is diluted from 20% Grifols albumin stock solution (200 mg/ml).

For ABI-009 dosing solutions, first make a stock ABI-009 solution of 10 mg/ml, then dilute to desired concentrations for dosing solution using HSA-saline solution. A vial of 100 mg of ABI-009 was dissolved in 10 ml of 0.9% saline to prepare a solution of 10 mg/ml.

ABI-009 solution of 5 mg/ml was prepared by diluting 0.6 ml of stock solution (10 mg/ml) with 0.6 ml of HSA-0.9% saline to prepare a solution of 5.0 mg/ml for group 4. ABI-009 solution of 1.7 mg/ml was prepared by diluting 0.3 ml of ABI-009 solution from group 4 (5.0 mg/ml) with 0.6 ml of HSA-0.9% saline to prepare a solution of 1.7 mg/ml for group 3.

### 2. Dosing

The rats were anesthetized, weighed, and administered with ABI-009 solutions, HSA solution and saline according to Table 8 by subcutaneous (SC) injection every 4 days for 4 weeks (7 injections).

**Table 8. Dosing volume**

| Group | Test articles | ROA | Dose (mg/kg) | Dosing Sol (mg/ml) | Dose Volume (ml/kg) |
|---|---|---|---|---|---|
| 1 | 0.9% Saline | SC | 0 | 0 | 1.0 |
| 2 | HSA in 0.9% Saline | SC | 0 (90 mg HSA) | 0 (90 mg HSA) | 1.0 |
| 3 | ABI-009 | SC | 1.7 | 1.7 | 1.0 |
| 4 | ABI-009 | SC | 5 | 5 | 1.0 |
| 5 | ABI-009 | SC | 10 | 10 | 1.0 |

Rats were examined once daily for clinical signs of overall toxicity and the local injection sites for reactions to subcutaneous injection. The signs of clinical distress were observed to determine toxicity. Piloerection, weight loss, lethargy, discharges, neurological symptoms, morbidity, redness and inflammation of injection site, and any other signs considered abnormal for animal behavior. Pictures of the injection site for all rats were taken before and after the SC injection.

### 3. Sample collection and analysis

For rats treated with ABI-009 (Groups 3, 4, and 5), rats were anesthetized and bled for samples into pre-chilled K2EDTA tubes before each administration (except 1st dose). Whole blood was collected, stored in labeled Eppendorf tubes at -80°C, and analyzed for trough sirolimus levels.

All animals were euthanized at the final euthanasia points of Day 29 (96 hrs post week 4 Day 25 ABI-009 administrations). At the final euthanasia time point, whole blood samples were collected for analysis of trough sirolimus level. The brain, lung, liver, heart, pancreas, and bone marrow were collected, flushed with saline to remove the blood, divided into 2 portions, and flash frozen in individually labeled tubes, and stored at -80°C. The frozen blood samples from ABI-009 treated groups (Groups 3, 4, and 5) are shipped on dry ice to BASi. Trough sirolimus blood levels were analyzed by BASi by LC/MS/MS method.

At the final euthanasia time point, skin and lower dermal layer at region of SC administration were excised for histological analysis by H&E staining for signs of inflammation by histopathology. Fifteen formalin-fixed rat skin samples were subject to histopathologic measurement and processed routinely. One slide from each block was sectioned and stained with hematoxylin and eosin (H&E). Slides were evaluated by a board-certified veterinary pathologist using light microscopy. Histologic lesions were graded for severity 0-5 (0=not present/normal, 1 = minimal, 2 = mild, 3 = moderate, 4 = marked, 5 = severe). Mean scores of different groups were analyzed by t-test.

### C. Results

### 4. Systemic toxicity

The signs of clinical distress were observed daily to determine toxicity. Piloerection, weight loss, lethargy, discharges, neurological symptoms, morbidity, redness and inflammation of injection site, and any other signs considered abnormal for animal behavior. Rats were normal post dosing of saline, HSA, and ABI-009 at current dose regimen (1.7-10 mg/kg, 7 doses), with no signs of clinic stress observed during the study.

There was no body weight loss (< 20%), and all treatment groups gained weight during the study (Table 9). The results showed that rats tolerated subcutaneous injection of ABI-009 over a dose range of 1.7-10.0 mg/kg.

**Table 9. Effect of Treatment on the Body Weight of Rats**

| | | Body weight (g) | | | | | | |
|---|---|---|---|---|---|---|---|---|
| Groups | Mouse # | Day 1 | Day 5 | Day 9 | Day 13 | Day 17 | Day 21 | Day 25 |
| Group 1 | 1 | 181 | 187 | 195 | 202 | 207 | 210 | 213 |
| 0.9% saline | 2 | 200 | 196 | 206 | 210 | 214 | 218 | 228 |
| 1 ml/kg | 3 | 187 | 191 | 193 | 201 | 204 | 209 | 219 |
| | average | 189 | 191 | 198 | 204 | 208 | 212 | 220 |
| | SD | 9.71 | 4.51 | 7.00 | 4.93 | 5.13 | 4.93 | 7.55 |
| Group 2 | 4 | 182 | 188 | 196 | 201 | 210 | 212 | 222 |
| HSAin0.9% saline | 5 | 197 | 200 | 208 | 214 | 221 | 226 | 239 |
| 1 ml/kg | 6 | 173 | 180 | 188 | 199 | 207 | 211 | 216 |
| | average | 184 | 189 | 197 | 205 | 213 | 216 | 226 |
| | SD | 12.12 | 10.07 | 10.07 | 8.14 | 7.37 | 8.39 | 11.93 |
| Group 3 | 7 | 191 | 189 | 192 | 199 | 207 | 206 | 215 |
| ABI-009 | 8 | 186 | 189 | 186 | 193 | 199 | 200 | 209 |
| 1.7 mg/kg | 9 | 186 | 188 | 189 | 195 | 205 | 205 | 212 |
| | average | 188 | 189 | 189 | 196 | 204 | 204 | 212 |
| | SD | 2.89 | 0.58 | 3.00 | 3.06 | 4.16 | 3.21 | 3.00 |
| Group 4 | 10 | 195 | 193 | 192 | 196 | 200 | 199 | 208 |
| ABI-009 | 11 | 181 | 182 | 189 | 193 | 195 | 198 | 202 |
| 5 mg/kg | 12 | 196 | 197 | 190 | 195 | 204 | 202 | 208 |
| | average | 191 | 191 | 190 | 195 | 200 | 200 | 206 |
| | SD | 8.39 | 7.77 | 1.53 | 1.53 | 4.51 | 2.08 | 3.46 |
| Group 5 | 13 | 182 | 179 | 182 | 183 | 191 | 192 | 198 |
| ABI-009 | 14 | 188 | 180 | 187 | 189 | 193 | 198 | 197 |
| 10 mg/kg | 15 | 190 | 183 | 189 | 193 | 198 | 195 | 204 |
| | average | 187 | 181 | 186 | 188 | 194 | 195 | 200 |
| | SD | 4.16 | 2.08 | 3.61 | 5.03 | 3.61 | 3.00 | 3.79 |

### 5. Local toxicity

Fifteen formalin-fixed rat skin samples from the region of SC administration were subject to histopathologic measurement. Histopathologic findings in skin samples included necrosis and mixed infiltrates of inflammatory cells in perivascular zones; both lesions were observed in the subcutaneous tissues/subcutis.

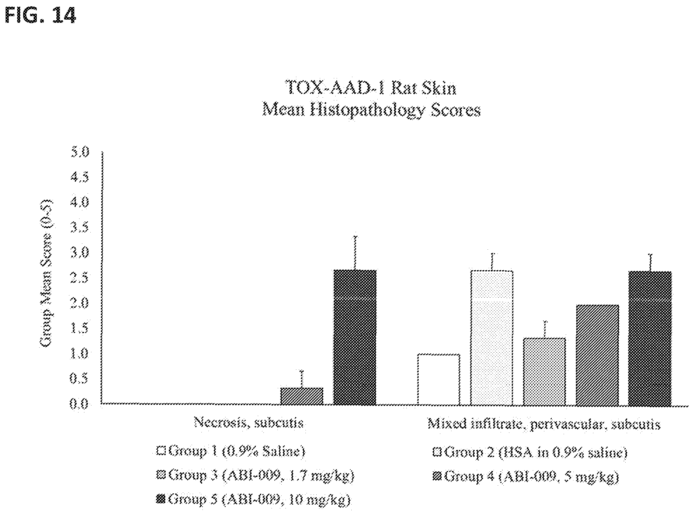

Necrosis was focal and characterized by a region of loss of normal cells, neutrophil infiltration, hemorrhage, and fibrin exudation, with variable adjacent fibroplasia. Necrosis was only observed in samples from animals treated with ABI-009 at 5 mg/kg (Group 4, 1 animal with minimal necrosis) and 10 mg/kg (Group 5, all 3 animals with mild to marked necrosis) dose levels, whereas saline (Group 1), HSA (Group 2), and ABI-009 at 1.7 mg/kg (Group 3) caused no necrosis. *See* Table 10 and FIG. 14. Only ABI-009 at the highest dose of 10 mg/kg showed significantly increased necrosis score compared with HSA group (P = 0.02, t-test).

**Table 10. Effect of Treatment on the Body Weight of Rats**

| Group | Sample | Necrosis, subcutis | Mixed infiltrate, perivascular, subcutis |
|---|---|---|---|
| Group 1 (0.9% Saline) | 1 | 0 | 1 |
| | 2 | 0 | 1 |
| | 3 | 0 | 1 |
| | mean | 0.00 | 1.00 |
| | SEM | 0.00 | 0.00 |
| Group 2 (HSA in 0.9% saline) | 4 | 0 | 2 |
| | 5 | 0 | 3 |
| | 6 | 0 | 3 |
| | mean | 0.00 | 2.67 |
| | SEM | 0.00 | 0.33 |
| | p vs Grp 1 | | 0.01 |
| Group 3 (ABI-009, 1.7 mg/kg) | 7 | 0 | 1 |
| | 8 | 0 | 2 |
| | 9 | 0 | 1 |
| | mean | 0.00 | 1.33 |
| | SEM | 0.00 | 0.33 |
| | p vs Grp 2 | | 0.05 |
| Group 4 (ABI-009, 5 mg/kg) | 10 | 0 | 2 |
| | 11 | 1 | 2 |
| | 12 | 0 | 2 |
| | mean | 0.33 | 2.00 |
| | SEM | 0.33 | 0.00 |
| | p vs Grp 2 | 0.37 | 0.12 |
| Group 5 (ABI-009, 10 mg/kg) | 13 | 2 | 3 |
| | 14 | 4 | 3 |
| | 15 | 2 | 2 |
| | mean | 2.67 | 2.67 |
| | SEM | 1.00 | 0.00 |
| | p vs Grp 2 | 0.02 | 1.00 |

Mixed inflammatory cell infiltration in subcuticular perivascular zones was characterized by infiltration and aggregation of lymphocytes, plasma cells, macrophages, occasional multinucleated giant cells, and variable numbers of neutrophils. Mixed inflammatory cell infiltration was observed in all treatment groups, with mean scores being the highest in animals treated with HSA (Group 2) and ABI-009 at 10 mg/kg (Group 5). For low dose ABI-009 injection at 1.7 mg/kg (Group 3), the mean score was similar to control group receiving saline injection (Group 1)*. See* Table 10 and FIG. 14. High mixed inflammatory cell infiltration observed in the HSA group (Group 2) compared with saline control (P = 0.01, t-test) suggests that local inflammation was largely caused by the injection of the heteroprotein human serum albumin.

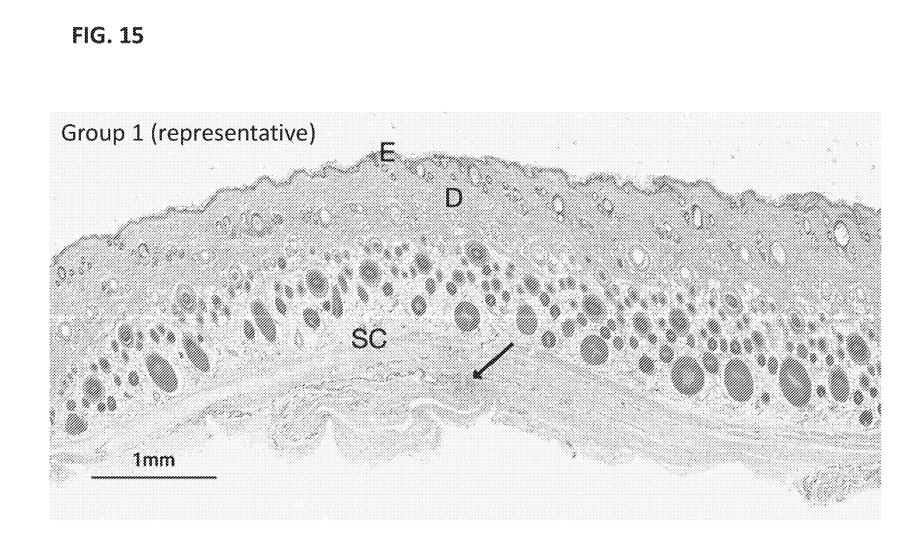

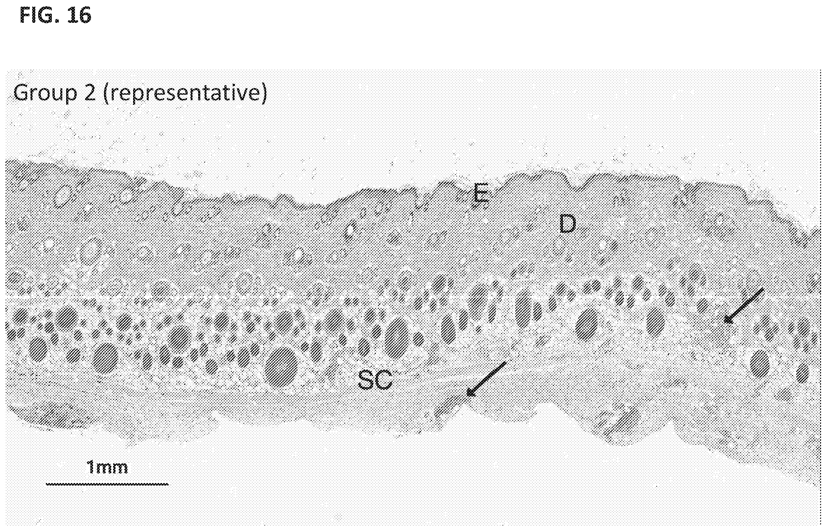

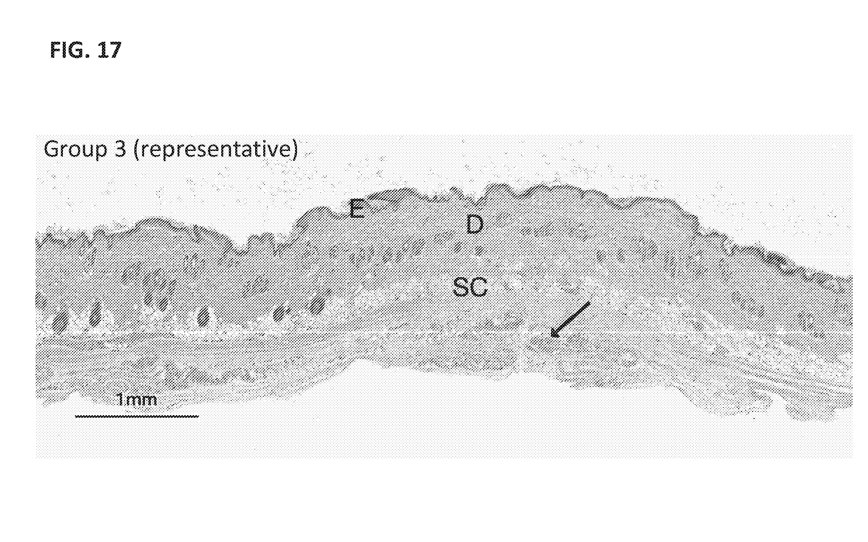

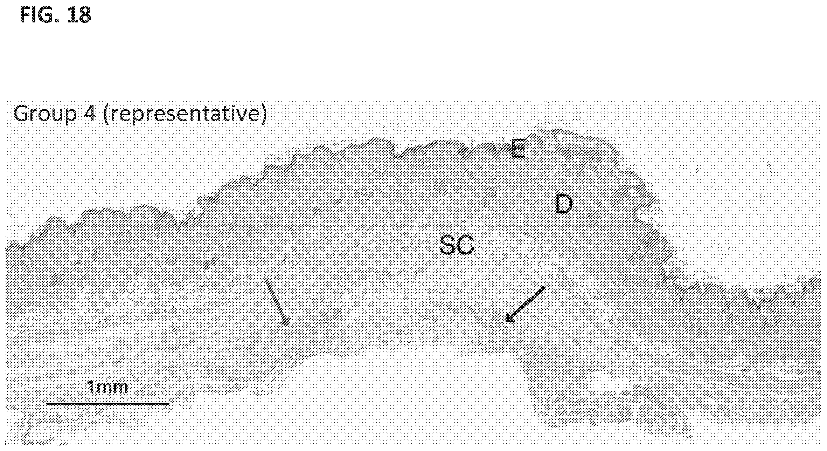

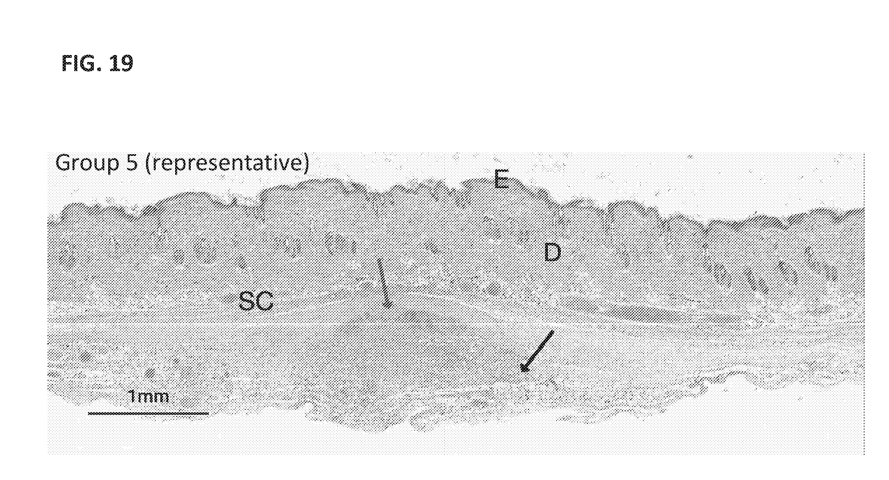

Representative histology images for rats in each group were shown in FIGS. 15-19.

For ABI-009 treatment groups, there were dose-associated increases in local toxicities with increasing ABI-009 dose. At the lowest dose of ABI-009 1.7 mg/kg, the histology of local injection sites was similar to the saline control group; whereas necrosis and subcutaneous tissue inflammatory cell infiltration were the most severe in the ABI-009-treated animals at the 10 mg/kg dose level.

### 6. Trough sirolimus blood levels

Trough sirolimus blood samples were collected before each injection (at Day 5, 9, 13, 17, 21, 25, 29) for groups treated with ABI-009 (except the 1^{st} dose on Day 1) and analyzed by BASi using LC/MS/MS method. Individual trough levels are shown in Table 11. Most trough sirolimus blood levels 4 days after SC injection were consistently in the range of 2-20 ng/ml. Two samples in the ABI-009 10 mg/kg group (Group 5) were clearly outliers. The reason for this observation cannot be ascertained. However, the abnormal high trough levels only occurred in the highest ABI-009 dose group that also showed mild to marked necrosis in the subcutaneous tissue, suggesting that skin lesions may hamper the normal absorption of ABI-009 and lead to prolonged drug retention.

**Table 11. Trough Sirolimus Blood Levels**

| Days / ID | Group 3 (ABI-009 1.7 mg/kg) | | | Group 4 (ABI-009 5 mg/kg) | | | Group 5 (ABI-009 10 mg/kg) | | |
|---|---|---|---|---|---|---|---|---|---|
| | #3-7 | #3-8 | #3-9 | #4-10 | #4-11 | #4-12 | #5-13 | #5-14 | #5-15 |
| 5 | 3.1 | 2.38 | 2.56 | 4.5 | 3.63 | 6 | 3.28 | 8.37 | 4.54 |
| 9 | 5.56 | 7.91 | 4.16 | 6.42 | 4.57 | 7.67 | 19.1 | 19.3 | 4.64 |
| 13 | 2.92 | 3.1 | 3.35 | 18.3 | 5.97 | 9.8 | 4.9 | 6.64 | 3.87 |
| 17 | 4.02 | 13 | 2.04 | 1.58 | 3.64 | 9.7 | 11.4 | 6.79 | 14.8 |
| 21 | 0.24 | 1.69 | 3.39 | 3.44 | 3.63 | 4.8 | *ALQ 201** | 6.83 | 5.27 |
| 25 | 5.32 | 2.18 | 3.06 | 7.03 | 4.5 | 19.7 | 3.28 | 8.34 | 5.6 |
| 29 | 3.04 | 3.17 | 2.77 | 5.1 | 3.64 | 9.03 | 4.34 | 4.69 | *92.8** |
| Mea n | 3.760 | | | 6.793 | | | 7.683 | | |
| SEM | 0.5736 | | | 1.005 | | | 1.139 | | |

For each ABI-009 treatment group, there was no significant drug accumulation over the time course of the study, as trough blood sirolimus levels remained generally stable. There was a dose-dependent increase in mean trough blood sirolimus levels with increasing ABI-009 dose. Compared with ABI-009 1.7 mg/kg group, higher trough levels were observed in ABI-009 5 mg/kg group (P = 0.06) and 10 mg/kg group (P = 0.01) (FIG. 20).

In summary, rats were normal post dosing of ABI-009 at current dose regimen (1.7-10 mg/kg, 7 doses), with no body weight loss observed during the study. The histopathology results demonstrated dose-associated local signs of toxicity, with mild to marked necrosis at the highest ABI-009 dose (10 mg/kg). Mixed inflammation cells infiltration may possibly be caused by the heteroprotein HSA. ABI-009 at 1.7 mg/kg (solution concentration 1.7 mg/ml) showed local injection responses similar to saline control. There was no significant drug accumulation following repeated SC injections. Trough blood sirolimus levels increased with higher ABI-009 dose.

The results showed that rats tolerated systemically with multiple doses of ABI-009 over a range of 1.7-10.0 mg/kg with subcutaneous injections. Locally, ABI-009 solution at 1.7 mg/ml concentration was well tolerated. There was no adverse effect observed for this dosage level.

## Claims

1. A composition comprising nanoparticles comprising rapamycin and a carrier protein for use in a method of treating pulmonary hypertension in an individual, wherein the method comprises subcutaneously administering to the individual a dose of from about 1 mg/m² to about 10 mg/m² of rapamycin in the composition.

2. The composition for use of claim 1, wherein the dose of rapamycin in the composition is from about 2.5 mg/m² to about 5 mg/m²;
preferably wherein the dose of rapamycin in the composition is about 5 mg/m².

3. The composition for use of claim 1 or 2, wherein the concentration of rapamycin in the blood of the individual is at least about 2 ng/ml five days after administration of the nanoparticle composition; and/or
wherein the concentration of rapamycin in the blood of the individual is no more than about 20 ng/ml seven days after administration of the nanoparticle composition.

4. The composition for use of any one of claims 1 to 3, wherein nanoparticle composition is administered at least once a week, no more than once a week, once a week, once every two weeks, two out of three weeks, or three out of four weeks; and/or
wherein the nanoparticle composition is administered for at least about four weeks.

5. The composition for use of any one of claims 1 to 4, wherein the pulmonary hypertension is pulmonary arterial hypertension (PAH);
preferably wherein the PAH is selected from the group consisting of idiopathic pulmonary arterial hypertension (IPAH), heritable pulmonary arterial hypertension (HPAH), drug and toxin induced PAH, PAH associated with connective tissue disease, and PAH associated with congenital heart defects; and/or
preferably wherein the individual has a WHO functional class III or IV PAH.

6. The composition for use of any one of claims 1 to 5, wherein the composition comprises more than about 50% of rapamycin in nanoparticle form; and/or
wherein the carrier protein is albumin, preferably human serum albumin.

7. The composition for use of any one of claims 1 to 6, wherein the average diameter of the nanoparticles in the composition is no greater than about 200 nm.

8. The composition for use of any one of claims 1 to 7, wherein the weight ratio of the carrier protein to rapamycin in the nanoparticles is less than about 18:1.

9. The composition for use of any one of claims 1 to 8, wherein the individual has had at least one prior therapy for pulmonary hypertension;
preferably wherein the individual has had at least two prior therapies for pulmonary hypertension.

10. The composition for use of claim 9, wherein the prior therapy comprises administering an agent selected from the group consisting of a prostacyclin analogue, an endothelin-1 receptor antagonist, a phosphodiesterase 5 (PDE-5) inhibitor and a soluble guanylate cyclase (sGC) stimulator.

11. The composition for use of claim 9 or 10, wherein the individual has progressed on the prior therapy.

12. The composition for use of any one of claims 1 to 11, wherein the method further comprises administering to the individual a second therapy;
preferably wherein the second therapy is selected from the group consisting of a vasodilator, a prostacyclin analogue, an endothelin-1 receptor antagonist, a PDE-5 inhibitor, an sGC stimulator, epoprostenol, iloprost, treprostiml, bosentan, macitentan, ambrisentan, sildenafil, tadalafil, riociguat and a tyrosine kinase inhibitor, preferably imatinib; and/or
preferably wherein the composition is administered simultaneously, concurrently or sequentially with the second therapy.

13. The composition for use of any one of claims 1 to 12, wherein the individual is a human.

14. The composition for use of any one of claims 1 to 13, wherein the composition is in form of a unit dosage form, wherein the unit dosage form comprises: (a) said composition; and (b) a pharmaceutical acceptable carrier.

15. A kit for use in a method of treating pulmonary hypertension in an individual, wherein the kit comprises: (a) a composition comprising nanoparticles comprising rapamycin and a carrier protein; and (b) instructions for said use of said composition, wherein the method comprises subcutaneously administering to the individual a dose of from about 1 mg/m² to about 10 mg/m² of rapamycin in said composition.

## Patentansprüche

1. Zusammensetzung, umfassend Nanopartikel, umfassend Rapamycin und ein Trägerprotein zur Verwendung in einem Verfahren zur Behandlung der pulmonalen Hypertonie bei einem Individuum, wobei das Verfahren das subkutane Verabreichen einer Dosis von etwa 1 mg/m² bis etwa 10 mg/m² Rapamycin in der Zusammensetzung an das Individuum umfasst.

2. Zusammensetzung zur Verwendung nach Anspruch 1, wobei die Dosis von Rapamycin in der Zusammensetzung etwa 2,5 mg/m² bis etwa 5 mg/m² beträgt;
bevorzugt wobei die Dosis von Rapamycin in der Zusammensetzung etwa 5 mg/m² beträgt.

3. Zusammensetzung zur Verwendung nach Anspruch 1 oder 2, wobei die Konzentration von Rapamycin im Blut des Individuums fünf Tage nach Verabreichung der Nanopartikelzusammensetzung mindestens etwa 2 ng/ml beträgt; und/oder
wobei die Konzentration von Rapamycin im Blut des Individuums sieben Tage nach Verabreichung der Nanopartikelzusammensetzung nicht mehr als etwa 20 ng/ml beträgt.

4. Zusammensetzung zur Verwendung nach einem der Ansprüche 1 bis 3, wobei die Nanopartikelzusammensetzung mindestens einmal wöchentlich, nicht mehr als einmal wöchentlich, einmal wöchentlich, einmal alle zwei Wochen, zwei von drei Wochen oder drei von vier Wochen verabreicht wird; und/oder
wobei die Nanopartikelzusammensetzung für mindestens etwa vier Wochen verabreicht wird.

5. Zusammensetzung zur Verwendung nach einem der Ansprüche 1 bis 4, wobei die pulmonale Hypertonie pulmonal-arterielle Hypertonie (PAH) ist;
bevorzugt wobei die PAH aus der Gruppe ausgewählt ist, die aus idiopathischer pulmonal-arterieller Hypertonie (IPAH), hereditärer pulmonal-arterieller Hypertonie (HPAH), durch Arzneimittel und Toxine induzierter PAH, mit einer Bindegewebserkrankung assoziierter PAH und mit angeborenen Herzfehlern assoziierter PAH besteht; und/oder
bevorzugt wobei das Individuum eine PAH der WHO-Funktionsklasse III oder IV aufweist.

6. Zusammensetzung zur Verwendung nach einem der Ansprüche 1 bis 5, wobei die Zusammensetzung mehr als etwa 50 % Rapamycin in Nanopartikelform umfasst; und/oder
wobei das Trägerprotein Albumin, bevorzugt Humanserumalbumin ist.

7. Zusammensetzung zur Verwendung nach einem der Ansprüche 1 bis 6, wobei der durchschnittliche Durchmesser der Nanopartikel in der Zusammensetzung nicht größer als etwa 200 nm ist.

8. Zusammensetzung zur Verwendung nach einem der Ansprüche 1 bis 7, wobei das Gewichtsverhältnis des Trägerproteins zu Rapamycin in den Nanopartikeln weniger als etwa 18:1 beträgt.

9. Zusammensetzung zur Verwendung nach einem der Ansprüche 1 bis 8, wobei das Individuum mindestens eine vorherige Therapie für pulmonale Hypertonie gehabt hat; bevorzugt wobei das Individuum mindestens zwei vorherige Therapien für pulmonale Hypertonie gehabt hat.

10. Zusammensetzung zur Verwendung nach Anspruch 9, wobei die vorherige Therapie das Verabreichen eines Wirkstoffs umfasst, der aus der Gruppe ausgewählt ist, die aus einem Prostacyclin-Analogon, einem Endothelin-1-Rezeptorantagonisten, einem Phosphodiesterase-5-(PDE-5)-Hemmer und einem Stimulator der löslichen Guanylatzyklase (sGC) besteht.

11. Zusammensetzung zur Verwendung nach Anspruch 9 oder 10, wobei das Individuum auf die vorherige Therapie angesprochen hat.

12. Zusammensetzung zur Verwendung nach einem der Ansprüche 1 bis 11, wobei das Verfahren weiter das Verabreichen einer zweiten Therapie an das Individuum umfasst;
bevorzugt wobei die zweite Therapie aus der Gruppe ausgewählt ist, die aus einem Vasodilatator, einem Prostacyclin-Analogon, einem Endothelin-1-Rezeptorantagonisten, einem PDE-5-Hemmer, einem sGC-Stimulator, Epoprostenol, Iloprost, Treprostinil, Bosentan, Macitentan, Ambrisentan, Sildenafil, Tadalafil, Riociguat und einem Tyrosinkinaseinhibitor, bevorzugt Imatinib, besteht; und/oder
bevorzugt wobei die Zusammensetzung gleichzeitig, parallel oder sequenziell mit der zweiten Therapie verabreicht wird.

13. Zusammensetzung zur Verwendung nach einem der Ansprüche 1 bis 12, wobei das Individuum ein Mensch ist.

14. Zusammensetzung zur Verwendung nach einem der Ansprüche 1 bis 13, wobei die Zusammensetzung in einer Einheitsdosierungsform vorliegt, wobei die Einheitsdosierungsform umfasst: (a) die Zusammensetzung; und (b) einen pharmazeutisch annehmbaren Träger.

15. Kit zur Verwendung in einem Verfahren zur Behandlung der pulmonalen Hypertonie bei einem Individuum, wobei das Kit umfasst: (a) eine Zusammensetzung, umfassend Nanopartikel, umfassend Rapamycin und ein Trägerprotein; und (b) Anweisungen für die Verwendung der Zusammensetzung, wobei das Verfahren das subkutane Verabreichen einer Dosis von etwa 1 mg/m² bis etwa 10 mg/m² Rapamycin in der Zusammensetzung an das Individuum umfasst.

## Revendications

1. Composition comprenant des nanoparticules comprenant de la rapamycine et une protéine porteuse pour une utilisation dans un procédé de traitement de l'hypertension pulmonaire chez un individu, dans laquelle le procédé comprend une administration par voie sous-cutanée à l'individu d'une dose d'environ 1 mg/m² à environ 10 mg/m² de rapamycine dans la composition.

2. Composition pour une utilisation selon la revendication 1, dans laquelle la dose de rapamycine dans la composition est d'environ 2,5 mg/m² à environ 5 mg/m² ;
de préférence dans laquelle la dose de rapamycine dans la composition est d'environ 5 mg/m².

3. Composition pour une utilisation selon la revendication 1 ou 2, dans laquelle la concentration de rapamycine dans le sang de l'individu est d'au moins environ 2 ng/ml cinq jours après l'administration de la composition nanoparticulaire ; et/ou
dans laquelle la concentration de rapamycine dans le sang de l'individu n'est pas supérieure à environ 20 ng/ml sept jours après l'administration de la composition nanoparticulaire.

4. Composition pour une utilisation selon l'une quelconque des revendications 1 à 3, dans laquelle la composition nanoparticulaire est administrée au moins une fois par semaine, pas plus d'une fois par semaine, une fois par semaine, une fois toutes les deux semaines, deux semaines sur trois, ou trois semaines sur quatre ;
et/ou
dans laquelle la composition nanoparticulaire est administrée pendant au moins environ quatre semaines.

5. Composition pour une utilisation selon l'une quelconque des revendications 1 à 4, dans laquelle l'hypertension pulmonaire est une hypertension artérielle pulmonaire (HTAP) ;
de préférence dans laquelle l'HTAP est sélectionnée parmi le groupe consistant en une hypertension artérielle pulmonaire idiopathique (HTAP idiopathique), une hypertension artérielle pulmonaire héréditaire (HTAP héréditaire), une hypertension artérielle pulmonaire induite par des médicaments et des toxines, une hypertension artérielle pulmonaire associée à une tissu conjonctif, et une hypertension artérielle pulmonaire associée à une cardiopathie congénitale ; et/ou
de préférence dans laquelle l'individu présente une HTAP de classe fonctionnelle III ou IV de l'OMS.

6. Composition pour une utilisation selon l'une quelconque des revendications 1 à 5, dans laquelle la composition comprend plus d'environ 50 % de rapamycine sous forme de nanoparticules ; et/ou
dans laquelle la protéine porteuse est une albumine, de préférence une albumine sérique humaine.

7. Composition pour une utilisation selon l'une quelconque des revendications 1 à 6, dans laquelle le diamètre moyen des nanoparticules dans la composition n'est pas supérieur à environ 200 nm.

8. Composition pour une utilisation selon l'une quelconque des revendications 1 à 7, dans laquelle le rapport en poids de la protéine porteuse sur la rapamycine dans les nanoparticules est inférieur à environ 18:1.

9. Composition pour une utilisation selon l'une quelconque des revendications 1 à 8, dans laquelle l'individu a eu au moins une thérapie antérieure pour l'hypertension pulmonaire ;
de préférence dans laquelle l'individu a eu au moins deux thérapies antérieures pour l'hypertension pulmonaire.

10. Composition pour une utilisation selon la revendication 9, dans laquelle la thérapie antérieure comprend une administration d'un agent sélectionné parmi le groupe consistant en un analogue de la prostacycline, un antagoniste du récepteur de l'endothéline-1, un inhibiteur de la phosphodiestérase 5 (PDE-5) et un stimulateur de la guanylate cyclase soluble (sGC).

11. Composition pour une utilisation selon la revendication 9 ou 10, dans laquelle l'individu a progressé sous la thérapie antérieure.

12. Composition pour une utilisation selon l'une quelconque des revendications 1 à 11, dans laquelle le procédé comprend en outre une administration à l'individu d'une deuxième thérapie ;
de préférence dans laquelle la deuxième thérapie est sélectionnée parmi le groupe consistant en un vasodilatateur, un analogue de la prostacycline, un antagoniste du récepteur de l'endothéline-1, un inhibiteur de la PDE-5, un stimulateur de la sGC, l'époprosténol, l'iloprost, le treprostinil, le bosentan, le macitentan, l'ambrisentan, le sildénafil, le tadalafil, le riociguat et un inhibiteur de la tyrosine kinase, de préférence l'imatinib ; et/ou
de préférence dans laquelle la composition est administrée simultanément, concurremment ou séquentiellement avec la deuxième thérapie.

13. Composition pour une utilisation selon l'une quelconque des revendications 1 à 12, dans laquelle l'individu est un être humain.

14. Composition pour une utilisation selon l'une quelconque des revendications 1 à 13, dans laquelle la composition est sous la forme d'une forme de dose unitaire, dans laquelle la forme de dose unitaire comprend : (a) ladite composition ; et (b) un support pharmaceutiquement acceptable.

15. Kit pour une utilisation dans un procédé de traitement de l'hypertension pulmonaire chez un individu, dans lequel le kit comprend : (a) une composition comprenant des nanoparticules comprenant de la rapamycine et une protéine porteuse ; et (b) des instructions pour ladite utilisation de ladite composition, dans lequel le procédé comprend une administration par voie sous-cutanée à l'individu d'une dose d'environ 1 mg/m² à environ 10 mg/m² de rapamycine dans ladite composition.
